(19) Europäisches Patentamt
European Patent Office
Office européen des brevets

(11) EP 3 915 581 A1

(12) **EUROPEAN PATENT APPLICATION**
published in accordance with Art. 153(4) EPC

(43) Date of publication:
**01.12.2021 Bulletin 2021/48**

(21) Application number: **20745918.1**

(22) Date of filing: **24.01.2020**

(51) Int Cl.:
***A61K 39/395*** *(2006.01)* ***A61P 1/00*** *(2006.01)*
***A61P 11/00*** *(2006.01)* ***A61P 35/00*** *(2006.01)*
***C07K 16/28*** *(2006.01)* ***C07K 16/46*** *(2006.01)*
***C12N 15/13*** *(2006.01)*

(86) International application number:
**PCT/JP2020/002476**

(87) International publication number:
**WO 2020/153467 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.01.2019 JP 2019009845**

(71) Applicant: **CHUGAI SEIYAKU KABUSHIKI KAISHA**
**Tokyo 115-8543 (JP)**

(72) Inventors:
- **NEZU, Junichi**
  **Kamakura-shi, Kanagawa 247-8530 (JP)**
- **KODAMA, Tatsushi**
  **Synapse, 138623 (SG)**
- **HOSHINO, Mayumi**
  **Kamakura-shi, Kanagawa 247-8530 (JP)**
- **KIMURA, Naoki**
  **Kamakura-shi, Kanagawa 247-8530 (JP)**
- **NAGANO, Kohji**
  **Kamakura-shi, Kanagawa 247-8530 (JP)**
- **KATO, Kuniyasu**
  **Kamakura-shi, Kanagawa 247-8530 (JP)**
- **YOKOTA, Yukari**
  **Kamakura-shi, Kanagawa 247-8530 (JP)**

(74) Representative: **Vossius & Partner**
**Patentanwälte Rechtsanwälte mbB**
**Siebertstraße 3**
**81675 München (DE)**

(54) **NOVEL CANCER ANTIGENS AND ANTIBODIES OF SAID ANTIGENS**

(57) In a non-limiting embodiment, isolated antibodies that bind to any of the following proteins are provided: XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, and TMPRSS4.

EP 3 915 581 A1

SEQUENCE LISTING

<110> CHUGAI SEIYAKU KABUSHIKI KAISHA

<120> Novel tumor antigens and antibodies thereof

<130> C1-A1834P

<150> JP 2019-009845
<151> 2019-01-24

<160> 86

<170> PatentIn version 3.5

<210> 1
<211> 696
<212> PRT
<213> Homo sapiens

<400> 1

```
Met Lys Phe Ala Glu His Leu Ser Ala His Ile Thr Pro Glu Trp Arg
1               5                   10                  15

Lys Gln Tyr Ile Gln Tyr Glu Ala Phe Lys Asp Met Leu Tyr Ser Ala
            20                  25                  30

Gln Asp Gln Ala Pro Ser Val Glu Val Thr Asp Glu Asp Thr Val Lys
        35                  40                  45

Arg Tyr Phe Ala Lys Phe Glu Glu Lys Phe Phe Gln Thr Cys Glu Lys
    50                  55                  60

Glu Leu Ala Lys Ile Asn Thr Phe Tyr Ser Glu Lys Leu Ala Glu Ala
65                  70                  75                  80

Gln Arg Arg Phe Ala Thr Leu Gln Asn Glu Leu Gln Ser Ser Leu Asp
                85                  90                  95

Ala Gln Lys Glu Ser Thr Gly Val Thr Thr Leu Arg Gln Arg Arg Lys
            100                 105                 110

Pro Val Phe His Leu Ser His Glu Glu Arg Val Gln His Arg Asn Ile
        115                 120                 125

Lys Asp Leu Lys Leu Ala Phe Ser Glu Phe Tyr Leu Ser Leu Ile Leu
    130                 135                 140

Leu Gln Asn Tyr Gln Asn Leu Asn Phe Thr Gly Phe Arg Lys Ile Leu
145                 150                 155                 160

Lys Lys His Asp Lys Ile Leu Glu Thr Ser Arg Gly Ala Asp Trp Arg
```

165 170 175

Val Ala His Val Glu Val Ala Pro Phe Tyr Thr Cys Lys Lys Ile Asn
180 185 190

Gln Leu Ile Ser Glu Thr Glu Ala Val Val Thr Asn Glu Leu Glu Asp
195 200 205

Gly Asp Arg Gln Lys Ala Met Lys Arg Leu Arg Val Pro Pro Leu Gly
210 215 220

Ala Ala Gln Pro Ala Pro Ala Trp Thr Thr Phe Arg Val Gly Leu Phe
225 230 235 240

Cys Gly Ile Phe Ile Val Leu Asn Ile Thr Leu Val Leu Ala Ala Val
245 250 255

Phe Lys Leu Glu Thr Asp Arg Ser Ile Trp Pro Leu Ile Arg Ile Tyr
260 265 270

Arg Gly Gly Phe Leu Leu Ile Glu Phe Leu Phe Leu Leu Gly Ile Asn
275 280 285

Thr Tyr Gly Trp Arg Gln Ala Gly Val Asn His Val Leu Ile Phe Glu
290 295 300

Leu Asn Pro Arg Ser Asn Leu Ser His Gln His Leu Phe Glu Ile Ala
305 310 315 320

Gly Phe Leu Gly Ile Leu Trp Cys Leu Ser Leu Leu Ala Cys Phe Phe
325 330 335

Ala Pro Ile Ser Val Ile Pro Thr Tyr Val Tyr Pro Leu Ala Leu Tyr
340 345 350

Gly Phe Met Val Phe Phe Leu Ile Asn Pro Thr Lys Thr Phe Tyr Tyr
355 360 365

Lys Ser Arg Phe Trp Leu Leu Lys Leu Leu Phe Arg Val Phe Thr Ala
370 375 380

Pro Phe His Lys Val Gly Phe Ala Asp Phe Trp Leu Ala Asp Gln Leu
385 390 395 400

Asn Ser Leu Ser Val Ile Leu Met Asp Leu Glu Tyr Met Ile Cys Phe
405 410 415

Tyr Ser Leu Glu Leu Lys Trp Asp Glu Ser Lys Gly Leu Leu Pro Asn
420 425 430

Asn Ser Glu Glu Ser Gly Ile Cys His Lys Tyr Thr Tyr Gly Val Arg
435 440 445

Ala Ile Val Gln Cys Ile Pro Ala Trp Leu Arg Phe Ile Gln Cys Leu
450 455 460

Arg Arg Tyr Arg Asp Thr Lys Arg Ala Phe Pro His Leu Val Asn Ala
465 470 475 480

Gly Lys Tyr Ser Thr Thr Phe Phe Met Val Thr Phe Ala Ala Leu Tyr
485 490 495

Ser Thr His Lys Glu Arg Gly His Ser Asp Thr Met Val Phe Phe Tyr
500 505 510

Leu Trp Ile Val Phe Tyr Ile Ile Ser Ser Cys Tyr Thr Leu Ile Trp
515 520 525

Asp Leu Lys Met Asp Trp Gly Leu Phe Asp Lys Asn Ala Gly Glu Asn
530 535 540

Thr Phe Leu Arg Glu Glu Ile Val Tyr Pro Gln Lys Ala Tyr Tyr Tyr
545 550 555 560

Cys Ala Ile Ile Glu Asp Val Ile Leu Arg Phe Ala Trp Thr Ile Gln
565 570 575

Ile Ser Ile Thr Ser Thr Thr Leu Leu Pro His Ser Gly Asp Ile Ile
580 585 590

Ala Thr Val Phe Ala Pro Leu Glu Val Phe Arg Arg Phe Val Trp Asn
595 600 605

Phe Phe Arg Leu Glu Asn Glu His Leu Asn Asn Cys Gly Glu Phe Arg
610 615 620

Ala Val Arg Asp Ile Ser Val Ala Pro Leu Asn Ala Asp Asp Gln Thr
625 630 635 640

Leu Leu Glu Gln Met Met Asp Gln Asp Asp Gly Val Arg Asn Arg Gln
645 650 655

Lys Asn Arg Ser Trp Lys Tyr Asn Gln Ser Ile Ser Leu Arg Arg Pro
660 665 670

```
Arg Leu Ala Ser Gln Ser Lys Ala Arg Asp Thr Lys Val Leu Ile Glu
        675                 680                 685

Asp Thr Asp Asp Glu Ala Asn Thr
    690                 695
```

<210> 2
<211> 564
<212> PRT
<213> Homo sapiens

<400> 2

```
Met Gly Asn Trp Val Val Asn His Trp Phe Ser Val Leu Phe Leu Val
1               5                   10                  15

Val Trp Leu Gly Leu Asn Val Phe Leu Phe Val Asp Ala Phe Leu Lys
            20                  25                  30

Tyr Glu Lys Ala Asp Lys Tyr Tyr Tyr Thr Arg Lys Ile Leu Gly Ser
        35                  40                  45

Thr Leu Ala Cys Ala Arg Ala Ser Ala Leu Cys Leu Asn Phe Asn Ser
    50                  55                  60

Thr Leu Ile Leu Leu Pro Val Cys Arg Asn Leu Leu Ser Phe Leu Arg
65                  70                  75                  80

Gly Thr Cys Ser Phe Cys Ser Arg Thr Leu Arg Lys Gln Leu Asp His
                85                  90                  95

Asn Leu Thr Phe His Lys Leu Val Ala Tyr Met Ile Cys Leu His Thr
            100                 105                 110

Ala Ile His Ile Ile Ala His Leu Phe Asn Phe Asp Cys Tyr Ser Arg
        115                 120                 125

Ser Arg Gln Ala Thr Asp Gly Ser Leu Ala Ser Ile Leu Ser Ser Leu
    130                 135                 140

Ser His Asp Glu Lys Lys Gly Gly Ser Trp Leu Asn Pro Ile Gln Ser
145                 150                 155                 160

Arg Asn Thr Thr Val Glu Tyr Val Thr Phe Thr Ser Ile Ala Gly Leu
                165                 170                 175

Thr Gly Val Ile Met Thr Ile Ala Leu Ile Leu Met Val Thr Ser Ala
            180                 185                 190
```

Thr Glu Phe Ile Arg Arg Ser Tyr Phe Glu Val Phe Trp Tyr Thr His
195 200 205

His Leu Phe Ile Phe Tyr Ile Leu Gly Leu Gly Ile His Gly Ile Gly
210 215 220

Gly Ile Val Arg Gly Gln Thr Glu Glu Ser Met Asn Glu Ser His Pro
225 230 235 240

Arg Lys Cys Ala Glu Ser Phe Glu Met Trp Asp Asp Arg Asp Ser His
245 250 255

Cys Arg Arg Pro Lys Phe Glu Gly His Pro Pro Glu Ser Trp Lys Trp
260 265 270

Ile Leu Ala Pro Val Ile Leu Tyr Ile Cys Glu Arg Ile Leu Arg Phe
275 280 285

Tyr Arg Ser Gln Gln Lys Val Val Ile Thr Lys Val Val Met His Pro
290 295 300

Ser Lys Val Leu Glu Leu Gln Met Asn Lys Arg Gly Phe Ser Met Glu
305 310 315 320

Val Gly Gln Tyr Ile Phe Val Asn Cys Pro Ser Ile Ser Leu Leu Glu
325 330 335

Trp His Pro Phe Thr Leu Thr Ser Ala Pro Glu Glu Asp Phe Phe Ser
340 345 350

Ile His Ile Arg Ala Ala Gly Asp Trp Thr Glu Asn Leu Ile Arg Ala
355 360 365

Phe Glu Gln Gln Tyr Ser Pro Ile Pro Arg Ile Glu Val Asp Gly Pro
370 375 380

Phe Gly Thr Ala Ser Glu Asp Val Phe Gln Tyr Glu Val Ala Val Leu
385 390 395 400

Val Gly Ala Gly Ile Gly Val Thr Pro Phe Ala Ser Ile Leu Lys Ser
405 410 415

Ile Trp Tyr Lys Phe Gln Cys Ala Asp His Asn Leu Lys Thr Lys Lys
420 425 430

Ile Tyr Phe Tyr Trp Ile Cys Arg Glu Thr Gly Ala Phe Ser Trp Phe
435 440 445

Asn Asn Leu Leu Thr Ser Leu Glu Gln Glu Met Glu Glu Leu Gly Lys
450 455 460

Val Gly Phe Leu Asn Tyr Arg Leu Phe Leu Thr Gly Trp Asp Ser Asn
465 470 475 480

Ile Val Gly His Ala Ala Leu Asn Phe Asp Lys Ala Thr Asp Ile Val
485 490 495

Thr Gly Leu Lys Gln Lys Thr Ser Phe Gly Arg Pro Met Trp Asp Asn
500 505 510

Glu Phe Ser Thr Ile Ala Thr Ser His Pro Lys Ser Val Val Gly Val
515 520 525

Phe Leu Cys Gly Pro Arg Thr Leu Ala Lys Ser Leu Arg Lys Cys Cys
530 535 540

His Arg Tyr Ser Ser Leu Asp Pro Arg Lys Val Gln Phe Tyr Phe Asn
545 550 555 560

Lys Glu Asn Phe

<210> 3
<211> 410
<212> PRT
<213> Homo sapiens

<400> 3

Met Glu Asp Pro Ser Gly Ala Arg Glu Pro Arg Ala Arg Pro Arg Glu
1 5 10 15

Arg Asp Pro Gly Arg Arg Pro His Pro Asp Gln Gly Arg Thr His Asp
20 25 30

Arg Pro Arg Asp Arg Pro Gly Asp Pro Arg Arg Lys Arg Ser Ser Asp
35 40 45

Gly Asn Arg Arg Arg Asp Gly Asp Arg Asp Pro Glu Arg Asp Gln Glu
50 55 60

Arg Asp Gly Asn Arg Asp Arg Asn Arg Asp Arg Glu Arg Glu Arg Glu
65 70 75 80

Arg Glu Arg Asp Pro Asp Arg Gly Pro Arg Arg Asp Thr His Arg Asp
85 90 95

```
Ala Gly Pro Arg Ala Gly Glu His Gly Val Trp Glu Lys Pro Arg Gln
            100                 105                 110

Ser Arg Thr Arg Asp Gly Ala Arg Gly Leu Thr Trp Asp Ala Ala Ala
        115                 120                 125

Pro Pro Gly Pro Ala Pro Trp Glu Ala Pro Glu Pro Pro Gln Pro Gln
    130                 135                 140

Arg Lys Gly Asp Pro Gly Arg Arg Arg Pro Glu Ser Glu Pro Pro Ser
145                 150                 155                 160

Glu Arg Tyr Leu Pro Ser Thr Pro Arg Pro Gly Arg Glu Glu Val Glu
                165                 170                 175

Tyr Tyr Gln Ser Glu Ala Glu Gly Leu Leu Glu Cys His Lys Cys Lys
            180                 185                 190

Tyr Leu Cys Thr Gly Arg Gly Val Val Gln Ile Val Glu Val Val Leu
        195                 200                 205

Asn Gly Met Val Leu Ile Cys Ile Val Ala Ser Tyr Phe Val Leu Ala
    210                 215                 220

Gly Phe Ser Ala Ser Phe Ser Ser Gly Gly Gly Phe Gly Asn Asn Tyr
225                 230                 235                 240

Tyr Ser Pro Phe Glu Gly Thr Glu Leu Glu Gln Val Arg Gln Leu Asp
                245                 250                 255

Gln Gln Tyr Thr Ile Leu Arg Ser Pro Leu Ile Tyr Gly Gly Val Ala
            260                 265                 270

Val Ser Leu Gly Leu Gly Val Leu Thr Met Gly Val Leu Leu Gln Gly
        275                 280                 285

Ala Lys Ser Arg Thr Met Leu Ser Gly Lys Trp Leu Leu Thr Glu Ala
    290                 295                 300

Ala Phe Ser Leu Leu Ala Ala Val Gly Tyr Cys Thr Gly Ile Gly Val
305                 310                 315                 320

Tyr Leu His Val Ala Leu Gln Ile Asn Ser Thr Asp Thr Cys Lys Thr
                325                 330                 335

Arg Glu Arg Leu Tyr Ala Arg Lys Gly Leu Thr Trp Met Asp Cys Gln
```

```
            340                 345                 350

Leu Ala Gly Thr Asp Gly Ala Ala Ala Thr Phe Ala Cys Leu Leu Val
        355                 360                 365

Ile Met Tyr Gly Ala Ser Val Val Leu Ala Leu Arg Ser Tyr Arg Glu
    370                 375                 380

Gln Lys Arg Tyr Lys Gly Ser Arg Glu Gln Pro Gly Ser Tyr Ser Asp
385                 390                 395                 400

Ala Pro Glu Tyr Leu Trp Ser Gly Thr Leu
                405                 410


<210>  4
<211>  401
<212>  PRT
<213>  Homo sapiens

<400>  4

Met Glu Asp Pro Ser Gly Ala Arg Glu Pro Arg Ala Arg Pro Arg Glu
1               5                   10                  15

Arg Asp Pro Gly Arg Arg Pro His Pro Asp Gln Gly Arg Thr His Asp
            20                  25                  30

Arg Pro Arg Asp Arg Pro Gly Asp Pro Arg Arg Lys Arg Ser Ser Asp
        35                  40                  45

Gly Asn Arg Arg Arg Asp Gly Asp Arg Asp Pro Glu Arg Asp Gln Glu
    50                  55                  60

Arg Asp Gly Asn Arg Asp Arg Asn Arg Asp Arg Glu Arg Glu Arg Glu
65                  70                  75                  80

Arg Glu Arg Asp Pro Asp Arg Gly Pro Arg Arg Asp Thr His Arg Asp
                85                  90                  95

Ala Gly Pro Arg Ala Gly Glu His Gly Val Trp Glu Lys Pro Arg Gln
            100                 105                 110

Ser Arg Thr Arg Asp Gly Ala Arg Gly Leu Thr Trp Asp Ala Ala Ala
        115                 120                 125

Pro Pro Gly Pro Ala Pro Trp Glu Ala Pro Glu Pro Pro Gln Pro Gln
    130                 135                 140

Arg Lys Gly Asp Pro Gly Arg Arg Arg Pro Glu Ser Glu Pro Pro Ser
```

```
145                 150                 155                 160

Glu Arg Tyr Leu Pro Ser Thr Pro Arg Pro Gly Arg Glu Glu Val Glu
                165                 170                 175

Tyr Tyr Gln Ser Glu Ala Glu Gly Leu Leu Glu Cys His Lys Cys Lys
            180                 185                 190

Tyr Leu Cys Thr Gly Arg Ala Cys Cys Gln Met Leu Glu Val Leu Leu
        195                 200                 205

Asn Leu Leu Ile Leu Ala Cys Ser Ser Val Ser Tyr Ser Ser Thr Gly
    210                 215                 220

Gly Tyr Thr Gly Ile Thr Ser Leu Gly Gly Ile Tyr Tyr Tyr Gln Phe
225                 230                 235                 240

Gly Gly Ala Tyr Ser Gly Phe Asp Gly Ala Asp Gly Glu Lys Ala Gln
                245                 250                 255

Gln Leu Asp Val Gln Phe Tyr Gln Leu Lys Leu Pro Met Val Thr Val
            260                 265                 270

Ala Met Ala Cys Ser Gly Ala Leu Thr Ala Leu Cys Cys Leu Phe Val
        275                 280                 285

Ala Met Gly Val Leu Arg Val Pro Trp His Cys Pro Leu Leu Leu Val
    290                 295                 300

Thr Glu Gly Leu Leu Asp Met Leu Ile Ala Gly Gly Tyr Ile Pro Ala
305                 310                 315                 320

Leu Tyr Phe Tyr Phe His Tyr Leu Ser Ala Ala Tyr Gly Ser Pro Val
                325                 330                 335

Cys Lys Glu Arg Gln Ala Leu Tyr Gln Ser Lys Gly Tyr Ser Gly Phe
            340                 345                 350

Gly Cys Ser Phe His Gly Ala Asp Ile Gly Ala Gly Ile Phe Ala Ala
        355                 360                 365

Leu Gly Ile Val Val Phe Ala Leu Gly Ala Val Leu Ala Ile Lys Gly
    370                 375                 380

Tyr Arg Lys Val Arg Lys Leu Lys Glu Lys Pro Ala Glu Met Phe Glu
385                 390                 395                 400
```

Phe

<210> 5
<211> 252
<212> PRT
<213> Homo sapiens

<400> 5

Met Ala Gln Leu Cys Gly Leu Arg Arg Ser Arg Ala Phe Leu Ala Leu
1 5 10 15

Leu Gly Ser Leu Leu Leu Ser Gly Val Leu Ala Ala Asp Arg Glu Arg
20 25 30

Ser Ile His Asp Phe Cys Leu Val Ser Lys Val Val Gly Arg Cys Arg
35 40 45

Ala Ser Met Pro Arg Trp Trp Tyr Asn Val Thr Asp Gly Ser Cys Gln
50 55 60

Leu Phe Val Tyr Gly Gly Cys Asp Gly Asn Ser Asn Asn Tyr Leu Thr
65 70 75 80

Lys Glu Glu Cys Leu Lys Lys Cys Ala Thr Val Thr Glu Asn Ala Thr
85 90 95

Gly Asp Leu Ala Thr Ser Arg Asn Ala Ala Asp Ser Ser Val Pro Ser
100 105 110

Ala Pro Arg Arg Gln Asp Ser Glu Asp His Ser Ser Asp Met Phe Asn
115 120 125

Tyr Glu Glu Tyr Cys Thr Ala Asn Ala Val Thr Gly Pro Cys Arg Ala
130 135 140

Ser Phe Pro Arg Trp Tyr Phe Asp Val Glu Arg Asn Ser Cys Asn Asn
145 150 155 160

Phe Ile Tyr Gly Gly Cys Arg Gly Asn Lys Asn Ser Tyr Arg Ser Glu
165 170 175

Glu Ala Cys Met Leu Arg Cys Phe Arg Gln Gln Glu Asn Pro Pro Leu
180 185 190

Pro Leu Gly Ser Lys Val Val Val Leu Ala Gly Leu Phe Val Met Val
195 200 205

```
Leu Ile Leu Phe Leu Gly Ala Ser Met Val Tyr Leu Ile Arg Val Ala
    210                 215                 220

Arg Arg Asn Gln Glu Arg Ala Leu Arg Thr Val Trp Ser Ser Gly Asp
225                 230                 235                 240

Asp Lys Glu Gln Leu Val Lys Asn Thr Tyr Val Leu
                245                 250
```

<210> 6
<211> 330
<212> PRT
<213> Homo sapiens

<400> 6

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175
```

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 7
<211> 431
<212> PRT
<213> Homo sapiens

<400> 7

Met Phe Phe Gly Gly Glu Gly Ser Leu Thr Tyr Thr Leu Val Ile Ile
1 5 10 15

Cys Phe Leu Thr Leu Arg Leu Ser Ala Ser Gln Asn Cys Leu Lys Lys
20 25 30

Ser Leu Glu Asp Val Val Ile Asp Ile Gln Ser Ser Leu Ser Lys Gly
35 40 45

Ile Arg Gly Asn Glu Pro Val Tyr Thr Ser Thr Gln Glu Asp Cys Ile
50 55 60

```
Asn Ser Cys Cys Ser Thr Lys Asn Ile Ser Gly Asp Lys Ala Cys Asn
65                  70                  75                  80

Leu Met Ile Phe Asp Thr Arg Lys Thr Ala Arg Gln Pro Asn Cys Tyr
                85                  90                  95

Leu Phe Phe Cys Pro Asn Glu Glu Ala Cys Pro Leu Lys Pro Ala Lys
            100                 105                 110

Gly Leu Met Ser Tyr Arg Ile Ile Thr Asp Phe Pro Ser Leu Thr Arg
        115                 120                 125

Asn Leu Pro Ser Gln Glu Leu Pro Gln Glu Asp Ser Leu Leu His Gly
    130                 135                 140

Gln Phe Ser Gln Ala Val Thr Pro Leu Ala His His His Thr Asp Tyr
145                 150                 155                 160

Ser Lys Pro Thr Asp Ile Ser Trp Arg Asp Thr Leu Ser Gln Lys Phe
                165                 170                 175

Gly Ser Ser Asp His Leu Glu Lys Leu Phe Lys Met Asp Glu Ala Ser
            180                 185                 190

Ala Gln Leu Leu Ala Tyr Lys Glu Lys Gly His Ser Gln Ser Ser Gln
        195                 200                 205

Phe Ser Ser Asp Gln Glu Ile Ala His Leu Leu Pro Glu Asn Val Ser
    210                 215                 220

Ala Leu Pro Ala Thr Val Ala Val Ala Ser Pro His Thr Thr Ser Ala
225                 230                 235                 240

Thr Pro Lys Pro Ala Thr Leu Leu Pro Thr Asn Ala Ser Val Thr Pro
                245                 250                 255

Ser Gly Thr Ser Gln Pro Gln Leu Ala Thr Thr Ala Pro Pro Val Thr
            260                 265                 270

Thr Val Thr Ser Gln Pro Pro Thr Thr Leu Ile Ser Thr Val Phe Thr
        275                 280                 285

Arg Ala Ala Ala Thr Leu Gln Ala Met Ala Thr Thr Ala Val Leu Thr
    290                 295                 300

Thr Thr Phe Gln Ala Pro Thr Asp Ser Lys Gly Ser Leu Glu Thr Ile
305                 310                 315                 320
```

Pro Phe Thr Glu Ile Ser Asn Leu Thr Leu Asn Thr Gly Asn Val Tyr
325 330 335

Asn Pro Thr Ala Leu Ser Met Ser Asn Val Glu Ser Ser Thr Met Asn
340 345 350

Lys Thr Ala Ser Trp Glu Gly Arg Glu Ala Ser Pro Gly Ser Ser Ser
355 360 365

Gln Gly Ser Val Pro Glu Asn Gln Tyr Gly Leu Pro Phe Glu Lys Trp
370 375 380

Leu Leu Ile Gly Ser Leu Leu Phe Gly Val Leu Phe Leu Val Ile Gly
385 390 395 400

Leu Val Leu Leu Gly Arg Ile Leu Ser Glu Ser Leu Arg Arg Lys Arg
405 410 415

Tyr Ser Arg Leu Asp Tyr Leu Ile Asn Gly Ile Tyr Val Asp Ile
420 425 430

<210> 8
<211> 1212
<212> PRT
<213> Homo sapiens

<400> 8

Met Glu Pro Arg Pro Thr Ala Pro Ser Ser Gly Ala Pro Gly Leu Ala
1 5 10 15

Gly Val Gly Glu Thr Pro Ser Ala Ala Ala Leu Ala Ala Ala Arg Val
20 25 30

Glu Leu Pro Gly Thr Ala Val Pro Ser Val Pro Glu Asp Ala Ala Pro
35 40 45

Ala Ser Arg Asp Gly Gly Gly Val Arg Asp Glu Gly Pro Ala Ala Ala
50 55 60

Gly Asp Gly Leu Gly Arg Pro Leu Gly Pro Thr Pro Ser Gln Ser Arg
65 70 75 80

Phe Gln Val Asp Leu Val Ser Glu Asn Ala Gly Arg Ala Ala Ala Ala
85 90 95

Ala Ala Ala Ala Ala Ala Ala Ala Ala Ala Ala Gly Ala Gly Ala Gly
100 105 110

```
Ala Lys Gln Thr Pro Ala Asp Gly Glu Ala Ser Gly Glu Ser Glu Pro
        115                 120                 125

Ala Lys Gly Ser Glu Glu Ala Lys Gly Arg Phe Arg Val Asn Phe Val
    130                 135                 140

Asp Pro Ala Ala Ser Ser Ser Ala Glu Asp Ser Leu Ser Asp Ala Ala
145                 150                 155                 160

Gly Val Gly Val Asp Gly Pro Asn Val Ser Phe Gln Asn Gly Gly Asp
                165                 170                 175

Thr Val Leu Ser Glu Gly Ser Ser Leu His Ser Gly Gly Gly Gly Gly
            180                 185                 190

Ser Gly His His Gln His Tyr Tyr Tyr Asp Thr His Thr Asn Thr Tyr
        195                 200                 205

Tyr Leu Arg Thr Phe Gly His Asn Thr Met Asp Ala Val Pro Arg Ile
    210                 215                 220

Asp His Tyr Arg His Thr Ala Ala Gln Leu Gly Glu Lys Leu Leu Arg
225                 230                 235                 240

Pro Ser Leu Ala Glu Leu His Asp Glu Leu Glu Lys Glu Pro Phe Glu
                245                 250                 255

Asp Gly Phe Ala Asn Gly Glu Glu Ser Thr Pro Thr Arg Asp Ala Val
            260                 265                 270

Val Thr Tyr Thr Ala Glu Ser Lys Gly Val Val Lys Phe Gly Trp Ile
        275                 280                 285

Lys Gly Val Leu Val Arg Cys Met Leu Asn Ile Trp Gly Val Met Leu
    290                 295                 300

Phe Ile Arg Leu Ser Trp Ile Val Gly Gln Ala Gly Ile Gly Leu Ser
305                 310                 315                 320

Val Leu Val Ile Met Met Ala Thr Val Val Thr Thr Ile Thr Gly Leu
                325                 330                 335

Ser Thr Ser Ala Ile Ala Thr Asn Gly Phe Val Arg Gly Gly Gly Ala
            340                 345                 350

Tyr Tyr Leu Ile Ser Arg Ser Leu Gly Pro Glu Phe Gly Gly Ala Ile
        355                 360                 365
```

Gly Leu Ile Phe Ala Phe Ala Asn Ala Val Ala Val Ala Met Tyr Val
370 375 380

Val Gly Phe Ala Glu Thr Val Val Glu Leu Leu Lys Glu His Ser Ile
385 390 395 400

Leu Met Ile Asp Glu Ile Asn Asp Ile Arg Ile Ile Gly Ala Ile Thr
405 410 415

Val Val Ile Leu Leu Gly Ile Ser Val Ala Gly Met Glu Trp Glu Ala
420 425 430

Lys Ala Gln Ile Val Leu Leu Val Ile Leu Leu Leu Ala Ile Gly Asp
435 440 445

Phe Val Ile Gly Thr Phe Ile Pro Leu Glu Ser Lys Lys Pro Lys Gly
450 455 460

Phe Phe Gly Tyr Lys Ser Glu Ile Phe Asn Glu Asn Phe Gly Pro Asp
465 470 475 480

Phe Arg Glu Glu Glu Thr Phe Phe Ser Val Phe Ala Ile Phe Phe Pro
485 490 495

Ala Ala Thr Gly Ile Leu Ala Gly Ala Asn Ile Ser Gly Asp Leu Ala
500 505 510

Asp Pro Gln Ser Ala Ile Pro Lys Gly Thr Leu Leu Ala Ile Leu Ile
515 520 525

Thr Thr Leu Val Tyr Val Gly Ile Ala Val Ser Val Gly Ser Cys Val
530 535 540

Val Arg Asp Ala Thr Gly Asn Val Asn Asp Thr Ile Val Thr Glu Leu
545 550 555 560

Thr Asn Cys Thr Ser Ala Ala Cys Lys Leu Asn Phe Asp Phe Ser Ser
565 570 575

Cys Glu Ser Ser Pro Cys Ser Tyr Gly Leu Met Asn Asn Phe Gln Val
580 585 590

Met Ser Met Val Ser Gly Phe Thr Pro Leu Ile Ser Ala Gly Ile Phe
595 600 605

Ser Ala Thr Leu Ser Ser Ala Leu Ala Ser Leu Val Ser Ala Pro Lys

610 615 620

Ile Phe Gln Ala Leu Cys Lys Asp Asn Ile Tyr Pro Ala Phe Gln Met
625 630 635 640

Phe Ala Lys Gly Tyr Gly Lys Asn Asn Glu Pro Leu Arg Gly Tyr Ile
645 650 655

Leu Thr Phe Leu Ile Ala Leu Gly Phe Ile Leu Ile Ala Glu Leu Asn
660 665 670

Val Ile Ala Pro Ile Ile Ser Asn Phe Phe Leu Ala Ser Tyr Ala Leu
675 680 685

Ile Asn Phe Ser Val Phe His Ala Ser Leu Ala Lys Ser Pro Gly Trp
690 695 700

Arg Pro Ala Phe Lys Tyr Tyr Asn Met Trp Ile Ser Leu Leu Gly Ala
705 710 715 720

Ile Leu Cys Cys Ile Val Met Phe Val Ile Asn Trp Trp Ala Ala Leu
725 730 735

Leu Thr Tyr Val Ile Val Leu Gly Leu Tyr Ile Tyr Val Thr Tyr Lys
740 745 750

Lys Pro Asp Val Asn Trp Gly Ser Ser Thr Gln Ala Leu Thr Tyr Leu
755 760 765

Asn Ala Leu Gln His Ser Ile Arg Leu Ser Gly Val Glu Asp His Val
770 775 780

Lys Asn Phe Arg Pro Gln Cys Leu Val Met Thr Gly Ala Pro Asn Ser
785 790 795 800

Arg Pro Ala Leu Leu His Leu Val His Asp Phe Thr Lys Asn Val Gly
805 810 815

Leu Met Ile Cys Gly His Val His Met Gly Pro Arg Arg Gln Ala Met
820 825 830

Lys Glu Met Ser Ile Asp Gln Ala Lys Tyr Gln Arg Trp Leu Ile Lys
835 840 845

Asn Lys Met Lys Ala Phe Tyr Ala Pro Val His Ala Asp Asp Leu Arg
850 855 860

Glu Gly Ala Gln Tyr Leu Met Gln Ala Ala Gly Leu Gly Arg Met Lys
865 870 875 880

Pro Asn Thr Leu Val Leu Gly Phe Lys Lys Asp Trp Leu Gln Ala Asp
885 890 895

Met Arg Asp Val Asp Met Tyr Ile Asn Leu Phe His Asp Ala Phe Asp
900 905 910

Ile Gln Tyr Gly Val Val Val Ile Arg Leu Lys Glu Gly Leu Asp Ile
915 920 925

Ser His Leu Gln Gly Gln Glu Glu Leu Leu Ser Ser Gln Glu Lys Ser
930 935 940

Pro Gly Thr Lys Asp Val Val Val Ser Val Glu Tyr Ser Lys Lys Ser
945 950 955 960

Asp Leu Asp Thr Ser Lys Pro Leu Ser Glu Lys Pro Ile Thr His Lys
965 970 975

Val Glu Glu Glu Asp Gly Lys Thr Ala Thr Gln Pro Leu Leu Lys Lys
980 985 990

Glu Ser Lys Gly Pro Ile Val Pro Leu Asn Val Ala Asp Gln Lys Leu
995 1000 1005

Leu Glu Ala Ser Thr Gln Phe Gln Lys Lys Gln Gly Lys Asn Thr
1010 1015 1020

Ile Asp Val Trp Trp Leu Phe Asp Asp Gly Gly Leu Thr Leu Leu
1025 1030 1035

Ile Pro Tyr Leu Leu Thr Thr Lys Lys Lys Trp Lys Asp Cys Lys
1040 1045 1050

Ile Arg Val Phe Ile Gly Gly Lys Ile Asn Arg Ile Asp His Asp
1055 1060 1065

Arg Arg Ala Met Ala Thr Leu Leu Ser Lys Phe Arg Ile Asp Phe
1070 1075 1080

Ser Asp Ile Met Val Leu Gly Asp Ile Asn Thr Lys Pro Lys Lys
1085 1090 1095

Glu Asn Ile Ile Ala Phe Glu Glu Ile Ile Glu Pro Tyr Arg Leu
1100 1105 1110

His Glu Asp Asp Lys Glu Gln Asp Ile Ala Asp Lys Met Lys Glu
1115 1120 1125

Asp Glu Pro Trp Arg Ile Thr Asp Asn Glu Leu Glu Leu Tyr Lys
1130 1135 1140

Thr Lys Thr Tyr Arg Gln Ile Arg Leu Asn Glu Leu Leu Lys Glu
1145 1150 1155

His Ser Ser Thr Ala Asn Ile Ile Val Met Ser Leu Pro Val Ala
1160 1165 1170

Arg Lys Gly Ala Val Ser Ser Ala Leu Tyr Met Ala Trp Leu Glu
1175 1180 1185

Ala Leu Ser Lys Asp Leu Pro Pro Ile Leu Leu Val Arg Gly Asn
1190 1195 1200

His Gln Ser Val Leu Thr Phe Tyr Ser
1205 1210

<210> 9
<211> 836
<212> PRT
<213> Homo sapiens

<400> 9

Met Ala Gly Leu Asn Cys Gly Val Ser Ile Ala Leu Leu Gly Val Leu
1 5 10 15

Leu Leu Gly Ala Ala Arg Leu Pro Arg Gly Ala Glu Ala Phe Glu Ile
20 25 30

Ala Leu Pro Arg Glu Ser Asn Ile Thr Val Leu Ile Lys Leu Gly Thr
35 40 45

Pro Thr Leu Leu Ala Lys Pro Cys Tyr Ile Val Ile Ser Lys Arg His
50 55 60

Ile Thr Met Leu Ser Ile Lys Ser Gly Glu Arg Ile Val Phe Thr Phe
65 70 75 80

Ser Cys Gln Ser Pro Glu Asn His Phe Val Ile Glu Ile Gln Lys Asn
85 90 95

Ile Asp Cys Met Ser Gly Pro Cys Pro Phe Gly Glu Val Gln Leu Gln
100 105 110

Pro Ser Thr Ser Leu Leu Pro Thr Leu Asn Arg Thr Phe Ile Trp Asp
115 120 125

Val Lys Ala His Lys Ser Ile Gly Leu Glu Leu Gln Phe Ser Ile Pro
130 135 140

Arg Leu Arg Gln Ile Gly Pro Gly Glu Ser Cys Pro Asp Gly Val Thr
145 150 155 160

His Ser Ile Ser Gly Arg Ile Asp Ala Thr Val Val Arg Ile Gly Thr
165 170 175

Phe Cys Ser Asn Gly Thr Val Ser Arg Ile Lys Met Gln Glu Gly Val
180 185 190

Lys Met Ala Leu His Leu Pro Trp Phe His Pro Arg Asn Val Ser Gly
195 200 205

Phe Ser Ile Ala Asn Arg Ser Ser Ile Lys Arg Leu Cys Ile Ile Glu
210 215 220

Ser Val Phe Glu Gly Glu Gly Ser Ala Thr Leu Met Ser Ala Asn Tyr
225 230 235 240

Pro Glu Gly Phe Pro Glu Asp Glu Leu Met Thr Trp Gln Phe Val Val
245 250 255

Pro Ala His Leu Arg Ala Ser Val Ser Phe Leu Asn Phe Asn Leu Ser
260 265 270

Asn Cys Glu Arg Lys Glu Glu Arg Val Glu Tyr Tyr Ile Pro Gly Ser
275 280 285

Thr Thr Asn Pro Glu Val Phe Lys Leu Glu Asp Lys Gln Pro Gly Asn
290 295 300

Met Ala Gly Asn Phe Asn Leu Ser Leu Gln Gly Cys Asp Gln Asp Ala
305 310 315 320

Gln Ser Pro Gly Ile Leu Arg Leu Gln Phe Gln Val Leu Val Gln His
325 330 335

Pro Gln Asn Glu Ser Asn Lys Ile Tyr Val Val Asp Leu Ser Asn Glu
340 345 350

Arg Ala Met Ser Leu Thr Ile Glu Pro Arg Pro Val Lys Gln Ser Arg
355 360 365

Lys Phe Val Pro Gly Cys Phe Val Cys Leu Glu Ser Arg Thr Cys Ser
370 375 380

Ser Asn Leu Thr Leu Thr Ser Gly Ser Lys His Lys Ile Ser Phe Leu
385 390 395 400

Cys Asp Asp Leu Thr Arg Leu Trp Met Asn Val Glu Lys Thr Ile Ser
405 410 415

Cys Thr Asp His Arg Tyr Cys Gln Arg Lys Ser Tyr Ser Leu Gln Val
420 425 430

Pro Ser Asp Ile Leu His Leu Pro Val Glu Leu His Asp Phe Ser Trp
435 440 445

Lys Leu Leu Val Pro Lys Asp Arg Leu Ser Leu Val Leu Val Pro Ala
450 455 460

Gln Lys Leu Gln Gln His Thr His Glu Lys Pro Cys Asn Thr Ser Phe
465 470 475 480

Ser Tyr Leu Val Ala Ser Ala Ile Pro Ser Gln Asp Leu Tyr Phe Gly
485 490 495

Ser Phe Cys Pro Gly Gly Ser Ile Lys Gln Ile Gln Val Lys Gln Asn
500 505 510

Ile Ser Val Thr Leu Arg Thr Phe Ala Pro Ser Phe Gln Gln Glu Ala
515 520 525

Ser Arg Gln Gly Leu Thr Val Ser Phe Ile Pro Tyr Phe Lys Glu Glu
530 535 540

Gly Val Phe Thr Val Thr Pro Asp Thr Lys Ser Lys Val Tyr Leu Arg
545 550 555 560

Thr Pro Asn Trp Asp Arg Gly Leu Pro Ser Leu Thr Ser Val Ser Trp
565 570 575

Asn Ile Ser Val Pro Arg Asp Gln Val Ala Cys Leu Thr Phe Phe Lys
580 585 590

Glu Arg Ser Gly Val Val Cys Gln Thr Gly Arg Ala Phe Met Ile Ile
595 600 605

Gln Glu Gln Arg Thr Arg Ala Glu Glu Ile Phe Ser Leu Asp Glu Asp

610 615 620

Val Leu Pro Lys Pro Ser Phe His His His Ser Phe Trp Val Asn Ile
625 630 635 640

Ser Asn Cys Ser Pro Thr Ser Gly Lys Gln Leu Asp Leu Leu Phe Ser
645 650 655

Val Thr Leu Thr Pro Arg Thr Val Asp Leu Thr Val Ile Leu Ile Ala
660 665 670

Ala Val Gly Gly Gly Val Leu Leu Leu Ser Ala Leu Gly Leu Ile Ile
675 680 685

Cys Cys Val Lys Lys Lys Lys Lys Lys Thr Asn Lys Gly Pro Ala Val
690 695 700

Gly Ile Tyr Asn Asp Asn Ile Asn Thr Glu Met Pro Arg Gln Pro Lys
705 710 715 720

Lys Phe Gln Lys Gly Arg Lys Asp Asn Asp Ser His Val Tyr Ala Val
725 730 735

Ile Glu Asp Thr Met Val Tyr Gly His Leu Leu Gln Asp Ser Ser Gly
740 745 750

Ser Phe Leu Gln Pro Glu Val Asp Thr Tyr Arg Pro Phe Gln Gly Thr
755 760 765

Met Gly Val Cys Pro Pro Ser Pro Pro Thr Ile Cys Ser Arg Ala Pro
770 775 780

Thr Ala Lys Leu Ala Thr Glu Glu Pro Pro Pro Arg Ser Pro Pro Glu
785 790 795 800

Ser Glu Ser Glu Pro Tyr Thr Phe Ser His Pro Asn Asn Gly Asp Val
805 810 815

Ser Ser Lys Asp Thr Asp Ile Pro Leu Leu Asn Thr Gln Glu Pro Met
820 825 830

Glu Pro Ala Glu
835

<210> 10
<211> 702
<212> PRT
<213> Homo sapiens

<400> 10

```
Met Glu Ser Pro Ser Ala Pro Pro His Arg Trp Cys Ile Pro Trp Gln
1               5                   10                  15

Arg Leu Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Val His Asn Leu Pro Gln His Leu Phe Gly
    50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240
```

```
Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300

Asp Thr Gly Leu Asn Arg Thr Thr Val Thr Thr Ile Thr Val Tyr Ala
305                 310                 315                 320

Glu Pro Pro Lys Pro Phe Ile Thr Ser Asn Asn Ser Asn Pro Val Glu
                325                 330                 335

Asp Glu Asp Ala Val Ala Leu Thr Cys Glu Pro Glu Ile Gln Asn Thr
            340                 345                 350

Thr Tyr Leu Trp Trp Val Asn Asn Gln Ser Leu Pro Val Ser Pro Arg
        355                 360                 365

Leu Gln Leu Ser Asn Asp Asn Arg Thr Leu Thr Leu Leu Ser Val Thr
    370                 375                 380

Arg Asn Asp Val Gly Pro Tyr Glu Cys Gly Ile Gln Asn Glu Leu Ser
385                 390                 395                 400

Val Asp His Ser Asp Pro Val Ile Leu Asn Val Leu Tyr Gly Pro Asp
                405                 410                 415

Asp Pro Thr Ile Ser Pro Ser Tyr Thr Tyr Tyr Arg Pro Gly Val Asn
            420                 425                 430

Leu Ser Leu Ser Cys His Ala Ala Ser Asn Pro Pro Ala Gln Tyr Ser
        435                 440                 445

Trp Leu Ile Asp Gly Asn Ile Gln Gln His Thr Gln Glu Leu Phe Ile
    450                 455                 460

Ser Asn Ile Thr Glu Lys Asn Ser Gly Leu Tyr Thr Cys Gln Ala Asn
465                 470                 475                 480

Asn Ser Ala Ser Gly His Ser Arg Thr Thr Val Lys Thr Ile Thr Val
                485                 490                 495
```

Ser Ala Glu Leu Pro Lys Pro Ser Ile Ser Ser Asn Asn Ser Lys Pro
500 505 510

Val Glu Asp Lys Asp Ala Val Ala Phe Thr Cys Glu Pro Glu Ala Gln
515 520 525

Asn Thr Thr Tyr Leu Trp Trp Val Asn Gly Gln Ser Leu Pro Val Ser
530 535 540

Pro Arg Leu Gln Leu Ser Asn Gly Asn Arg Thr Leu Thr Leu Phe Asn
545 550 555 560

Val Thr Arg Asn Asp Ala Arg Ala Tyr Val Cys Gly Ile Gln Asn Ser
565 570 575

Val Ser Ala Asn Arg Ser Asp Pro Val Thr Leu Asp Val Leu Tyr Gly
580 585 590

Pro Asp Thr Pro Ile Ile Ser Pro Pro Asp Ser Ser Tyr Leu Ser Gly
595 600 605

Ala Asn Leu Asn Leu Ser Cys His Ser Ala Ser Asn Pro Ser Pro Gln
610 615 620

Tyr Ser Trp Arg Ile Asn Gly Ile Pro Gln Gln His Thr Gln Val Leu
625 630 635 640

Phe Ile Ala Lys Ile Thr Pro Asn Asn Asn Gly Thr Tyr Ala Cys Phe
645 650 655

Val Ser Asn Leu Ala Thr Gly Arg Asn Asn Ser Ile Val Lys Ser Ile
660 665 670

Thr Val Ser Ala Ser Gly Thr Ser Pro Gly Leu Ser Ala Gly Ala Thr
675 680 685

Val Gly Ile Met Ile Gly Val Leu Val Gly Val Ala Leu Ile
690 695 700

<210> 11
<211> 510
<212> PRT
<213> Homo sapiens

<400> 11

Met Pro Leu Ser Leu Gly Ala Glu Met Trp Gly Pro Glu Ala Trp Leu
1 5 10 15

```
Leu Leu Leu Leu Leu Leu Ala Ser Phe Thr Gly Arg Cys Pro Ala Gly
            20                  25                  30

Glu Leu Glu Thr Ser Asp Val Val Thr Val Val Leu Gly Gln Asp Ala
        35                  40                  45

Lys Leu Pro Cys Phe Tyr Arg Gly Asp Ser Gly Glu Gln Val Gly Gln
    50                  55                  60

Val Ala Trp Ala Arg Val Asp Ala Gly Glu Gly Ala Gln Glu Leu Ala
65                  70                  75                  80

Leu Leu His Ser Lys Tyr Gly Leu His Val Ser Pro Ala Tyr Glu Gly
                85                  90                  95

Arg Val Glu Gln Pro Pro Pro Pro Arg Asn Pro Leu Asp Gly Ser Val
            100                 105                 110

Leu Leu Arg Asn Ala Val Gln Ala Asp Glu Gly Glu Tyr Glu Cys Arg
        115                 120                 125

Val Ser Thr Phe Pro Ala Gly Ser Phe Gln Ala Arg Leu Arg Leu Arg
    130                 135                 140

Val Leu Val Pro Pro Leu Pro Ser Leu Asn Pro Gly Pro Ala Leu Glu
145                 150                 155                 160

Glu Gly Gln Gly Leu Thr Leu Ala Ala Ser Cys Thr Ala Glu Gly Ser
                165                 170                 175

Pro Ala Pro Ser Val Thr Trp Asp Thr Glu Val Lys Gly Thr Thr Ser
            180                 185                 190

Ser Arg Ser Phe Lys His Ser Arg Ser Ala Ala Val Thr Ser Glu Phe
        195                 200                 205

His Leu Val Pro Ser Arg Ser Met Asn Gly Gln Pro Leu Thr Cys Val
    210                 215                 220

Val Ser His Pro Gly Leu Leu Gln Asp Gln Arg Ile Thr His Ile Leu
225                 230                 235                 240

His Val Ser Phe Leu Ala Glu Ala Ser Val Arg Gly Leu Glu Asp Gln
                245                 250                 255

Asn Leu Trp His Ile Gly Arg Glu Gly Ala Met Leu Lys Cys Leu Ser
            260                 265                 270
```

```
Glu Gly Gln Pro Pro Pro Ser Tyr Asn Trp Thr Arg Leu Asp Gly Pro
        275                 280                 285

Leu Pro Ser Gly Val Arg Val Asp Gly Asp Thr Leu Gly Phe Pro Pro
    290                 295                 300

Leu Thr Thr Glu His Ser Gly Ile Tyr Val Cys His Val Ser Asn Glu
305                 310                 315                 320

Phe Ser Ser Arg Asp Ser Gln Val Thr Val Asp Val Leu Asp Pro Gln
                325                 330                 335

Glu Asp Ser Gly Lys Gln Val Asp Leu Val Ser Ala Ser Val Val Val
            340                 345                 350

Val Gly Val Ile Ala Ala Leu Leu Phe Cys Leu Leu Val Val Val Val
        355                 360                 365

Val Leu Met Ser Arg Tyr His Arg Arg Lys Ala Gln Gln Met Thr Gln
    370                 375                 380

Lys Tyr Glu Glu Glu Leu Thr Leu Thr Arg Glu Asn Ser Ile Arg Arg
385                 390                 395                 400

Leu His Ser His His Thr Asp Pro Arg Ser Gln Pro Glu Glu Ser Val
                405                 410                 415

Gly Leu Arg Ala Glu Gly His Pro Asp Ser Leu Lys Asp Asn Ser Ser
            420                 425                 430

Cys Ser Val Met Ser Glu Glu Pro Glu Gly Arg Ser Tyr Ser Thr Leu
        435                 440                 445

Thr Thr Val Arg Glu Ile Glu Thr Gln Thr Glu Leu Leu Ser Pro Gly
    450                 455                 460

Ser Gly Arg Ala Glu Glu Glu Glu Asp Gln Asp Glu Gly Ile Lys Gln
465                 470                 475                 480

Ala Met Asn His Phe Val Gln Glu Asn Gly Thr Leu Arg Ala Lys Pro
                485                 490                 495

Thr Gly Asn Gly Ile Tyr Ile Asn Gly Arg Gly His Leu Val
            500                 505                 510
```

<210> 12

<211> 910
<212> PRT
<213> Homo sapiens

<400> 12

Met Gly Thr Pro Arg Ala Gln His Pro Pro Pro Pro Gln Leu Leu Phe
1 5 10 15

Leu Ile Leu Leu Ser Cys Pro Trp Ile Gln Gly Leu Pro Leu Lys Glu
20 25 30

Glu Glu Ile Leu Pro Glu Pro Gly Ser Glu Thr Pro Thr Val Ala Ser
35 40 45

Glu Ala Leu Ala Glu Leu Leu His Gly Ala Leu Leu Arg Arg Gly Pro
50 55 60

Glu Met Gly Tyr Leu Pro Gly Ser Asp Arg Asp Pro Thr Leu Ala Thr
65 70 75 80

Pro Pro Ala Gly Gln Thr Leu Ala Val Pro Ser Leu Pro Arg Ala Thr
85 90 95

Glu Pro Gly Thr Gly Pro Leu Thr Thr Ala Val Thr Pro Asn Gly Val
100 105 110

Arg Gly Ala Gly Pro Thr Ala Pro Glu Leu Leu Thr Pro Pro Pro Gly
115 120 125

Thr Thr Ala Pro Pro Pro Pro Ser Pro Ala Ser Pro Gly Pro Pro Leu
130 135 140

Gly Pro Glu Gly Gly Glu Glu Glu Thr Thr Thr Thr Ile Ile Thr Thr
145 150 155 160

Thr Thr Val Thr Thr Thr Val Thr Ser Pro Val Leu Cys Asn Asn Asn
165 170 175

Ile Ser Glu Gly Glu Gly Tyr Val Glu Ser Pro Asp Leu Gly Ser Pro
180 185 190

Val Ser Arg Thr Leu Gly Leu Leu Asp Cys Thr Tyr Ser Ile His Val
195 200 205

Tyr Pro Gly Tyr Gly Ile Glu Ile Gln Val Gln Thr Leu Asn Leu Ser
210 215 220

Gln Glu Glu Glu Leu Leu Val Leu Ala Gly Gly Gly Ser Pro Gly Leu

```
225                 230                 235                 240

Ala Pro Arg Leu Leu Ala Asn Ser Ser Met Leu Gly Glu Gly Gln Val
                245                 250                 255

Leu Arg Ser Pro Thr Asn Arg Leu Leu Leu His Phe Gln Ser Pro Arg
            260                 265                 270

Val Pro Arg Gly Gly Gly Phe Arg Ile His Tyr Gln Ala Tyr Leu Leu
        275                 280                 285

Ser Cys Gly Phe Pro Pro Arg Pro Ala His Gly Asp Val Ser Val Thr
    290                 295                 300

Asp Leu His Pro Gly Gly Thr Ala Thr Phe His Cys Asp Ser Gly Tyr
305                 310                 315                 320

Gln Leu Gln Gly Glu Glu Thr Leu Ile Cys Leu Asn Gly Thr Arg Pro
                325                 330                 335

Ser Trp Asn Gly Glu Thr Pro Ser Cys Met Ala Ser Cys Gly Gly Thr
            340                 345                 350

Ile His Asn Ala Thr Leu Gly Arg Ile Val Ser Pro Glu Pro Gly Gly
        355                 360                 365

Ala Val Gly Pro Asn Leu Thr Cys Arg Trp Val Ile Glu Ala Ala Glu
    370                 375                 380

Gly Arg Arg Leu His Leu His Phe Glu Arg Val Ser Leu Asp Glu Asp
385                 390                 395                 400

Asn Asp Arg Leu Met Val Arg Ser Gly Gly Ser Pro Leu Ser Pro Val
                405                 410                 415

Ile Tyr Asp Ser Asp Met Asp Asp Val Pro Glu Arg Gly Leu Ile Ser
            420                 425                 430

Asp Ala Gln Ser Leu Tyr Val Glu Leu Leu Ser Glu Thr Pro Ala Asn
        435                 440                 445

Pro Leu Leu Leu Ser Leu Arg Phe Glu Ala Phe Glu Glu Asp Arg Cys
    450                 455                 460

Phe Ala Pro Phe Leu Ala His Gly Asn Val Thr Thr Thr Asp Pro Glu
465                 470                 475                 480
```

Tyr Arg Pro Gly Ala Leu Ala Thr Phe Ser Cys Leu Pro Gly Tyr Ala
485 490 495

Leu Glu Pro Pro Gly Pro Pro Asn Ala Ile Glu Cys Val Asp Pro Thr
500 505 510

Glu Pro His Trp Asn Asp Thr Glu Pro Ala Cys Lys Ala Met Cys Gly
515 520 525

Gly Glu Leu Ser Glu Pro Ala Gly Val Val Leu Ser Pro Asp Trp Pro
530 535 540

Gln Ser Tyr Ser Pro Gly Gln Asp Cys Val Trp Gly Val His Val Gln
545 550 555 560

Glu Glu Lys Arg Ile Leu Leu Gln Val Glu Ile Leu Asn Val Arg Glu
565 570 575

Gly Asp Met Leu Thr Leu Phe Asp Gly Asp Gly Pro Ser Ala Arg Val
580 585 590

Leu Ala Gln Leu Arg Gly Pro Gln Pro Arg Arg Arg Leu Leu Ser Ser
595 600 605

Gly Pro Asp Leu Thr Leu Gln Phe Gln Ala Pro Pro Gly Pro Pro Asn
610 615 620

Pro Gly Leu Gly Gln Gly Phe Val Leu His Phe Lys Glu Val Pro Arg
625 630 635 640

Asn Asp Thr Cys Pro Glu Leu Pro Pro Pro Glu Trp Gly Trp Arg Thr
645 650 655

Ala Ser His Gly Asp Leu Ile Arg Gly Thr Val Leu Thr Tyr Gln Cys
660 665 670

Glu Pro Gly Tyr Glu Leu Leu Gly Ser Asp Ile Leu Thr Cys Gln Trp
675 680 685

Asp Leu Ser Trp Ser Ala Ala Pro Pro Ala Cys Gln Lys Ile Met Thr
690 695 700

Cys Ala Asp Pro Gly Glu Ile Ala Asn Gly His Arg Thr Ala Ser Asp
705 710 715 720

Ala Gly Phe Pro Val Gly Ser His Val Gln Tyr Arg Cys Leu Pro Gly
725 730 735

Tyr Ser Leu Glu Gly Ala Ala Met Leu Thr Cys Tyr Ser Arg Asp Thr
740 745 750

Gly Thr Pro Lys Trp Ser Asp Arg Val Pro Lys Cys Ala Leu Lys Tyr
755 760 765

Glu Pro Cys Leu Asn Pro Gly Val Pro Glu Asn Gly Tyr Gln Thr Leu
770 775 780

Tyr Lys His His Tyr Gln Ala Gly Glu Ser Leu Arg Phe Phe Cys Tyr
785 790 795 800

Glu Gly Phe Glu Leu Ile Gly Glu Val Thr Ile Thr Cys Val Pro Gly
805 810 815

His Pro Ser Gln Trp Thr Ser Gln Pro Pro Leu Cys Lys Val Thr Gln
820 825 830

Thr Thr Asp Pro Ser Arg Gln Leu Glu Gly Gly Asn Leu Ala Leu Ala
835 840 845

Ile Leu Leu Pro Leu Gly Leu Val Ile Val Leu Gly Ser Gly Val Tyr
850 855 860

Ile Tyr Tyr Thr Lys Leu Gln Gly Lys Ser Leu Phe Gly Phe Ser Gly
865 870 875 880

Ser His Ser Tyr Ser Pro Ile Thr Val Glu Ser Asp Phe Ser Asn Pro
885 890 895

Leu Tyr Glu Ala Gly Asp Thr Arg Glu Tyr Glu Val Ser Ile
900 905 910

<210> 13
<211> 555
<212> PRT
<213> Homo sapiens

<400> 13

Met Ala Arg Pro Asp Asp Glu Glu Gly Ala Ala Val Ala Pro Gly His
1 5 10 15

Pro Leu Ala Lys Gly Tyr Leu Pro Leu Pro Arg Gly Ala Pro Val Gly
20 25 30

Lys Glu Ser Val Glu Leu Gln Asn Gly Pro Lys Ala Gly Thr Phe Pro
35 40 45

```
Val Asn Gly Ala Pro Arg Asp Ser Leu Ala Ala Ala Ser Gly Val Leu
    50                  55                  60

Gly Gly Pro Gln Thr Pro Leu Ala Pro Glu Glu Glu Thr Gln Ala Arg
65                  70                  75                  80

Leu Leu Pro Ala Gly Ala Gly Ala Glu Thr Pro Gly Ala Glu Ser Ser
                85                  90                  95

Pro Leu Pro Leu Thr Ala Leu Ser Pro Arg Arg Phe Val Val Leu Leu
            100                 105                 110

Ile Phe Ser Leu Tyr Ser Leu Val Asn Ala Phe Gln Trp Ile Gln Tyr
        115                 120                 125

Ser Ile Ile Ser Asn Val Phe Glu Gly Phe Tyr Gly Val Thr Leu Leu
    130                 135                 140

His Ile Asp Trp Leu Ser Met Val Tyr Met Leu Ala Tyr Val Pro Leu
145                 150                 155                 160

Ile Phe Pro Ala Thr Trp Leu Leu Asp Thr Arg Gly Leu Arg Leu Thr
                165                 170                 175

Ala Leu Leu Gly Ser Gly Leu Asn Cys Leu Gly Ala Trp Ile Lys Cys
            180                 185                 190

Gly Ser Val Gln Gln His Leu Phe Trp Val Thr Met Leu Gly Gln Cys
        195                 200                 205

Leu Cys Ser Val Ala Gln Val Phe Ile Leu Gly Leu Pro Ser Arg Ile
    210                 215                 220

Ala Ser Val Trp Phe Gly Pro Lys Glu Val Ser Thr Ala Cys Ala Thr
225                 230                 235                 240

Ala Val Leu Gly Asn Gln Leu Gly Thr Ala Val Gly Phe Leu Leu Pro
                245                 250                 255

Pro Val Leu Val Pro Asn Thr Gln Asn Asp Thr Asn Leu Leu Ala Cys
            260                 265                 270

Asn Ile Ser Thr Met Phe Tyr Gly Thr Ser Ala Val Ala Thr Leu Leu
        275                 280                 285

Phe Ile Leu Thr Ala Ile Ala Phe Lys Glu Lys Pro Arg Tyr Pro Pro
    290                 295                 300
```

Ser Gln Ala Gln Ala Ala Leu Gln Asp Ser Pro Pro Glu Glu Tyr Ser
305 310 315 320

Tyr Lys Lys Ser Ile Arg Asn Leu Phe Lys Asn Ile Pro Phe Val Leu
325 330 335

Leu Leu Ile Thr Tyr Gly Ile Met Thr Gly Ala Phe Tyr Ser Val Ser
340 345 350

Thr Leu Leu Asn Gln Met Ile Leu Thr Tyr Tyr Glu Gly Glu Glu Val
355 360 365

Asn Ala Gly Arg Ile Gly Leu Thr Leu Val Val Ala Gly Met Val Gly
370 375 380

Ser Ile Leu Cys Gly Leu Trp Leu Asp Tyr Thr Lys Thr Tyr Lys Gln
385 390 395 400

Thr Thr Leu Ile Val Tyr Ile Leu Ser Phe Ile Gly Met Val Ile Phe
405 410 415

Thr Phe Thr Leu Asp Leu Arg Tyr Ile Ile Ile Val Phe Val Thr Gly
420 425 430

Gly Val Leu Gly Phe Phe Met Thr Gly Tyr Leu Pro Leu Gly Phe Glu
435 440 445

Phe Ala Val Glu Ile Thr Tyr Pro Glu Ser Glu Gly Thr Ser Ser Gly
450 455 460

Leu Leu Asn Ala Ser Ala Gln Ile Phe Gly Ile Leu Phe Thr Leu Ala
465 470 475 480

Gln Gly Lys Leu Thr Ser Asp Tyr Gly Pro Lys Ala Gly Asn Ile Phe
485 490 495

Leu Cys Val Trp Met Phe Ile Gly Ile Ile Leu Thr Ala Leu Ile Lys
500 505 510

Ser Asp Leu Arg Arg His Asn Ile Asn Ile Gly Ile Thr Asn Val Asp
515 520 525

Val Lys Ala Ile Pro Ala Asp Ser Pro Thr Asp Gln Glu Pro Lys Thr
530 535 540

Val Met Leu Ser Lys Gln Ser Glu Ser Ala Ile

545 550 555

<210> 14
<211> 507
<212> PRT
<213> Homo sapiens

<400> 14

Met Ala Gly Ala Gly Pro Lys Arg Arg Ala Leu Ala Ala Pro Ala Ala
1 5 10 15

Glu Glu Lys Glu Glu Ala Arg Glu Lys Met Leu Ala Ala Lys Ser Ala
20 25 30

Asp Gly Ser Ala Pro Ala Gly Glu Gly Glu Gly Val Thr Leu Gln Arg
35 40 45

Asn Ile Thr Leu Leu Asn Gly Val Ala Ile Ile Val Gly Thr Ile Ile
50 55 60

Gly Ser Gly Ile Phe Val Thr Pro Thr Gly Val Leu Lys Glu Ala Gly
65 70 75 80

Ser Pro Gly Leu Ala Leu Val Val Trp Ala Ala Cys Gly Val Phe Ser
85 90 95

Ile Val Gly Ala Leu Cys Tyr Ala Glu Leu Gly Thr Thr Ile Ser Lys
100 105 110

Ser Gly Gly Asp Tyr Ala Tyr Met Leu Glu Val Tyr Gly Ser Leu Pro
115 120 125

Ala Phe Leu Lys Leu Trp Ile Glu Leu Leu Ile Ile Arg Pro Ser Ser
130 135 140

Gln Tyr Ile Val Ala Leu Val Phe Ala Thr Tyr Leu Leu Lys Pro Leu
145 150 155 160

Phe Pro Thr Cys Pro Val Pro Glu Glu Ala Ala Lys Leu Val Ala Cys
165 170 175

Leu Cys Val Leu Leu Leu Thr Ala Val Asn Cys Tyr Ser Val Lys Ala
180 185 190

Ala Thr Arg Val Gln Asp Ala Phe Ala Ala Ala Lys Leu Leu Ala Leu
195 200 205

Ala Leu Ile Ile Leu Leu Gly Phe Val Gln Ile Gly Lys Gly Asp Val

```
        210                 215                 220

Ser Asn Leu Asp Pro Asn Phe Ser Phe Glu Gly Thr Lys Leu Asp Val
225                 230                 235                 240

Gly Asn Ile Val Leu Ala Leu Tyr Ser Gly Leu Phe Ala Tyr Gly Gly
                245                 250                 255

Trp Asn Tyr Leu Asn Phe Val Thr Glu Glu Met Ile Asn Pro Tyr Arg
            260                 265                 270

Asn Leu Pro Leu Ala Ile Ile Ile Ser Leu Pro Ile Val Thr Leu Val
        275                 280                 285

Tyr Val Leu Thr Asn Leu Ala Tyr Phe Thr Thr Leu Ser Thr Glu Gln
    290                 295                 300

Met Leu Ser Ser Glu Ala Val Ala Val Asp Phe Gly Asn Tyr His Leu
305                 310                 315                 320

Gly Val Met Ser Trp Ile Ile Pro Val Phe Val Gly Leu Ser Cys Phe
                325                 330                 335

Gly Ser Val Asn Gly Ser Leu Phe Thr Ser Ser Arg Leu Phe Phe Val
            340                 345                 350

Gly Ser Arg Glu Gly His Leu Pro Ser Ile Leu Ser Met Ile His Pro
        355                 360                 365

Gln Leu Leu Thr Pro Val Pro Ser Leu Val Phe Thr Cys Val Met Thr
    370                 375                 380

Leu Leu Tyr Ala Phe Ser Lys Asp Ile Phe Ser Val Ile Asn Phe Phe
385                 390                 395                 400

Ser Phe Phe Asn Trp Leu Cys Val Ala Leu Ala Ile Ile Gly Met Ile
                405                 410                 415

Trp Leu Arg His Arg Lys Pro Glu Leu Glu Arg Pro Ile Lys Val Asn
            420                 425                 430

Leu Ala Leu Pro Val Phe Phe Ile Leu Ala Cys Leu Phe Leu Ile Ala
        435                 440                 445

Val Ser Phe Trp Lys Thr Pro Val Glu Cys Gly Ile Gly Phe Thr Ile
    450                 455                 460
```

Ile Leu Ser Gly Leu Pro Val Tyr Phe Phe Gly Val Trp Trp Lys Asn
465 470 475 480

Lys Pro Lys Trp Leu Leu Gln Gly Ile Phe Ser Thr Thr Val Leu Cys
485 490 495

Gln Lys Leu Met Gln Val Val Pro Gln Glu Thr
500 505

<210> 15
<211> 339
<212> PRT
<213> Homo sapiens

<400> 15

Met Glu Ser Arg Lys Asp Ile Thr Asn Gln Glu Glu Leu Trp Lys Met
1 5 10 15

Lys Pro Arg Arg Asn Leu Glu Glu Asp Asp Tyr Leu His Lys Asp Thr
20 25 30

Gly Glu Thr Ser Met Leu Lys Arg Pro Val Leu Leu His Leu His Gln
35 40 45

Thr Ala His Ala Asp Glu Phe Asp Cys Pro Ser Glu Leu Gln His Thr
50 55 60

Gln Glu Leu Phe Pro Gln Trp His Leu Pro Ile Lys Ile Ala Ala Ile
65 70 75 80

Ile Ala Ser Leu Thr Phe Leu Tyr Thr Leu Leu Arg Glu Val Ile His
85 90 95

Pro Leu Ala Thr Ser His Gln Gln Tyr Phe Tyr Lys Ile Pro Ile Leu
100 105 110

Val Ile Asn Lys Val Leu Pro Met Val Ser Ile Thr Leu Leu Ala Leu
115 120 125

Val Tyr Leu Pro Gly Val Ile Ala Ala Ile Val Gln Leu His Asn Gly
130 135 140

Thr Lys Tyr Lys Lys Phe Pro His Trp Leu Asp Lys Trp Met Leu Thr
145 150 155 160

Arg Lys Gln Phe Gly Leu Leu Ser Phe Phe Phe Ala Val Leu His Ala
165 170 175

Ile Tyr Ser Leu Ser Tyr Pro Met Arg Arg Ser Tyr Arg Tyr Lys Leu
180 185 190

Leu Asn Trp Ala Tyr Gln Gln Val Gln Gln Asn Lys Glu Asp Ala Trp
195 200 205

Ile Glu His Asp Val Trp Arg Met Glu Ile Tyr Val Ser Leu Gly Ile
210 215 220

Val Gly Leu Ala Ile Leu Ala Leu Leu Ala Val Thr Ser Ile Pro Ser
225 230 235 240

Val Ser Asp Ser Leu Thr Trp Arg Glu Phe His Tyr Ile Gln Ser Lys
245 250 255

Leu Gly Ile Val Ser Leu Leu Leu Gly Thr Ile His Ala Leu Ile Phe
260 265 270

Ala Trp Asn Lys Trp Ile Asp Ile Lys Gln Phe Val Trp Tyr Thr Pro
275 280 285

Pro Thr Phe Met Ile Ala Val Phe Leu Pro Ile Val Val Leu Ile Phe
290 295 300

Lys Ser Ile Leu Phe Leu Pro Cys Leu Arg Lys Lys Ile Leu Lys Ile
305 310 315 320

Arg His Gly Trp Glu Asp Val Thr Lys Ile Asn Lys Thr Glu Ile Cys
325 330 335

Ser Gln Leu

<210> 16
<211> 582
<212> PRT
<213> Homo sapiens

<400> 16

Met Ser Pro Ala Pro Arg Pro Pro Arg Cys Leu Leu Leu Pro Leu Leu
1 5 10 15

Thr Leu Gly Thr Ala Leu Ala Ser Leu Gly Ser Ala Gln Ser Ser Ser
20 25 30

Phe Ser Pro Glu Ala Trp Leu Gln Gln Tyr Gly Tyr Leu Pro Pro Gly
35 40 45

```
Asp Leu Arg Thr His Thr Gln Arg Ser Pro Gln Ser Leu Ser Ala Ala
    50                  55                  60

Ile Ala Ala Met Gln Lys Phe Tyr Gly Leu Gln Val Thr Gly Lys Ala
65                  70                  75                  80

Asp Ala Asp Thr Met Lys Ala Met Arg Arg Pro Arg Cys Gly Val Pro
                85                  90                  95

Asp Lys Phe Gly Ala Glu Ile Lys Ala Asn Val Arg Arg Lys Arg Tyr
            100                 105                 110

Ala Ile Gln Gly Leu Lys Trp Gln His Asn Glu Ile Thr Phe Cys Ile
        115                 120                 125

Gln Asn Tyr Thr Pro Lys Val Gly Glu Tyr Ala Thr Tyr Glu Ala Ile
    130                 135                 140

Arg Lys Ala Phe Arg Val Trp Glu Ser Ala Thr Pro Leu Arg Phe Arg
145                 150                 155                 160

Glu Val Pro Tyr Ala Tyr Ile Arg Glu Gly His Glu Lys Gln Ala Asp
                165                 170                 175

Ile Met Ile Phe Phe Ala Glu Gly Phe His Gly Asp Ser Thr Pro Phe
            180                 185                 190

Asp Gly Glu Gly Gly Phe Leu Ala His Ala Tyr Phe Pro Gly Pro Asn
        195                 200                 205

Ile Gly Gly Asp Thr His Phe Asp Ser Ala Glu Pro Trp Thr Val Arg
    210                 215                 220

Asn Glu Asp Leu Asn Gly Asn Asp Ile Phe Leu Val Ala Val His Glu
225                 230                 235                 240

Leu Gly His Ala Leu Gly Leu Glu His Ser Ser Asp Pro Ser Ala Ile
                245                 250                 255

Met Ala Pro Phe Tyr Gln Trp Met Asp Thr Glu Asn Phe Val Leu Pro
            260                 265                 270

Asp Asp Asp Arg Arg Gly Ile Gln Gln Leu Tyr Gly Gly Glu Ser Gly
        275                 280                 285

Phe Pro Thr Lys Met Pro Pro Gln Pro Arg Thr Thr Ser Arg Pro Ser
    290                 295                 300
```

Val Pro Asp Lys Pro Lys Asn Pro Thr Tyr Gly Pro Asn Ile Cys Asp
305 310 315 320

Gly Asn Phe Asp Thr Val Ala Met Leu Arg Gly Glu Met Phe Val Phe
325 330 335

Lys Glu Arg Trp Phe Trp Arg Val Arg Asn Asn Gln Val Met Asp Gly
340 345 350

Tyr Pro Met Pro Ile Gly Gln Phe Trp Arg Gly Leu Pro Ala Ser Ile
355 360 365

Asn Thr Ala Tyr Glu Arg Lys Asp Gly Lys Phe Val Phe Phe Lys Gly
370 375 380

Asp Lys His Trp Val Phe Asp Glu Ala Ser Leu Glu Pro Gly Tyr Pro
385 390 395 400

Lys His Ile Lys Glu Leu Gly Arg Gly Leu Pro Thr Asp Lys Ile Asp
405 410 415

Ala Ala Leu Phe Trp Met Pro Asn Gly Lys Thr Tyr Phe Phe Arg Gly
420 425 430

Asn Lys Tyr Tyr Arg Phe Asn Glu Glu Leu Arg Ala Val Asp Ser Glu
435 440 445

Tyr Pro Lys Asn Ile Lys Val Trp Glu Gly Ile Pro Glu Ser Pro Arg
450 455 460

Gly Ser Phe Met Gly Ser Asp Glu Val Phe Thr Tyr Phe Tyr Lys Gly
465 470 475 480

Asn Lys Tyr Trp Lys Phe Asn Asn Gln Lys Leu Lys Val Glu Pro Gly
485 490 495

Tyr Pro Lys Ser Ala Leu Arg Asp Trp Met Gly Cys Pro Ser Gly Gly
500 505 510

Arg Pro Asp Glu Gly Thr Glu Glu Glu Thr Glu Val Ile Ile Ile Glu
515 520 525

Val Asp Glu Glu Gly Gly Gly Ala Val Ser Ala Ala Ala Val Val Leu
530 535 540

Pro Val Leu Leu Leu Leu Leu Val Leu Ala Val Gly Leu Ala Val Phe
545 550 555 560

Phe Phe Arg Arg His Gly Thr Pro Arg Arg Leu Leu Tyr Cys Gln Arg
565 570 575

Ser Leu Leu Asp Lys Val
580

<210> 17
<211> 655
<212> PRT
<213> Homo sapiens

<400> 17

Met Gly Thr Ser Pro Ser Ser Ser Thr Ala Leu Ala Ser Cys Ser Arg
1 5 10 15

Ile Ala Arg Arg Ala Thr Ala Thr Met Ile Ala Gly Ser Leu Leu Leu
20 25 30

Leu Gly Phe Leu Ser Thr Thr Thr Ala Gln Pro Glu Gln Lys Ala Ser
35 40 45

Asn Leu Ile Gly Thr Tyr Arg His Val Asp Arg Ala Thr Gly Gln Val
50 55 60

Leu Thr Cys Asp Lys Cys Pro Ala Gly Thr Tyr Val Ser Glu His Cys
65 70 75 80

Thr Asn Thr Ser Leu Arg Val Cys Ser Ser Cys Pro Val Gly Thr Phe
85 90 95

Thr Arg His Glu Asn Gly Ile Glu Lys Cys His Asp Cys Ser Gln Pro
100 105 110

Cys Pro Trp Pro Met Ile Glu Lys Leu Pro Cys Ala Ala Leu Thr Asp
115 120 125

Arg Glu Cys Thr Cys Pro Pro Gly Met Phe Gln Ser Asn Ala Thr Cys
130 135 140

Ala Pro His Thr Val Cys Pro Val Gly Trp Gly Val Arg Lys Lys Gly
145 150 155 160

Thr Glu Thr Glu Asp Val Arg Cys Lys Gln Cys Ala Arg Gly Thr Phe
165 170 175

Ser Asp Val Pro Ser Ser Val Met Lys Cys Lys Ala Tyr Thr Asp Cys
180 185 190

Leu Ser Gln Asn Leu Val Val Ile Lys Pro Gly Thr Lys Glu Thr Asp
195 200 205

Asn Val Cys Gly Thr Leu Pro Ser Phe Ser Ser Ser Thr Ser Pro Ser
210 215 220

Pro Gly Thr Ala Ile Phe Pro Arg Pro Glu His Met Glu Thr His Glu
225 230 235 240

Val Pro Ser Ser Thr Tyr Val Pro Lys Gly Met Asn Ser Thr Glu Ser
245 250 255

Asn Ser Ser Ala Ser Val Arg Pro Lys Val Leu Ser Ser Ile Gln Glu
260 265 270

Gly Thr Val Pro Asp Asn Thr Ser Ser Ala Arg Gly Lys Glu Asp Val
275 280 285

Asn Lys Thr Leu Pro Asn Leu Gln Val Val Asn His Gln Gln Gly Pro
290 295 300

His His Arg His Ile Leu Lys Leu Leu Pro Ser Met Glu Ala Thr Gly
305 310 315 320

Gly Glu Lys Ser Ser Thr Pro Ile Lys Gly Pro Lys Arg Gly His Pro
325 330 335

Arg Gln Asn Leu His Lys His Phe Asp Ile Asn Glu His Leu Pro Trp
340 345 350

Met Ile Val Leu Phe Leu Leu Leu Val Leu Val Val Ile Val Val Cys
355 360 365

Ser Ile Arg Lys Ser Ser Arg Thr Leu Lys Lys Gly Pro Arg Gln Asp
370 375 380

Pro Ser Ala Ile Val Glu Lys Ala Gly Leu Lys Lys Ser Met Thr Pro
385 390 395 400

Thr Gln Asn Arg Glu Lys Trp Ile Tyr Tyr Cys Asn Gly His Gly Ile
405 410 415

Asp Ile Leu Lys Leu Val Ala Ala Gln Val Gly Ser Gln Trp Lys Asp
420 425 430

Ile Tyr Gln Phe Leu Cys Asn Ala Ser Glu Arg Glu Val Ala Ala Phe

435 440 445

Ser Asn Gly Tyr Thr Ala Asp His Glu Arg Ala Tyr Ala Ala Leu Gln
450 455 460

His Trp Thr Ile Arg Gly Pro Glu Ala Ser Leu Ala Gln Leu Ile Ser
465 470 475 480

Ala Leu Arg Gln His Arg Arg Asn Asp Val Val Glu Lys Ile Arg Gly
485 490 495

Leu Met Glu Asp Thr Thr Gln Leu Glu Thr Asp Lys Leu Ala Leu Pro
500 505 510

Met Ser Pro Ser Pro Leu Ser Pro Ser Pro Ile Pro Ser Pro Asn Ala
515 520 525

Lys Leu Glu Asn Ser Ala Leu Leu Thr Val Glu Pro Ser Pro Gln Asp
530 535 540

Lys Asn Lys Gly Phe Phe Val Asp Glu Ser Glu Pro Leu Leu Arg Cys
545 550 555 560

Asp Ser Thr Ser Ser Gly Ser Ser Ala Leu Ser Arg Asn Gly Ser Phe
565 570 575

Ile Thr Lys Glu Lys Lys Asp Thr Val Leu Arg Gln Val Arg Leu Asp
580 585 590

Pro Cys Asp Leu Gln Pro Ile Phe Asp Asp Met Leu His Phe Leu Asn
595 600 605

Pro Glu Glu Leu Arg Val Ile Glu Glu Ile Pro Gln Ala Glu Asp Lys
610 615 620

Leu Asp Arg Leu Phe Glu Ile Ile Gly Val Lys Ser Gln Glu Ala Ser
625 630 635 640

Gln Thr Leu Leu Asp Ser Val Tyr Ser His Leu Pro Asp Leu Leu
645 650 655

<210> 18
<211> 437
<212> PRT
<213> Homo sapiens

<400> 18

Met Leu Gln Asp Pro Asp Ser Asp Gln Pro Leu Asn Ser Leu Asp Val

```
1               5                   10                  15

Lys Pro Leu Arg Lys Pro Arg Ile Pro Met Glu Thr Phe Arg Lys Val
            20                  25                  30

Gly Ile Pro Ile Ile Ile Ala Leu Leu Ser Leu Ala Ser Ile Ile Ile
        35                  40                  45

Val Val Val Leu Ile Lys Val Ile Leu Asp Lys Tyr Tyr Phe Leu Cys
    50                  55                  60

Gly Gln Pro Leu His Phe Ile Pro Arg Lys Gln Leu Cys Asp Gly Glu
65                  70                  75                  80

Leu Asp Cys Pro Leu Gly Glu Asp Glu Glu His Cys Val Lys Ser Phe
                85                  90                  95

Pro Glu Gly Pro Ala Val Ala Val Arg Leu Ser Lys Asp Arg Ser Thr
            100                 105                 110

Leu Gln Val Leu Asp Ser Ala Thr Gly Asn Trp Phe Ser Ala Cys Phe
        115                 120                 125

Asp Asn Phe Thr Glu Ala Leu Ala Glu Thr Ala Cys Arg Gln Met Gly
    130                 135                 140

Tyr Ser Ser Lys Pro Thr Phe Arg Ala Val Glu Ile Gly Pro Asp Gln
145                 150                 155                 160

Asp Leu Asp Val Val Glu Ile Thr Glu Asn Ser Gln Glu Leu Arg Met
                165                 170                 175

Arg Asn Ser Ser Gly Pro Cys Leu Ser Gly Ser Leu Val Ser Leu His
            180                 185                 190

Cys Leu Ala Cys Gly Lys Ser Leu Lys Thr Pro Arg Val Val Gly Gly
        195                 200                 205

Glu Glu Ala Ser Val Asp Ser Trp Pro Trp Gln Val Ser Ile Gln Tyr
    210                 215                 220

Asp Lys Gln His Val Cys Gly Gly Ser Ile Leu Asp Pro His Trp Val
225                 230                 235                 240

Leu Thr Ala Ala His Cys Phe Arg Lys His Thr Asp Val Phe Asn Trp
                245                 250                 255
```

Lys Val Arg Ala Gly Ser Asp Lys Leu Gly Ser Phe Pro Ser Leu Ala
260 265 270

Val Ala Lys Ile Ile Ile Ile Glu Phe Asn Pro Met Tyr Pro Lys Asp
275 280 285

Asn Asp Ile Ala Leu Met Lys Leu Gln Phe Pro Leu Thr Phe Ser Gly
290 295 300

Thr Val Arg Pro Ile Cys Leu Pro Phe Phe Asp Glu Glu Leu Thr Pro
305 310 315 320

Ala Thr Pro Leu Trp Ile Ile Gly Trp Gly Phe Thr Lys Gln Asn Gly
325 330 335

Gly Lys Met Ser Asp Ile Leu Leu Gln Ala Ser Val Gln Val Ile Asp
340 345 350

Ser Thr Arg Cys Asn Ala Asp Asp Ala Tyr Gln Gly Glu Val Thr Glu
355 360 365

Lys Met Met Cys Ala Gly Ile Pro Glu Gly Gly Val Asp Thr Cys Gln
370 375 380

Gly Asp Ser Gly Gly Pro Leu Met Tyr Gln Ser Asp Gln Trp His Val
385 390 395 400

Val Gly Ile Val Ser Trp Gly Tyr Gly Cys Gly Gly Pro Ser Thr Pro
405 410 415

Gly Val Tyr Thr Lys Val Ser Ala Tyr Leu Asn Trp Ile Tyr Asn Val
420 425 430

Trp Lys Ala Glu Leu
435

<210> 19
<211> 326
<212> PRT
<213> Homo sapiens

<400> 19

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285
```

```
Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325
```

<210> 20
<211> 377
<212> PRT
<213> Homo sapiens

<400> 20

```
Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175
```

Pro Lys Asp Thr Leu Met Ile Ser Arg Thr Pro Glu Val Thr Cys Val
180 185 190

Val Val Asp Val Ser His Glu Asp Pro Glu Val Gln Phe Lys Trp Tyr
195 200 205

Val Asp Gly Val Glu Val His Asn Ala Lys Thr Lys Pro Arg Glu Glu
210 215 220

Gln Tyr Asn Ser Thr Phe Arg Val Val Ser Val Leu Thr Val Leu His
225 230 235 240

Gln Asp Trp Leu Asn Gly Lys Glu Tyr Lys Cys Lys Val Ser Asn Lys
245 250 255

Ala Leu Pro Ala Pro Ile Glu Lys Thr Ile Ser Lys Thr Lys Gly Gln
260 265 270

Pro Arg Glu Pro Gln Val Tyr Thr Leu Pro Pro Ser Arg Glu Glu Met
275 280 285

Thr Lys Asn Gln Val Ser Leu Thr Cys Leu Val Lys Gly Phe Tyr Pro
290 295 300

Ser Asp Ile Ala Val Glu Trp Glu Ser Ser Gly Gln Pro Glu Asn Asn
305 310 315 320

Tyr Asn Thr Thr Pro Pro Met Leu Asp Ser Asp Gly Ser Phe Phe Leu
325 330 335

Tyr Ser Lys Leu Thr Val Asp Lys Ser Arg Trp Gln Gln Gly Asn Ile
340 345 350

Phe Ser Cys Ser Val Met His Glu Ala Leu His Asn Arg Phe Thr Gln
355 360 365

Lys Ser Leu Ser Leu Ser Pro Gly Lys
370 375

<210> 21
<211> 327
<212> PRT
<213> Homo sapiens

<400> 21

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Lys Thr
65 70 75 80

Tyr Thr Cys Asn Val Asp His Lys Pro Ser Asn Thr Lys Val Asp Lys
85 90 95

Arg Val Glu Ser Lys Tyr Gly Pro Pro Cys Pro Ser Cys Pro Ala Pro
100 105 110

Glu Phe Leu Gly Gly Pro Ser Val Phe Leu Phe Pro Pro Lys Pro Lys
115 120 125

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp
145 150 155 160

Gly Val Glu Val His Asn Ala Lys Thr Lys Pro Arg Glu Glu Gln Phe
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

<210> 22
<211> 21
<212> PRT
<213> Artificial sequence

<220>
<223> an artificially synthesized sequence

<400> 22

Phe Asn Asn Phe Thr Val Ser Phe Trp Leu Arg Val Pro Lys Val Ser
1 5 10 15

Ala Ser His Leu Glu
20

<210> 23
<211> 2154
<212> DNA
<213> Artificial sequence

<220>
<223> an artificially synthesized sequence

<400> 23
atgaagttcg ccgagcacct ctccgcgcac atcactcccg agtggaggaa gcaatacatc 60

cagtatgagg ctttcaagga tatgctgtat tcagctcagg accaggcacc ttctgtggaa 120

gttacagatg aggacacagt aaagaggtat tttgccaagt ttgaagagaa gtttttccaa 180

acctgtgaaa aagaacttgc caaaatcaac acattttatt cagagaagct cgcagaggct 240

cagcgcaggt ttgctacact tcagaatgag cttcagtcat cactggatgc acagaaagaa 300

agcactggtg ttactacgct gcgacaacgc agaaagccag tcttccactt gtcccatgag 360

gaacgtgtcc aacatagaaa tattaaagac cttaaactgg ccttcagtga gttctacctc 420

agtctaatcc tgctgcagaa ctatcagaat ctgaatttta cagggtttcg aaaaatcctg 480

aaaaagcatg acaagatcct ggaaacatct cgtggagcag attggcgagt ggctcacgta 540

```
gaggtggccc cattttatac atgcaagaaa atcaaccagc ttatctctga aactgaggct      600
gtagtgacca atgaacttga agatggtgac agacaaaagg ctatgaagcg tttacgtgtc      660
ccccctttgg gagctgctca gcctgcacca gcatggacta cttttagagt tggcctattt      720
tgtggaatat tcattgtact gaatattacc cttgtgcttg ccgctgtatt taaacttgaa      780
acagatagaa gtatatggcc cttgataaga atctatcggg gtggctttct tctgattgag      840
ttcctttttc tactgggcat caacacgtat ggttggagac aggctggagt aaaccatgta      900
ctcatctttg aacttaatcc gagaagcaat ttgtctcatc aacatctctt tgagattgct      960
ggattcctcg ggatattgtg gtgcctgagc cttctggcat gcttctttgc tccaattagt     1020
gtcatcccca catatgtgta tccacttgcc ctttatggat ttatggtttt cttccttatc     1080
aaccccacca aaactttcta ctataaatcc cggttttggc tgcttaaact gctgtttcga     1140
gtatttacag ccccttcca taaggtaggc tttgctgatt tctggctggc ggatcagctg      1200
aacagcctgt cagtgatact gatggacctg gaatatatga tctgcttcta cagtttggag     1260
ctcaaatggg atgaaagtaa gggcctgttg ccaaataatt cagaagaatc aggaatttgc     1320
cacaaatata catatggtgt gcgggccatt gttcagtgca ttcctgcttg gcttcgcttc     1380
atccagtgcc tgcgccgata tcgagacaca aaaagggcct ttcctcattt agttaatgct     1440
ggcaaatact ccacaacttt cttcatggtg acgtttgcag ccctttacag cactcacaaa     1500
gaacgaggtc actcggacac tatggtgttc ttttacctgt ggattgtctt ttatatcatc     1560
agttcctgct ataccctcat ctgggatctc aagatggact ggggtctctt cgataagaat     1620
gcaggagaga acactttcct ccgggaagag attgtatacc cccaaaaagc ctactactac     1680
tgtgccataa tagaggatgt gattctgcgc tttgcttgga ctatccaaat ctcgattacc     1740
tctacaactt tgttgcctca ttctggggac atcattgcta ctgtctttgc cccacttgag     1800
gttttccggc gatttgtgtg gaacttcttc cgcctggaga atgaacatct gaataactgt     1860
ggtgagttcc gtgctgtgcg ggacatctct gtggcccccc tgaacgcaga tgatcagact     1920
ctcctagaac agatgatgga ccaggatgat ggggtacgaa accgccagaa gaatcggtca     1980
tggaagtaca accagagcat atccctgcgc cggcctcgcc tcgcttctca atccaaggct     2040
cgtgacacta aggtattgat agaagacaca gatgatgaag ctaacacttt caacaacttc     2100
acagtgtctt tctggctgcg agtgcccaag gtgtctgcct cccatctgga atga           2154
```

<210> 24
<211> 717
<212> PRT
<213> Artificial sequence

<220>
<223> an artificially synthesized sequence

<400> 24

Met Lys Phe Ala Glu His Leu Ser Ala His Ile Thr Pro Glu Trp Arg
1               5                   10                  15

Lys Gln Tyr Ile Gln Tyr Glu Ala Phe Lys Asp Met Leu Tyr Ser Ala
            20                  25                  30

Gln Asp Gln Ala Pro Ser Val Glu Val Thr Asp Glu Asp Thr Val Lys
        35                  40                  45

Arg Tyr Phe Ala Lys Phe Glu Glu Lys Phe Phe Gln Thr Cys Glu Lys
    50                  55                  60

Glu Leu Ala Lys Ile Asn Thr Phe Tyr Ser Glu Lys Leu Ala Glu Ala
65                  70                  75                  80

Gln Arg Arg Phe Ala Thr Leu Gln Asn Glu Leu Gln Ser Ser Leu Asp
                85                  90                  95

Ala Gln Lys Glu Ser Thr Gly Val Thr Thr Leu Arg Gln Arg Arg Lys
            100                 105                 110

Pro Val Phe His Leu Ser His Glu Glu Arg Val Gln His Arg Asn Ile
        115                 120                 125

Lys Asp Leu Lys Leu Ala Phe Ser Glu Phe Tyr Leu Ser Leu Ile Leu
    130                 135                 140

Leu Gln Asn Tyr Gln Asn Leu Asn Phe Thr Gly Phe Arg Lys Ile Leu
145                 150                 155                 160

Lys Lys His Asp Lys Ile Leu Glu Thr Ser Arg Gly Ala Asp Trp Arg
                165                 170                 175

Val Ala His Val Glu Val Ala Pro Phe Tyr Thr Cys Lys Lys Ile Asn
            180                 185                 190

Gln Leu Ile Ser Glu Thr Glu Ala Val Val Thr Asn Glu Leu Glu Asp
        195                 200                 205

Gly Asp Arg Gln Lys Ala Met Lys Arg Leu Arg Val Pro Pro Leu Gly
    210                 215                 220

Ala Ala Gln Pro Ala Pro Ala Trp Thr Thr Phe Arg Val Gly Leu Phe
225                 230                 235                 240

Cys Gly Ile Phe Ile Val Leu Asn Ile Thr Leu Val Leu Ala Ala Val

245 250 255

Phe Lys Leu Glu Thr Asp Arg Ser Ile Trp Pro Leu Ile Arg Ile Tyr
260 265 270

Arg Gly Gly Phe Leu Leu Ile Glu Phe Leu Phe Leu Leu Gly Ile Asn
275 280 285

Thr Tyr Gly Trp Arg Gln Ala Gly Val Asn His Val Leu Ile Phe Glu
290 295 300

Leu Asn Pro Arg Ser Asn Leu Ser His Gln His Leu Phe Glu Ile Ala
305 310 315 320

Gly Phe Leu Gly Ile Leu Trp Cys Leu Ser Leu Leu Ala Cys Phe Phe
325 330 335

Ala Pro Ile Ser Val Ile Pro Thr Tyr Val Tyr Pro Leu Ala Leu Tyr
340 345 350

Gly Phe Met Val Phe Phe Leu Ile Asn Pro Thr Lys Thr Phe Tyr Tyr
355 360 365

Lys Ser Arg Phe Trp Leu Leu Lys Leu Leu Phe Arg Val Phe Thr Ala
370 375 380

Pro Phe His Lys Val Gly Phe Ala Asp Phe Trp Leu Ala Asp Gln Leu
385 390 395 400

Asn Ser Leu Ser Val Ile Leu Met Asp Leu Glu Tyr Met Ile Cys Phe
405 410 415

Tyr Ser Leu Glu Leu Lys Trp Asp Glu Ser Lys Gly Leu Leu Pro Asn
420 425 430

Asn Ser Glu Glu Ser Gly Ile Cys His Lys Tyr Thr Tyr Gly Val Arg
435 440 445

Ala Ile Val Gln Cys Ile Pro Ala Trp Leu Arg Phe Ile Gln Cys Leu
450 455 460

Arg Arg Tyr Arg Asp Thr Lys Arg Ala Phe Pro His Leu Val Asn Ala
465 470 475 480

Gly Lys Tyr Ser Thr Thr Phe Phe Met Val Thr Phe Ala Ala Leu Tyr
485 490 495

```
Ser Thr His Lys Glu Arg Gly His Ser Asp Thr Met Val Phe Phe Tyr
            500                 505                 510

Leu Trp Ile Val Phe Tyr Ile Ile Ser Ser Cys Tyr Thr Leu Ile Trp
        515                 520                 525

Asp Leu Lys Met Asp Trp Gly Leu Phe Asp Lys Asn Ala Gly Glu Asn
    530                 535                 540

Thr Phe Leu Arg Glu Glu Ile Val Tyr Pro Gln Lys Ala Tyr Tyr Tyr
545                 550                 555                 560

Cys Ala Ile Ile Glu Asp Val Ile Leu Arg Phe Ala Trp Thr Ile Gln
                565                 570                 575

Ile Ser Ile Thr Ser Thr Thr Leu Leu Pro His Ser Gly Asp Ile Ile
            580                 585                 590

Ala Thr Val Phe Ala Pro Leu Glu Val Phe Arg Arg Phe Val Trp Asn
        595                 600                 605

Phe Phe Arg Leu Glu Asn Glu His Leu Asn Asn Cys Gly Glu Phe Arg
    610                 615                 620

Ala Val Arg Asp Ile Ser Val Ala Pro Leu Asn Ala Asp Asp Gln Thr
625                 630                 635                 640

Leu Leu Glu Gln Met Met Asp Gln Asp Asp Gly Val Arg Asn Arg Gln
                645                 650                 655

Lys Asn Arg Ser Trp Lys Tyr Asn Gln Ser Ile Ser Leu Arg Arg Pro
            660                 665                 670

Arg Leu Ala Ser Gln Ser Lys Ala Arg Asp Thr Lys Val Leu Ile Glu
        675                 680                 685

Asp Thr Asp Asp Glu Ala Asn Thr Phe Asn Asn Phe Thr Val Ser Phe
    690                 695                 700

Trp Leu Arg Val Pro Lys Val Ser Ala Ser His Leu Glu
705                 710                 715
```

<210> 25
<211> 10
<212> PRT
<213> Artificial sequence

<220>
<223> an artificially synthesized sequence

<400> 25

Glu Gln Lys Leu Ile Ser Glu Glu Asp Leu
1               5                   10

<210> 26
<211> 2121
<212> DNA
<213> Artificial sequence

<220>
<223> an artificially synthesized sequence

<400> 26
atgaagttcg ccgagcacct ctccgcgcac atcactcccg agtggaggaa gcaatacatc 60

cagtatgagg ctttcaagga tatgctgtat tcagctcagg accaggcacc ttctgtggaa 120

gttacagatg aggacacagt aaagaggtat tttgccaagt ttgaagagaa gtttttccaa 180

acctgtgaaa aagaacttgc caaaatcaac acattttatt cagagaagct cgcagaggct 240

cagcgcaggt ttgctacact tcagaatgag cttcagtcat cactggatgc acagaaagaa 300

agcactggtg ttactacgct gcgacaacgc agaaagccag tcttccactt gtcccatgag 360

gaacgtgtcc aacatagaaa tattaaagac cttaaactgg ccttcagtga gttctacctc 420

agtctaatcc tgctgcagaa ctatcagaat ctgaatttta cagggtttcg aaaaatcctg 480

aaaaagcatg acaagatcct ggaaacatct cgtggagcag attggcgagt ggctcacgta 540

gaggtggccc cattttatac atgcaagaaa atcaaccagc ttatctctga aactgaggct 600

gtagtgacca atgaacttga agatggtgac agacaaaagg ctatgaagcg tttacgtgtc 660

ccccctttgg gagctgctca gcctgcacca gcatggacta cttttagagt tggcctattt 720

tgtggaatat tcattgtact gaatattacc cttgtgcttg ccgctgtatt taaacttgaa 780

acagatagaa gtatatggcc cttgataaga atctatcggg gtggctttct tctgattgag 840

ttcctttttc tactgggcat caacacgtat ggttggagac aggctggagt aaaccatgta 900

ctcatctttg aacttaatcc gagaagcaat ttgtctcatc aacatctctt tgagattgct 960

ggattcctcg ggatattgtg gtgcctgagc cttctggcat gcttctttgc tccaattagt 1020

gtcatcccca catatgtgta tccacttgcc ctttatggat ttatggtttt cttccttatc 1080

aaccccacca aaactttcta ctataaatcc cggttttggc tgcttaaact gctgtttcga 1140

gtatttacag ccccccttcca taaggtaggc tttgctgatt tctggctggc ggatcagctg 1200

aacagcctgt cagtgatact gatggacctg gaatatatga tctgcttcta cagtttggag 1260

ctcaaatggg atgaaagtaa gggcctgttg ccaaataatt cagaagaatc aggaatttgc 1320

cacaaatata catatggtgt gcgggccatt gttcagtgca ttcctgcttg gcttcgcttc 1380

atccagtgcc tgcgccgata tcgagacaca aaaagggcct ttcctcattt agttaatgct 1440

ggcaaatact ccacaacttt cttcatggtg acgtttgcag ccctttacag cactcacaaa 1500

gaacgaggtc actcggacac tatggtgttc ttttacctgt ggattgtctt ttatatcatc 1560

agttcctgct ataccctcat ctgggatctc aagatggact ggggtctctt cgataagaat 1620

gcaggagaga acactttcct ccgggaagag attgtatacc cccaaaaagc ctactactac 1680

tgtgccataa tagaggatgt gattctgcgc tttgcttgga ctatccaaat ctcgattacc 1740

tctacaactt tgttgcctca ttctggggac atcattgcta ctgtctttgc cccacttgag 1800

gttttccggc gatttgtgtg gaacttcttc cgcctggaga atgaacatct gaataactgt 1860

ggtgagttcc gtgctgtgcg ggacatctct gtggcccccc tgaacgcaga tgatcagact 1920

ctcctagaac agatgatgga ccaggatgat ggggtacgaa accgccagaa gaatcggtca 1980

tggaagtaca accagagcat atccctgcgc cggcctcgcc tcgcttctca atccaaggct 2040

cgtgacacta aggtattgat agaagacaca gatgatgaag ctaacactga gcagaagctc 2100

atctcagaag aagacctctg a 2121

<210> 27
<211> 706
<212> PRT
<213> Artificial sequence

<220>
<223> an artificially synthesized sequence

<400> 27

Met Lys Phe Ala Glu His Leu Ser Ala His Ile Thr Pro Glu Trp Arg
1 5 10 15

Lys Gln Tyr Ile Gln Tyr Glu Ala Phe Lys Asp Met Leu Tyr Ser Ala
20 25 30

Gln Asp Gln Ala Pro Ser Val Glu Val Thr Asp Glu Asp Thr Val Lys
35 40 45

Arg Tyr Phe Ala Lys Phe Glu Glu Lys Phe Phe Gln Thr Cys Glu Lys
50 55 60

Glu Leu Ala Lys Ile Asn Thr Phe Tyr Ser Glu Lys Leu Ala Glu Ala
65 70 75 80

Gln Arg Arg Phe Ala Thr Leu Gln Asn Glu Leu Gln Ser Ser Leu Asp
85 90 95

Ala Gln Lys Glu Ser Thr Gly Val Thr Thr Leu Arg Gln Arg Arg Lys
100 105 110

```
Pro Val Phe His Leu Ser His Glu Glu Arg Val Gln His Arg Asn Ile
        115                 120                 125

Lys Asp Leu Lys Leu Ala Phe Ser Glu Phe Tyr Leu Ser Leu Ile Leu
    130                 135                 140

Leu Gln Asn Tyr Gln Asn Leu Asn Phe Thr Gly Phe Arg Lys Ile Leu
145                 150                 155                 160

Lys Lys His Asp Lys Ile Leu Glu Thr Ser Arg Gly Ala Asp Trp Arg
                165                 170                 175

Val Ala His Val Glu Val Ala Pro Phe Tyr Thr Cys Lys Lys Ile Asn
            180                 185                 190

Gln Leu Ile Ser Glu Thr Glu Ala Val Val Thr Asn Glu Leu Glu Asp
        195                 200                 205

Gly Asp Arg Gln Lys Ala Met Lys Arg Leu Arg Val Pro Pro Leu Gly
    210                 215                 220

Ala Ala Gln Pro Ala Pro Ala Trp Thr Thr Phe Arg Val Gly Leu Phe
225                 230                 235                 240

Cys Gly Ile Phe Ile Val Leu Asn Ile Thr Leu Val Leu Ala Ala Val
                245                 250                 255

Phe Lys Leu Glu Thr Asp Arg Ser Ile Trp Pro Leu Ile Arg Ile Tyr
            260                 265                 270

Arg Gly Gly Phe Leu Leu Ile Glu Phe Leu Phe Leu Leu Gly Ile Asn
        275                 280                 285

Thr Tyr Gly Trp Arg Gln Ala Gly Val Asn His Val Leu Ile Phe Glu
    290                 295                 300

Leu Asn Pro Arg Ser Asn Leu Ser His Gln His Leu Phe Glu Ile Ala
305                 310                 315                 320

Gly Phe Leu Gly Ile Leu Trp Cys Leu Ser Leu Leu Ala Cys Phe Phe
                325                 330                 335

Ala Pro Ile Ser Val Ile Pro Thr Tyr Val Tyr Pro Leu Ala Leu Tyr
            340                 345                 350

Gly Phe Met Val Phe Phe Leu Ile Asn Pro Thr Lys Thr Phe Tyr Tyr
        355                 360                 365
```

Lys Ser Arg Phe Trp Leu Leu Lys Leu Leu Phe Arg Val Phe Thr Ala
370 375 380

Pro Phe His Lys Val Gly Phe Ala Asp Phe Trp Leu Ala Asp Gln Leu
385 390 395 400

Asn Ser Leu Ser Val Ile Leu Met Asp Leu Glu Tyr Met Ile Cys Phe
405 410 415

Tyr Ser Leu Glu Leu Lys Trp Asp Glu Ser Lys Gly Leu Leu Pro Asn
420 425 430

Asn Ser Glu Glu Ser Gly Ile Cys His Lys Tyr Thr Tyr Gly Val Arg
435 440 445

Ala Ile Val Gln Cys Ile Pro Ala Trp Leu Arg Phe Ile Gln Cys Leu
450 455 460

Arg Arg Tyr Arg Asp Thr Lys Arg Ala Phe Pro His Leu Val Asn Ala
465 470 475 480

Gly Lys Tyr Ser Thr Thr Phe Phe Met Val Thr Phe Ala Ala Leu Tyr
485 490 495

Ser Thr His Lys Glu Arg Gly His Ser Asp Thr Met Val Phe Phe Tyr
500 505 510

Leu Trp Ile Val Phe Tyr Ile Ile Ser Ser Cys Tyr Thr Leu Ile Trp
515 520 525

Asp Leu Lys Met Asp Trp Gly Leu Phe Asp Lys Asn Ala Gly Glu Asn
530 535 540

Thr Phe Leu Arg Glu Glu Ile Val Tyr Pro Gln Lys Ala Tyr Tyr Tyr
545 550 555 560

Cys Ala Ile Ile Glu Asp Val Ile Leu Arg Phe Ala Trp Thr Ile Gln
565 570 575

Ile Ser Ile Thr Ser Thr Thr Leu Leu Pro His Ser Gly Asp Ile Ile
580 585 590

Ala Thr Val Phe Ala Pro Leu Glu Val Phe Arg Arg Phe Val Trp Asn
595 600 605

Phe Phe Arg Leu Glu Asn Glu His Leu Asn Asn Cys Gly Glu Phe Arg

610 615 620

Ala Val Arg Asp Ile Ser Val Ala Pro Leu Asn Ala Asp Asp Gln Thr
625 630 635 640

Leu Leu Glu Gln Met Met Asp Gln Asp Asp Gly Val Arg Asn Arg Gln
645 650 655

Lys Asn Arg Ser Trp Lys Tyr Asn Gln Ser Ile Ser Leu Arg Arg Pro
660 665 670

Arg Leu Ala Ser Gln Ser Lys Ala Arg Asp Thr Lys Val Leu Ile Glu
675 680 685

Asp Thr Asp Asp Glu Ala Asn Thr Glu Gln Lys Leu Ile Ser Glu Glu
690 695 700

Asp Leu
705

<210> 28
<211> 1206
<212> DNA
<213> Homo sapiens

<400> 28
atggaagatc cgtcgggggc tcgcgagccc cgggcccggc cgagagagcg ggacccggga 60

cggcgccccc acccagacca aggccgcacc cacgatagac cgcgggaccg acccggggac 120

ccgcgcagga agcgaagcag cgacgggaac cggcgaaggg acggggaccg ggacccggag 180

agagaccagg agagggacgg gaaccgcgac cggaaccggg accgggagag ggagagagag 240

agggaaagag acccggaccg aggcccccgc cgggacacac acagggacgc gggccctcgc 300

gcaggtgaac acggagtttg ggaaaaaccg cgccaaagcc ggacgcggga cggagcccgg 360

ggactgacct gggacgcagc cgcgcctcct gggcccgcgc cctgggaagc cccggagccg 420

ccgcagccgc agaggaaggg agaccccggg cgccgcagac ccgaaagtga accccccttcg 480

gagagatatc tgccctcgac ccccaggcct ggacgagagg aggtggaata ttaccagtca 540

gaggcggaag gactcctgga atgccacaaa tgcaaatact tgtgcactgg gagagcctgc 600

tgccaaatgc tggaggttct cctgaacttg ctgatcctgg cctgcagctc tgtgtcttac 660

agttccacag gggctacac gggcatcacc agcttggggg gcatttacta ctatcagttc 720

ggaggggctt acagtggctt tgatggtgct gacggggaga aggcccagca actggatgtc 780

cagttctacc agctaaagct gcccatggtc actgtggcaa tggcctgtag tggagccctc 840

acagccctct gctgcctctt cgttgccatg ggtgtcctgc gggtcccgtg gcattgtcca 900

ctgttgctgg tgaccgaagg cttgttggac atgctcatcg cggggggta catcccggcc 960

ttgtacttct acttccacta cctctctgct gcctatggct ctcctgtgtg taaagagagg 1020

caggcgctgt accaaagcaa aggctacagc ggtttcggct gcagtttcca cggagcagat 1080

ataggagctg gaatctttgc tgccctgggc attgtggtct ttgccctggg ggctgtcctg 1140

gccataaagg gctaccgaaa agttaggaag ctaaaagaga agccagcaga aatgtttgaa 1200

ttttga 1206

<210> 29
<211> 1236
<212> DNA
<213> Artificial sequence

<220>
<223> an artificially synthesized sequence

<400> 29
atggagcaga agctcatctc agaagaagac ctcgaagatc cgtcgggggc tcgcgagccc 60

cgggcccggc cgagagagcg ggacccggga cggcgccccc acccagacca aggccgcacc 120

cacgatagac cgcgggaccg acccggggac ccgcgcagga agcgaagcag cgacgggaac 180

cggcgaaggg acggggaccg ggacccggag agagaccagg agagggacgg gaaccgcgac 240

cggaaccggg accgggagag ggagagagag agggaaagag acccggaccg aggcccccgc 300

cgggacacac acagggacgc gggccctcgc gcaggtgaac acggagtttg ggaaaaaccg 360

cgccaaagcc ggacgcggga cggagcccgg ggactgacct gggacgcagc cgcgcctcct 420

gggcccgcgc cctgggaagc cccggagccg ccgcagccgc agaggaaggg agaccccggg 480

cgccgcagac ccgaaagtga accccttcg gagagatatc tgccctcgac ccccaggcct 540

ggacgagagg aggtggaata ttaccagtca gaggcggaag gactcctgga atgccacaaa 600

tgcaaatact tgtgcactgg gagagcctgc tgccaaatgc tggaggttct cctgaacttg 660

ctgatcctgg cctgcagctc tgtgtcttac agttccacag gggctacac gggcatcacc 720

agcttggggg gcatttacta ctatcagttc ggaggggctt acagtggctt tgatggtgct 780

gacggggaga aggcccagca actggatgtc cagttctacc agctaaagct gcccatggtc 840

actgtggcaa tggcctgtag tggagccctc acagccctct gctgcctctt cgttgccatg 900

ggtgtcctgc gggtcccgtg gcattgtcca ctgttgctgg tgaccgaagg cttgttggac 960

atgctcatcg cggggggta catcccggcc ttgtacttct acttccacta cctctctgct 1020

gcctatggct ctcctgtgtg taaagagagg caggcgctgt accaaagcaa aggctacagc 1080

ggtttcggct gcagtttcca cggagcagat ataggagctg gaatctttgc tgccctgggc 1140

attgtggtct ttgccctggg ggctgtcctg gccataaagg gctaccgaaa agttaggaag 1200

ctaaaagaga agccagcaga aatgtttgaa ttttga 1236

<210> 30
<211> 411
<212> PRT
<213> Artificial sequence

<220>
<223> an artificially synthesized sequence

<400> 30

Met Glu Gln Lys Leu Ile Ser Glu Glu Asp Leu Glu Asp Pro Ser Gly
1               5                   10                  15

Ala Arg Glu Pro Arg Ala Arg Pro Arg Glu Arg Asp Pro Gly Arg Arg
            20                  25                  30

Pro His Pro Asp Gln Gly Arg Thr His Asp Arg Pro Arg Asp Arg Pro
        35                  40                  45

Gly Asp Pro Arg Arg Lys Arg Ser Ser Asp Gly Asn Arg Arg Arg Asp
    50                  55                  60

Gly Asp Arg Asp Pro Glu Arg Asp Gln Glu Arg Asp Gly Asn Arg Asp
65                  70                  75                  80

Arg Asn Arg Asp Arg Glu Arg Glu Arg Glu Arg Glu Arg Asp Pro Asp
                85                  90                  95

Arg Gly Pro Arg Arg Asp Thr His Arg Asp Ala Gly Pro Arg Ala Gly
            100                 105                 110

Glu His Gly Val Trp Glu Lys Pro Arg Gln Ser Arg Thr Arg Asp Gly
        115                 120                 125

Ala Arg Gly Leu Thr Trp Asp Ala Ala Ala Pro Pro Gly Pro Ala Pro
    130                 135                 140

Trp Glu Ala Pro Glu Pro Pro Gln Pro Gln Arg Lys Gly Asp Pro Gly
145                 150                 155                 160

Arg Arg Arg Pro Glu Ser Glu Pro Pro Ser Glu Arg Tyr Leu Pro Ser
                165                 170                 175

Thr Pro Arg Pro Gly Arg Glu Glu Val Glu Tyr Tyr Gln Ser Glu Ala
            180                 185                 190

Glu Gly Leu Leu Glu Cys His Lys Cys Lys Tyr Leu Cys Thr Gly Arg
        195                 200                 205

Ala Cys Cys Gln Met Leu Glu Val Leu Leu Asn Leu Leu Ile Leu Ala
210 215 220

Cys Ser Ser Val Ser Tyr Ser Ser Thr Gly Gly Tyr Thr Gly Ile Thr
225 230 235 240

Ser Leu Gly Gly Ile Tyr Tyr Tyr Gln Phe Gly Gly Ala Tyr Ser Gly
245 250 255

Phe Asp Gly Ala Asp Gly Glu Lys Ala Gln Gln Leu Asp Val Gln Phe
260 265 270

Tyr Gln Leu Lys Leu Pro Met Val Thr Val Ala Met Ala Cys Ser Gly
275 280 285

Ala Leu Thr Ala Leu Cys Cys Leu Phe Val Ala Met Gly Val Leu Arg
290 295 300

Val Pro Trp His Cys Pro Leu Leu Leu Val Thr Glu Gly Leu Leu Asp
305 310 315 320

Met Leu Ile Ala Gly Gly Tyr Ile Pro Ala Leu Tyr Phe Tyr Phe His
325 330 335

Tyr Leu Ser Ala Ala Tyr Gly Ser Pro Val Cys Lys Glu Arg Gln Ala
340 345 350

Leu Tyr Gln Ser Lys Gly Tyr Ser Gly Phe Gly Cys Ser Phe His Gly
355 360 365

Ala Asp Ile Gly Ala Gly Ile Phe Ala Ala Leu Gly Ile Val Val Phe
370 375 380

Ala Leu Gly Ala Val Leu Ala Ile Lys Gly Tyr Arg Lys Val Arg Lys
385 390 395 400

Leu Lys Glu Lys Pro Ala Glu Met Phe Glu Phe
405 410

<210> 31
<211> 28
<212> PRT
<213> Artificial sequence

<220>
<223> an artificially synthesized sequence

<400> 31

Trp Ser His Pro Gln Phe Glu Lys Gly Gly Gly Ser Gly Gly Gly Ser
1               5                   10                  15

Gly Gly Ser Ala Trp Ser His Pro Gln Phe Glu Lys
            20                  25

<210> 32
<211> 2175
<212> DNA
<213> Artificial sequence

<220>
<223> an artificially synthesized sequence

<400> 32
atgtaccaga ggatgctccg gtgcggtgcc gagctgggat cgcccggagg cggcagtagt 60

ggcggcgcag gggggcgcct ggccctgctc tggatagtcc cgctcaccct cagcggcctc 120

ctaggagtgg cctgggggc atccagtttg ggagcgcacc acatccacca taaacttgac 180

tacaaagacg atgacgacaa gggtgggtta gccaacccca cccgagtggg cggccgcgaa 240

ccatacccag gctcggcaga ggtgatccgg gagtctagca gtaccactgg catggtggtg 300

gggattgtcg cagcagcagc tctgtgcatc ctcatcctcc tctatgccat gaagaagcgc 360

agggccaagg gaggttcagg aggttcagga ggtctcgagc acggaactat cccattcaac 420

agaactcaca ggtccaagag aagctccgga atgggaaact gggtggttaa ccactggttt 480

tcagttttgt ttctggttgt ttggttaggg ctgaatgttt tcctgtttgt ggatgccttc 540

ctgaaatatg agaaggccga caaatactac tacacaagaa aaatccttgg gtcaacattg 600

gcctgtgccc gagcgtctgc tctctgcttg aattttaaca gcacgctgat cctgcttcct 660

gtgtgtcgca atctgctgtc cttcctgagg ggcacctgct cattttgcag ccgcacactg 720

agaaagcaat tggatcacaa cctcaccttc cacaagctgg tggcctatat gatctgccta 780

catacagcta ttcacatcat tgcacacctg tttaactttg actgctatag cagaagccga 840

caggccacag atggctccct tgcctccatt ctctccagcc tatctcatga tgagaaaaag 900

gggggttctt ggctaaatcc catccagtcc cgaaacacga cagtggagta tgtgacattc 960

accagcattg ctggtctcac tggagtgatc atgacaatag ccttgattct catggtaact 1020

tcagctactg agttcatccg gaggagttat tttgaagtct tctggtatac tcaccacctt 1080

tttatcttct atatccttgg cttagggatt cacggcattg gtggaattgt ccggggtcaa 1140

acagaggaga gcatgaatga gagtcatcct cgcaagtgtg cagagtcttt tgagatgtgg 1200

gatgatcgtg actcccactg taggcgccct aagtttgaag ggcatccccc tgagtcttgg 1260

aagtggatcc ttgcaccggt cattctttat atctgtgaaa ggatcctccg gttttaccgc 1320

tcccagcaga aggttgtgat taccaaggtt gttatgcacc catccaaagt tttggaattg 1380

cagatgaaca agcgtggctt cagcatggaa gtggggcagt atatctttgt taattgcccc 1440

tcaatctctc tcctggaatg gcatcctttt actttgacct ctgctccaga ggaagatttc 1500

ttctccattc atatccgagc agcaggggac tggacagaaa atctcataag ggctttcgaa 1560

caacaatatt caccaattcc caggattgaa gtggatggtc cctttggcac agccagtgag 1620

gatgttttcc agtatgaagt ggctgtgctg gttggagcag gaattggggt cacccccttt 1680

gcttctatct tgaaatccat ctggtacaaa ttccagtgtg cagaccacaa cctcaaaaca 1740

aaaaagatct atttctactg gatctgcagg gagacaggtg ccttttcctg gttcaacaac 1800

ctgttgactt ccctggaaca ggagatggag gaattaggca aagtgggttt tctaaactac 1860

cgtctcttcc tcaccggatg ggacagcaat attgttggtc atgcagcatt aaactttgac 1920

aaggccactg acatcgtgac aggtctgaaa cagaaaacct cctttgggag accaatgtgg 1980

gacaatgagt tttctacaat agctacctcc caccccaagt ctgtagtggg agttttctta 2040

tgtggccctc ggactttggc aaagagcctg cgcaaatgct gtcaccgata ttccagtctg 2100

gatcctagaa aggttcaatt ctacttcaac aaagaaaatt ttgagcagaa gctcatctca 2160

gaagaagacc tctga 2175

<210> 33
<211> 724
<212> PRT
<213> Artificial sequence

<220>
<223> an artificially synthesized sequence

<400> 33

Met Tyr Gln Arg Met Leu Arg Cys Gly Ala Glu Leu Gly Ser Pro Gly
1 5 10 15

Gly Gly Ser Ser Gly Gly Ala Gly Gly Arg Leu Ala Leu Leu Trp Ile
20 25 30

Val Pro Leu Thr Leu Ser Gly Leu Leu Gly Val Ala Trp Gly Ala Ser
35 40 45

Ser Leu Gly Ala His His Ile His His Lys Leu Asp Tyr Lys Asp Asp
50 55 60

Asp Asp Lys Gly Gly Leu Ala Asn Pro Thr Arg Val Gly Gly Arg Glu
65 70 75 80

Pro Tyr Pro Gly Ser Ala Glu Val Ile Arg Glu Ser Ser Ser Thr Thr
85 90 95

```
Gly Met Val Val Gly Ile Val Ala Ala Ala Ala Leu Cys Ile Leu Ile
            100                 105                 110

Leu Leu Tyr Ala Met Lys Lys Arg Arg Ala Lys Gly Gly Ser Gly Gly
        115                 120                 125

Ser Gly Gly Leu Glu His Gly Thr Ile Pro Phe Asn Arg Thr His Arg
    130                 135                 140

Ser Lys Arg Ser Ser Gly Met Gly Asn Trp Val Val Asn His Trp Phe
145                 150                 155                 160

Ser Val Leu Phe Leu Val Val Trp Leu Gly Leu Asn Val Phe Leu Phe
                165                 170                 175

Val Asp Ala Phe Leu Lys Tyr Glu Lys Ala Asp Lys Tyr Tyr Tyr Thr
            180                 185                 190

Arg Lys Ile Leu Gly Ser Thr Leu Ala Cys Ala Arg Ala Ser Ala Leu
        195                 200                 205

Cys Leu Asn Phe Asn Ser Thr Leu Ile Leu Leu Pro Val Cys Arg Asn
    210                 215                 220

Leu Leu Ser Phe Leu Arg Gly Thr Cys Ser Phe Cys Ser Arg Thr Leu
225                 230                 235                 240

Arg Lys Gln Leu Asp His Asn Leu Thr Phe His Lys Leu Val Ala Tyr
                245                 250                 255

Met Ile Cys Leu His Thr Ala Ile His Ile Ile Ala His Leu Phe Asn
            260                 265                 270

Phe Asp Cys Tyr Ser Arg Ser Arg Gln Ala Thr Asp Gly Ser Leu Ala
        275                 280                 285

Ser Ile Leu Ser Ser Leu Ser His Asp Glu Lys Lys Gly Gly Ser Trp
    290                 295                 300

Leu Asn Pro Ile Gln Ser Arg Asn Thr Thr Val Glu Tyr Val Thr Phe
305                 310                 315                 320

Thr Ser Ile Ala Gly Leu Thr Gly Val Ile Met Thr Ile Ala Leu Ile
                325                 330                 335

Leu Met Val Thr Ser Ala Thr Glu Phe Ile Arg Arg Ser Tyr Phe Glu
            340                 345                 350
```

Val Phe Trp Tyr Thr His His Leu Phe Ile Phe Tyr Ile Leu Gly Leu
355 360 365

Gly Ile His Gly Ile Gly Gly Ile Val Arg Gly Gln Thr Glu Glu Ser
370 375 380

Met Asn Glu Ser His Pro Arg Lys Cys Ala Glu Ser Phe Glu Met Trp
385 390 395 400

Asp Asp Arg Asp Ser His Cys Arg Arg Pro Lys Phe Glu Gly His Pro
405 410 415

Pro Glu Ser Trp Lys Trp Ile Leu Ala Pro Val Ile Leu Tyr Ile Cys
420 425 430

Glu Arg Ile Leu Arg Phe Tyr Arg Ser Gln Gln Lys Val Val Ile Thr
435 440 445

Lys Val Val Met His Pro Ser Lys Val Leu Glu Leu Gln Met Asn Lys
450 455 460

Arg Gly Phe Ser Met Glu Val Gly Gln Tyr Ile Phe Val Asn Cys Pro
465 470 475 480

Ser Ile Ser Leu Leu Glu Trp His Pro Phe Thr Leu Thr Ser Ala Pro
485 490 495

Glu Glu Asp Phe Phe Ser Ile His Ile Arg Ala Ala Gly Asp Trp Thr
500 505 510

Glu Asn Leu Ile Arg Ala Phe Glu Gln Gln Tyr Ser Pro Ile Pro Arg
515 520 525

Ile Glu Val Asp Gly Pro Phe Gly Thr Ala Ser Glu Asp Val Phe Gln
530 535 540

Tyr Glu Val Ala Val Leu Val Gly Ala Gly Ile Gly Val Thr Pro Phe
545 550 555 560

Ala Ser Ile Leu Lys Ser Ile Trp Tyr Lys Phe Gln Cys Ala Asp His
565 570 575

Asn Leu Lys Thr Lys Lys Ile Tyr Phe Tyr Trp Ile Cys Arg Glu Thr
580 585 590

Gly Ala Phe Ser Trp Phe Asn Asn Leu Leu Thr Ser Leu Glu Gln Glu
595 600 605

Met Glu Glu Leu Gly Lys Val Gly Phe Leu Asn Tyr Arg Leu Phe Leu
610 615 620

Thr Gly Trp Asp Ser Asn Ile Val Gly His Ala Ala Leu Asn Phe Asp
625 630 635 640

Lys Ala Thr Asp Ile Val Thr Gly Leu Lys Gln Lys Thr Ser Phe Gly
645 650 655

Arg Pro Met Trp Asp Asn Glu Phe Ser Thr Ile Ala Thr Ser His Pro
660 665 670

Lys Ser Val Val Gly Val Phe Leu Cys Gly Pro Arg Thr Leu Ala Lys
675 680 685

Ser Leu Arg Lys Cys Cys His Arg Tyr Ser Ser Leu Asp Pro Arg Lys
690 695 700

Val Gln Phe Tyr Phe Asn Lys Glu Asn Phe Glu Gln Lys Leu Ile Ser
705 710 715 720

Glu Glu Asp Leu

<210> 34
<211> 592
<212> PRT
<213> Artificial sequence

<220>
<223> an artificially synthesized sequence

<400> 34

Met Gly Asn Trp Val Val Asn His Trp Phe Ser Val Leu Phe Leu Val
1 5 10 15

Val Trp Leu Gly Leu Asn Val Phe Leu Phe Val Asp Ala Phe Leu Lys
20 25 30

Tyr Glu Lys Ala Asp Lys Tyr Tyr Tyr Thr Arg Lys Ile Leu Gly Ser
35 40 45

Thr Leu Ala Cys Ala Arg Ala Ser Ala Leu Cys Leu Asn Phe Asn Ser
50 55 60

Thr Leu Ile Leu Leu Pro Val Cys Arg Asn Leu Leu Ser Phe Leu Arg
65 70 75 80

```
Gly Thr Cys Ser Phe Cys Ser Arg Thr Leu Arg Lys Gln Leu Asp His
                85                  90                  95

Asn Leu Thr Phe His Lys Leu Val Ala Tyr Met Ile Cys Leu His Thr
            100                 105                 110

Ala Ile His Ile Ile Ala His Leu Phe Asn Phe Asp Cys Tyr Ser Arg
        115                 120                 125

Ser Arg Gln Ala Thr Asp Gly Ser Leu Ala Ser Ile Leu Ser Ser Leu
    130                 135                 140

Ser His Asp Glu Lys Lys Gly Gly Ser Trp Leu Asn Pro Ile Gln Ser
145                 150                 155                 160

Arg Asn Thr Thr Val Glu Tyr Val Thr Phe Thr Ser Ile Ala Gly Leu
                165                 170                 175

Thr Gly Val Ile Met Thr Ile Ala Leu Ile Leu Met Val Thr Ser Ala
            180                 185                 190

Thr Glu Phe Ile Arg Arg Ser Tyr Phe Glu Val Phe Trp Tyr Thr His
        195                 200                 205

His Leu Phe Ile Phe Tyr Ile Leu Gly Leu Gly Ile His Gly Ile Gly
    210                 215                 220

Gly Ile Val Arg Gly Gln Thr Glu Glu Ser Met Asn Glu Ser His Pro
225                 230                 235                 240

Arg Lys Cys Ala Glu Ser Phe Glu Met Trp Asp Asp Arg Asp Ser His
                245                 250                 255

Cys Arg Arg Pro Lys Phe Glu Gly His Pro Pro Glu Ser Trp Lys Trp
            260                 265                 270

Ile Leu Ala Pro Val Ile Leu Tyr Ile Cys Glu Arg Ile Leu Arg Phe
        275                 280                 285

Tyr Arg Ser Gln Gln Lys Val Val Ile Thr Lys Val Val Met His Pro
    290                 295                 300

Ser Lys Val Leu Glu Leu Gln Met Asn Lys Arg Gly Phe Ser Met Glu
305                 310                 315                 320

Val Gly Gln Tyr Ile Phe Val Asn Cys Pro Ser Ile Ser Leu Leu Glu
                325                 330                 335
```

```
Trp His Pro Phe Thr Leu Thr Ser Ala Pro Glu Glu Asp Phe Phe Ser
            340                 345                 350

Ile His Ile Arg Ala Ala Gly Asp Trp Thr Glu Asn Leu Ile Arg Ala
        355                 360                 365

Phe Glu Gln Gln Tyr Ser Pro Ile Pro Arg Ile Glu Val Asp Gly Pro
    370                 375                 380

Phe Gly Thr Ala Ser Glu Asp Val Phe Gln Tyr Glu Val Ala Val Leu
385                 390                 395                 400

Val Gly Ala Gly Ile Gly Val Thr Pro Phe Ala Ser Ile Leu Lys Ser
                405                 410                 415

Ile Trp Tyr Lys Phe Gln Cys Ala Asp His Asn Leu Lys Thr Lys Lys
            420                 425                 430

Ile Tyr Phe Tyr Trp Ile Cys Arg Glu Thr Gly Ala Phe Ser Trp Phe
        435                 440                 445

Asn Asn Leu Leu Thr Ser Leu Glu Gln Glu Met Glu Glu Leu Gly Lys
    450                 455                 460

Val Gly Phe Leu Asn Tyr Arg Leu Phe Leu Thr Gly Trp Asp Ser Asn
465                 470                 475                 480

Ile Val Gly His Ala Ala Leu Asn Phe Asp Lys Ala Thr Asp Ile Val
                485                 490                 495

Thr Gly Leu Lys Gln Lys Thr Ser Phe Gly Arg Pro Met Trp Asp Asn
            500                 505                 510

Glu Phe Ser Thr Ile Ala Thr Ser His Pro Lys Ser Val Val Gly Val
        515                 520                 525

Phe Leu Cys Gly Pro Arg Thr Leu Ala Lys Ser Leu Arg Lys Cys Cys
    530                 535                 540

His Arg Tyr Ser Ser Leu Asp Pro Arg Lys Val Gln Phe Tyr Phe Asn
545                 550                 555                 560

Lys Glu Asn Phe Trp Ser His Pro Gln Phe Glu Lys Gly Gly Gly Ser
                565                 570                 575

Gly Gly Gly Ser Gly Gly Ser Ala Trp Ser His Pro Gln Phe Glu Lys
```

580 585 590

<210> 35
<211> 6
<212> PRT
<213> Leporinae trouessart

<400> 35

Ser Ser Tyr Trp Ile Cys
1 5

<210> 36
<211> 18
<212> PRT
<213> Leporinae trouessart

<400> 36

Cys Ile Asn Ala Gly Asn Ser Gly Ser Thr Tyr Tyr Ala Ser Trp Ala
1 5 10 15

Lys Gly

<210> 37
<211> 20
<212> PRT
<213> Leporinae trouessart

<400> 37

Gly Ser Tyr Ser Tyr Gly Tyr Val Gly Tyr Val Tyr Thr Thr Ser Thr
1 5 10 15

Arg Leu Asp Leu
20

<210> 38
<211> 11
<212> PRT
<213> Leporinae trouessart

<400> 38

Gln Ala Ser Gln Asn Ile Gly Ser Asp Leu Ala
1 5 10

<210> 39
<211> 7
<212> PRT
<213> Leporinae trouessart

<400> 39

Arg Ala Ser Thr Leu Ala Ser

1 5

<210> 40
<211> 13
<212> PRT
<213> Leporinae trouessart

<400> 40

Gln Cys Thr Tyr Tyr Gly Ser Ile Tyr Val Pro Asn Ala
1 5 10

<210> 41
<211> 129
<212> PRT
<213> Leporinae trouessart

<400> 41

Gln Ser Leu Glu Glu Ser Gly Gly Asp Leu Val Lys Pro Gly Ala Ser
1 5 10 15

Leu Thr Leu Thr Cys Thr Ala Ser Gly Phe Ser Phe Ser Ser Ser Tyr
20 25 30

Trp Ile Cys Trp Val Arg Gln Ala Pro Gly Lys Gly Leu Glu Trp Ile
35 40 45

Ala Cys Ile Asn Ala Gly Asn Ser Gly Ser Thr Tyr Tyr Ala Ser Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Arg Gly Ser Tyr Ser Tyr Gly Tyr Val Gly Tyr Val Tyr Thr Thr
100 105 110

Ser Thr Arg Leu Asp Leu Trp Gly Gln Gly Thr Leu Val Thr Val Ser
115 120 125

Ser

<210> 42
<211> 111
<212> PRT
<213> Leporinae trouessart

<400> 42

Asp Val Val Met Thr Gln Thr Pro Ala Ser Val Ser Glu Pro Val Gly
1 5 10 15

Gly Thr Val Thr Ile Lys Cys Gln Ala Ser Gln Asn Ile Gly Ser Asp
20 25 30

Leu Ala Trp Tyr Gln Gln Lys Pro Gly Gln Pro Pro Lys Leu Leu Ile
35 40 45

Tyr Arg Ala Ser Thr Leu Ala Ser Gly Val Ser Ser Arg Phe Lys Gly
50 55 60

Ser Gly Ser Gly Thr Gln Phe Thr Leu Thr Ile Ser Asp Leu Glu Cys
65 70 75 80

Ala Asp Ala Ala Thr Tyr Tyr Cys Gln Cys Thr Tyr Tyr Gly Ser Ile
85 90 95

Tyr Val Pro Asn Ala Phe Gly Gly Gly Thr Glu Val Val Val Lys
100 105 110

<210> 43
<211> 6
<212> PRT
<213> Leporinae trouessart

<400> 43

Asp Trp Tyr Trp Val Gly
1 5

<210> 44
<211> 17
<212> PRT
<213> Leporinae trouessart

<400> 44

Cys Ile Val Thr Gly Asn Gly His Thr Tyr Tyr Ala Ser Trp Ala Lys
1 5 10 15

Gly

<210> 45
<211> 9
<212> PRT
<213> Leporinae trouessart

<400> 45

Gly Val Gly Ala Ser Arg Tyr Ala Leu

1 5

<210> 46
<211> 12
<212> PRT
<213> Leporinae trouessart

<400> 46

Gln Ala Ser Glu Ser Val Ala Ser Asn Trp Leu Ser
1 5 10

<210> 47
<211> 7
<212> PRT
<213> Leporinae trouessart

<400> 47

Phe Ala Ser Thr Leu Ala Ser
1 5

<210> 48
<211> 12
<212> PRT
<213> Leporinae trouessart

<400> 48

Ala Gly Tyr Lys Ser Asp Thr Thr Asp Gly Thr Ala
1 5 10

<210> 49
<211> 117
<212> PRT
<213> Leporinae trouessart

<400> 49

Gln Ser Leu Glu Glu Ser Gly Gly Asp Leu Val Lys Pro Gly Gly Thr
1 5 10 15

Leu Thr Leu Thr Cys Thr Ala Ser Gly Phe Ser Phe Ser Asp Trp Tyr
20 25 30

Trp Val Gly Trp Val Arg Gln Ala Pro Gly Lys Gly Leu Glu Trp Ile
35 40 45

Gly Cys Ile Val Thr Gly Asn Gly His Thr Tyr Tyr Ala Ser Trp Ala
50 55 60

Lys Gly Arg Phe Thr Ile Ser Lys Thr Ser Ser Thr Thr Val Thr Leu
65 70 75 80

Gln Leu Asn Ser Leu Thr Ala Ala Asp Thr Ala Thr Tyr Phe Cys Ala
85 90 95

Arg Gly Val Gly Ala Ser Arg Tyr Ala Leu Trp Gly Pro Gly Thr Leu
100 105 110

Val Thr Val Ser Ser
115

<210> 50
<211> 110
<212> PRT
<213> Leporinae trouessart

<400> 50

Ile Val Met Thr Gln Thr Pro Ser Ser Lys Ser Val Pro Val Gly Asp
1 5 10 15

Thr Val Thr Ile Asn Cys Gln Ala Ser Glu Ser Val Ala Ser Asn Trp
20 25 30

Leu Ser Trp Tyr Gln Gln Lys Pro Gly Gln Pro Pro Lys Leu Leu Ile
35 40 45

Tyr Phe Ala Ser Thr Leu Ala Ser Gly Val Pro Ser Arg Phe Lys Gly
50 55 60

Ser Gly Ser Gly Thr Gln Phe Thr Leu Thr Ile Ser Asp Val Val Cys
65 70 75 80

Asp Asp Ala Ala Thr Tyr Tyr Cys Ala Gly Tyr Lys Ser Asp Thr Thr
85 90 95

Asp Gly Thr Ala Phe Gly Gly Gly Thr Glu Val Val Val Lys
100 105 110

<210> 51
<211> 5
<212> PRT
<213> Leporinae trouessart

<400> 51

Tyr Tyr Phe Met Thr
1 5

<210> 52
<211> 16
<212> PRT
<213> Leporinae trouessart

<400> 52

Ile Ile Arg Val Asp Gly Ser Gly Tyr Tyr Ala Thr Trp Ala Lys Gly
1 5 10 15

<210> 53
<211> 8
<212> PRT
<213> Leporinae trouessart

<400> 53

Val Gly Asp Trp Ala Phe Asn Ile
1 5

<210> 54
<211> 11
<212> PRT
<213> Leporinae trouessart

<400> 54

Gln Ala Ser Glu Asp Ile Glu Ser Tyr Leu Ala
1 5 10

<210> 55
<211> 7
<212> PRT
<213> Leporinae trouessart

<400> 55

Arg Ala Ser Thr Leu Ala Ser
1 5

<210> 56
<211> 12
<212> PRT
<213> Leporinae trouessart

<400> 56

Gln Gln Gly His Thr Ile Asn Asn Ile Asp Asn Val
1 5 10

<210> 57
<211> 113
<212> PRT
<213> Leporinae trouessart

<400> 57

Gln Ser Leu Glu Glu Ser Gly Gly Arg Leu Val Thr Pro Gly Thr Pro
1 5 10 15

Leu Thr Leu Thr Cys Thr Ala Ser Gly Phe Ser Leu Ser Tyr Tyr Phe
20 25 30

Met Thr Trp Val Arg Gln Ala Pro Gly Lys Gly Leu Glu Tyr Ile Gly
35 40 45

Ile Ile Arg Val Asp Gly Ser Gly Tyr Tyr Ala Thr Trp Ala Lys Gly
50 55 60

Arg Phe Thr Ile Ser Arg Thr Ser Thr Thr Val Glu Leu Lys Ile Thr
65 70 75 80

Ser Pro Thr Thr Glu Asp Thr Ala Thr Tyr Phe Cys Ala Arg Val Gly
85 90 95

Asp Trp Ala Phe Asn Ile Trp Gly Pro Gly Thr Leu Val Thr Val Ser
100 105 110

Ser

<210> 58
<211> 109
<212> PRT
<213> Leporinae trouessart

<400> 58

Ile Lys Met Thr Gln Thr Pro Ala Ser Val Ser Thr Ala Val Gly Gly
1 5 10 15

Thr Val Ser Ile Asn Cys Gln Ala Ser Glu Asp Ile Glu Ser Tyr Leu
20 25 30

Ala Trp Phe Gln Gln Lys Pro Gly Gln Arg Pro Lys Leu Leu Ile Tyr
35 40 45

Arg Ala Ser Thr Leu Ala Ser Gly Val Ser Ser Arg Phe Lys Gly Ser
50 55 60

Gly Ser Gly Thr Glu Phe Thr Leu Thr Ile Ser Gly Val Glu Cys Ala
65 70 75 80

Asp Ala Ala Thr Tyr Tyr Cys Gln Gln Gly His Thr Ile Asn Asn Ile
85 90 95

Asp Asn Val Phe Gly Gly Gly Thr Glu Val Val Val Lys
100 105

<210> 59
<211> 5

<212> PRT
<213> Artificial sequence

<220>
<223> an artificially synthesized sequence

<400> 59

Asn Ala Trp Met His
1 5

<210> 60
<211> 19
<212> PRT
<213> Artificial sequence

<220>
<223> an artificially synthesized sequence

<400> 60

Gln Ile Lys Asp Lys Ser Gln Asn Tyr Ala Thr Tyr Val Ala Glu Ser
1 5 10 15

Val Lys Gly

<210> 61
<211> 11
<212> PRT
<213> Artificial sequence

<220>
<223> an artificially synthesized sequence

<400> 61

Val His Tyr Ala Ala Gly Tyr Gly Val Asp Ile
1 5 10

<210> 62
<211> 16
<212> PRT
<213> Artificial sequence

<220>
<223> an artificially synthesized sequence

<400> 62

Arg Ser Ser Gln Pro Leu Val His Ser Asn Arg Asn Thr Tyr Leu His
1 5 10 15

<210> 63
<211> 7
<212> PRT
<213> Artificial sequence

<220>
<223> an artificially synthesized sequence

<400> 63

Lys Val Ser Asn Arg Phe Ser
1 5

<210> 64
<211> 9
<212> PRT
<213> Artificial sequence

<220>
<223> an artificially synthesized sequence

<400> 64

Gly Gln Gly Thr Gln Val Pro Tyr Thr
1 5

<210> 65
<211> 122
<212> PRT
<213> Artificial sequence

<220>
<223> an artificially synthesized sequence

<400> 65

Gln Val Gln Leu Val Glu Ser Gly Gly Gly Val Val Gln Pro Gly Gly
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Asp Lys Ser Gln Asn Tyr Ala Thr Tyr Val Ala Glu
50 55 60

Ser Val Lys Gly Arg Phe Thr Ile Ser Arg Ala Asp Ser Lys Asn Ser
65 70 75 80

Ile Tyr Leu Gln Met Asn Ser Leu Lys Thr Glu Asp Thr Ala Val Tyr
85 90 95

Tyr Cys Arg Tyr Val His Tyr Ala Ala Gly Tyr Gly Val Asp Ile Trp
100 105 110

Gly Gln Gly Thr Thr Val Thr Val Ser Ser
115 120

<210> 66
<211> 112
<212> PRT
<213> Artificial sequence

<220>
<223> an artificially synthesized sequence

<400> 66

Asp Ile Val Met Thr Gln Ser Pro Leu Ser Leu Pro Val Thr Pro Gly
1 5 10 15

Glu Pro Ala Ser Ile Ser Cys Arg Ser Ser Gln Pro Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

<210> 67
<211> 6
<212> PRT
<213> Leporinae trouessart

<400> 67

Ser Asn Tyr Phe Met Cys
1 5

<210> 68
<211> 18
<212> PRT
<213> Leporinae trouessart

<400> 68

Cys Ile Tyr Thr Gly Ser Ser Gly Gly Thr Tyr Tyr Ala Thr Trp Ala
1 5 10 15

Lys Gly

<210> 69
<211> 9
<212> PRT
<213> Leporinae trouessart

<400> 69

Asp Thr Met Thr Ile Pro Phe Asn Leu
1               5

<210> 70
<211> 13
<212> PRT
<213> Leporinae trouessart

<400> 70

Gln Ala Ser Gln Ser Val Tyr Asn Asn Asn Asn Leu His
1               5                   10

<210> 71
<211> 7
<212> PRT
<213> Leporinae trouessart

<400> 71

Phe Ala Ser Thr Leu Ala Ser
1               5

<210> 72
<211> 13
<212> PRT
<213> Leporinae trouessart

<400> 72

Gln Gly Glu Phe Ser Cys Ser Ser Ala Asp Cys Asn Ala
1               5                   10

<210> 73
<211> 119
<212> PRT
<213> Leporinae trouessart

<400> 73

Gln Glu Gln Leu Glu Glu Ser Gly Gly Asp Leu Val Lys Pro Gly Ala
1               5                   10                  15

Ser Leu Thr Leu Thr Cys Thr Ala Ser Gly Phe Ser Phe Asn Ser Asn
            20                  25                  30

Tyr Phe Met Cys Trp Val Arg Gln Ala Pro Gly Lys Gly Leu Glu Trp

35 40 45

Ile Ala Cys Ile Tyr Thr Gly Ser Ser Gly Gly Thr Tyr Tyr Ala Thr
50 55 60

Trp Ala Lys Gly Arg Phe Thr Ile Ser Lys Thr Ser Ser Thr Thr Val
65 70 75 80

Thr Leu Gln Met Thr Ser Leu Thr Ala Ala Asp Thr Ala Thr Tyr Phe
85 90 95

Cys Ala Gly Asp Thr Met Thr Ile Pro Phe Asn Leu Trp Gly Pro Gly
100 105 110

Thr Leu Val Thr Val Ser Ser
115

<210> 74
<211> 112
<212> PRT
<213> Leporinae trouessart

<400> 74

Gln Val Leu Thr Gln Thr Ala Ser Pro Val Ser Ala Pro Val Gly Gly
1 5 10 15

Thr Val Thr Ile Asn Cys Gln Ala Ser Gln Ser Val Tyr Asn Asn Asn
20 25 30

Asn Leu His Trp Phe Gln Gln Lys Pro Gly Gln Arg Pro Lys Leu Leu
35 40 45

Ile Tyr Phe Ala Ser Thr Leu Ala Ser Gly Val Ser Ser Arg Phe Lys
50 55 60

Gly Ser Gly Ser Gly Thr Gln Phe Thr Leu Thr Ile Ser Gly Val Gln
65 70 75 80

Cys Asp Asp Ala Ala Thr Tyr Tyr Cys Gln Gly Glu Phe Ser Cys Ser
85 90 95

Ser Ala Asp Cys Asn Ala Phe Gly Gly Gly Thr Glu Val Val Val Lys
100 105 110

<210> 75
<211> 5
<212> PRT
<213> Leporinae trouessart

<400> 75

Asn Tyr Ala Met Ser
1 5

<210> 76
<211> 16
<212> PRT
<213> Leporinae trouessart

<400> 76

Ile Ile Ser Ser Ser Gly Ser Thr Tyr Tyr Ala Ser Trp Ala Lys Gly
1 5 10 15

<210> 77
<211> 10
<212> PRT
<213> Leporinae trouessart

<400> 77

Asp Leu Asp Ala Ser Thr Gly Thr Asp Ile
1 5 10

<210> 78
<211> 13
<212> PRT
<213> Leporinae trouessart

<400> 78

Gln Ala Ser Gln Ser Val Tyr Ser Asn Asn Tyr Leu Ser
1 5 10

<210> 79
<211> 7
<212> PRT
<213> Leporinae trouessart

<400> 79

Lys Ala Ser Thr Leu Ala Ser
1 5

<210> 80
<211> 12
<212> PRT
<213> Leporinae trouessart

<400> 80

Ala Gly Asp Tyr Lys Ser Ser Ser Asp Ile Arg Ala
1 5 10

<210> 81
<211> 115

<212> PRT
<213> Leporinae trouessart

<400> 81

Gln Ser Val Glu Glu Ser Gly Gly Arg Leu Val Thr Pro Gly Thr Pro
1 5 10 15

Leu Thr Leu Thr Cys Thr Val Ser Gly Phe Ser Leu Ser Asn Tyr Ala
20 25 30

Met Ser Trp Val Arg Gln Ala Pro Gly Lys Gly Leu Glu Trp Ile Gly
35 40 45

Ile Ile Ser Ser Ser Gly Ser Thr Tyr Tyr Ala Ser Trp Ala Lys Gly
50 55 60

Arg Phe Thr Ile Ser Lys Thr Ser Thr Thr Val Asp Leu Lys Ile Thr
65 70 75 80

Ser Pro Thr Thr Glu Asp Thr Ala Thr Tyr Phe Cys Ala Arg Asp Leu
85 90 95

Asp Ala Ser Thr Gly Thr Asp Ile Trp Gly Pro Gly Thr Leu Val Thr
100 105 110

Val Ser Ser
115

<210> 82
<211> 111
<212> PRT
<213> Leporinae trouessart

<400> 82

Ala Val Leu Thr Gln Thr Pro Ser Pro Val Ser Ala Ala Val Gly Gly
1 5 10 15

Thr Val Thr Ile Asn Cys Gln Ala Ser Gln Ser Val Tyr Ser Asn Asn
20 25 30

Tyr Leu Ser Trp Phe Gln Gln Lys Pro Gly Gln Pro Pro Lys Leu Leu
35 40 45

Ile Tyr Lys Ala Ser Thr Leu Ala Ser Gly Val Pro Ser Arg Phe Lys
50 55 60

Gly Ser Gly Ser Gly Thr Gln Phe Thr Leu Thr Ile Ser Asp Val Gln
65 70 75 80

```
Cys Asp Asp Ala Ala Thr Tyr Tyr Cys Ala Gly Asp Tyr Lys Ser Ser
                85                  90                  95

Ser Asp Ile Arg Ala Phe Gly Gly Gly Thr Glu Val Val Val Lys
            100                 105                 110


<210>  83
<211>  328
<212>  PRT
<213>  Artificial sequence

<220>
<223>  an artificially synthesized sequence

<400>  83

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Arg Gly Gly Pro Lys Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175
```

Glu Gln Tyr Ala Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Tyr Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Glu Ser Leu Ser Leu Ser Pro
325

<210> 84
<211> 328
<212> PRT
<213> Artificial sequence

<220>
<223> an artificially synthesized sequence

<400> 84

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser

50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Arg Gly Gly Pro Lys Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Ala Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Lys Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Tyr Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro
325

<210> 85
<211> 107
<212> PRT
<213> Artificial sequence

<220>
<223> an artificially synthesized sequence

<400> 85

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Cys
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 86
<211> 107
<212> PRT
<213> Artificial sequence

<220>
<223> an artificially synthesized sequence

<400> 86

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

```
Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105
```

## Description

[Technical Field]

The present disclosure relates to isolated antibodies that bind to or recognize any of the following proteins: XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, and TMPRSS4.

[Background Art]

Antibodies have received attention as drugs because of being highly stable in plasma and causing little side effects. Among them, many IgG-type antibody drugs have been launched, and a large number of antibody drugs are currently under development (NPLs 1 and 2).

Rituxan against CD20, cetuximab against EGFR, Herceptin against HER2, and the like have been approved so far as therapeutic drugs for cancer using antibody drugs (NPL 3). These antibody molecules bind to their antigens expressed on cancer cells and thereby exert cytotoxic activity against the cancer cells through ADCC activity, etc. Such cytotoxic activity based on ADCC activity, etc. is known to depend on the number of antigens expressed on target cells of therapeutic antibodies (NPL 4). Therefore, high expression levels of targeted antigens are preferred from the viewpoint of the effects of therapeutic antibodies. However, if an antigen, albeit having a high expression level, is expressed in normal tissues, the cytotoxic activity based on ADCC activity, etc. is exerted against the normal cells. Hence, side effects become a serious problem. Therefore, it is preferred that antigens targeted by therapeutic antibodies as therapeutic drugs for cancer should be expressed specifically on cancer cells. For example, an antibody molecule against EpCAM known as a cancer antigen had been considered promising as a therapeutic drug for cancer. However, the EpCAM is known to be also expressed in the pancreas. In actuality, it has been reported in clinical trials that the administration of an anti-EpCAM antibody causes pancreatitis as a side effect due to cytotoxic activity against the pancreas (NPL 5).

In the wake of the success of antibody drugs exerting cytotoxic activity based on ADCC activity, second-generation improved antibody molecules exerting strong cytotoxic activity have been reported as a result of, for example, enhancing ADCC activity by the removal of fucose from the N-linked oligosaccharide of a native human IgG1 Fc region (NPL 6) or enhancing ADCC activity by enhancing binding to FcyRIIIa through the amino acid substitution of a native human IgG1 Fc region (NPL 7). Improved antibody molecules exerting stronger cytotoxic activity, such as an antibody drug conjugate (ADC) containing an antibody conjugated with a drug having strong cytotoxic activity (NPL 8), and a low-molecular antibody exerting cytotoxic activity against cancer cells by recruiting T cells to the cancer cells (NPL 9) have also been reported as antibody drugs exerting cytotoxic activity against cancer cells under a mechanism other than NK cell-mediated ADCC activity as mentioned above.

Such antibody molecules exerting stronger cytotoxic activity can exert cytotoxic activity even against cancer cells expressing an antigen at a level that is not high, but also exert cytotoxic activity against normal tissues expressing the antigen at a low level, similarly to cancer cells. In actuality, EGFR-BiTE, a bispecific antibody against CD3 and EGFR, can exert strong cytotoxic activity against cancer cells and exert an antitumor effect, by recruiting T cells to the cancer cells, as compared with cetuximab, native human IgG1 against the EGFR. On the other hand, it has also been found that serious side effects appear by the administration of EGFR-BiTE to cynomolgus monkeys, because EGFR is also expressed in normal tissues (NPL 10). Also, ADC bivatuzumab mertansine containing mertansine conjugated with an antibody against CD44v6 highly expressed on cancer cells has been clinically found to cause severe dermal toxicity and hepatoxicity, because CD44v6 is also expressed in normal tissues (NPL 11).

As mentioned above, use of an antibody that can exert strong cytotoxic activity even against cancer cells expressing an antigen at low levels requires the target antigen to be expressed in an exceedingly cancer-specific manner. However, considering that a target antigen HER2 of Herceptin or a target antigen EGFR of cetuximab is also expressed in normal tissues, only a limited number of cancer antigens may be expressed in an exceedingly cancer-specific manner. Therefore, side effects ascribable to a cytotoxic effect on normal tissues may become a problem, though cytotoxic activity against cancer can be enhanced.

Various techniques have been developed as techniques applicable to second-generation antibody drugs. For example, techniques of improving effector functions, antigen-binding ability, pharmacokinetics, or stability or reducing a risk of immunogenicity have been reported (NPL 12). However, in order to solve the above-mentioned side effects and allow antibody drugs to act specifically on target cancer tissues, there is still a need to identify antigens that show almost no or little expression in normal tissues and are specifically expressed in cancer cells.

[Citation List]

[Non-Patent Literature]

[NPL 1] Monoclonal antibody successes in the clinic. Janice M Reichert, Clark J Rosensweig, Laura B Faden & Matthew C Dewitz, Nat. Biotechnol. (2005) 23, 1073 - 1078

[NPL 2] The therapeutic antibodies market to 2008. Pavlou AK, Belsey MJ., Eur. J. Pharm. Biopharm. (2005) 59 (3), 389-396

[NPL 3] Monoclonal antibodies: versatile platforms for cancer immunotherapy. Weiner LM, Surana R, Wang S., Nat. Rev. Immunol. (2010) 10 (5), 317-327

[NPL 4] Differential responses of human tumor cell lines to anti-p185HER2 monoclonal antibodies. Lewis GD, Figari I, Fendly B, Wong WL, Carter P, Gorman C, Shepard HM, Cancer Immunol. Immunotherapy (1993) 37, 255-263

[NPL 5] ING-1, a monoclonal antibody targeting Ep-CAM in patients with advanced adenocarcinomas. de Bono JS, Tolcher AW, Forero A, Vanhove GF, Takimoto C, Bauer RJ, Hammond LA, Patnaik A, White ML, Shen S, Khazaeli MB, Rowinsky EK, LoBuglio AF, Clin. Cancer Res. (2004) 10 (22), 7555-7565

[NPL 6] Non-fucosylated therapeutic antibodies as next-generation therapeutic antibodies. Satoh M, Iida S, Shitara K., Expert Opin. Biol. Ther. (2006) 6 (11), 1161-1173

[NPL 7] Optimizing engagement of the immune system by anti-tumor antibodies: an engineer's perspective. Desjarlais JR, Lazar GA, Zhukovsky EA, Chu SY., Drug Discov. Today (2007) 12 (21-22), 898-910

[NPL 8] Antibody-drug conjugates: targeted drug delivery for cancer. Alley SC, Okeley NM, Senter PD., Curr. Opin. Chem. Biol. (2010) 14 (4), 529-537

[NPL 9] BiTE: Teaching antibodies to engage T-cells for cancer therapy. Baeuerle PA, Kufer P, Bargou R., Curr. Opin. Mol. Ther. (2009) 11 (1), 22-30

[NPL 10] T cell-engaging BiTE antibodies specific for EGFR potently eliminate KRAS- and BRAF-mutated colorectal cancer cells. Lutterbuese R, Raum T, Kischel R, Hoffmann P, Mangold S, Rattel B, Friedrich M, Thomas O, Lorenczewski G, Rau D, Schaller E, Herrmann I, Wolf A, Urbig T, Baeuerle PA, Kufer P., Proc. Natl. Acad. Sci. U.S.A. (2010) 107 (28), 12605-12610

[NPL 11] Phase I trial with the CD44v6-targeting immunoconjugate bivatuzumab mertansine in head and neck squamous cell carcinoma. Riechelmann H, Sauter A, Golze W, Hanft G, Schroen C, Hoermann K, Erhardt T, Gronau S., Oral Oncol. (2008) 44 (9), 823-829

[NPL 12] Antibody engineering for the development of therapeutic antibodies. Kim SJ, Park Y, Hong HJ., Mol. Cells. (2005) 20 (1), 17-29

[Summary of Invention]

[Technical Problem]

The invention in the present disclosure was achieved in view of the above circumstances. In a non-limiting embodiment, an objective of this invention is to provide antibodies that bind to or recognize an antigen that shows almost no or little expression in normal tissues and is highly expressed specifically in cancer cells.

[Solution to Problem]

In a non-limiting embodiment, the present inventors conducted dedicated research and as a result discovered XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, and TMPRSS4 as cell surface proteins expressed at high levels in cancer tissues and at low levels in adjacent normal tissues and in normal tissues. Furthermore, the present inventors found that these proteins are highly cancer-specific antigens that are highly expressed in colorectal cancer tissues (particularly colorectal cancer tissues with a KRAS mutation) and/or lung cancer tissues.

The present disclosure is based on these findings, and specifically encompasses embodiments illustratively listed below:

[1] An isolated antibody that binds to any of the following proteins: XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, and TMPRSS4.

[2] The antibody of [1], which binds to an extracellular domain of the protein.

[3] The antibody of [1] or [2], which binds to any of:

(1) XPR1 represented by SEQ ID NO: 1,
(2) NOX1 represented by SEQ ID NO: 2,
(3) MARVELD3 isoform 1 represented by SEQ ID NO: 3,
(4) MARVELD3 isoform 2 represented by SEQ ID NO: 4,
(5) SPINT2 represented by SEQ ID NO: 5,
(6) MANSC1 represented by SEQ ID NO: 7,
(7) SLC12A2 represented by SEQ ID NO: 8,
(8) CDCP1 represented by SEQ ID NO: 9,
(9) SEZ6L2 represented by SEQ ID NO: 12,
(10) FLVCR1 represented by SEQ ID NO: 13,
(11) SLC7A5 represented by SEQ ID NO: 14,
(12) STEAP1 represented by SEQ ID NO: 15,
(13) MMP14 represented by SEQ ID NO: 16,
(14) TNFRSF21 represented by SEQ ID NO: 17, and
(15) TMPRSS4 represented by SEQ ID NO: 18.

[4] The antibody of [1] or [2], which binds to any of:

(1) an extracellular domain of XPR1, which is any of amino acids 1 to 108, 111 to 122, 159 to 171, 177 to 216, 423 to 448, 473 to 502, 660 to 670, 674 to 696, 177 to 190, 428 to 448, 660 to 670, 244, 256 to 273, 257 to 270, 258 to 264, 258 to 268, 256 to 270, 258 to 273, 260 to 270, 293 to 314, 336 to 343, 337 to 344, 338 to 341, 340 to 342, 340 to 344, 343 to 344, 340 to 345, 368 to 372, 392 to 398, 397 to 401, 398 to 402, 420 to 442, 420 to 506, 465 to 479, 497 to 507, 498 to 508, 529 to 555, 529 to 570, 582 to 586, 1 to 234, 1 to 236, 293 to 318, 367 to 442, 369 to 473, 500 to 507, 529 to 696, and 589 to 696 in the amino acid sequence represented by SEQ ID NO: 1;
(2) an extracellular domain of NOX1, which is any of amino acids 44 to 54, 131 to 161, 242 to 258, 1 to 4, 1 to 11, 18 to 55, 28 to 44, 31 to 44, 32 to 46, 34 to 50, 70 to 102, 70 to 103, 117 to 176, 120 to 172, 122 to 166, 122 to 172, 124 to 168, 190 to 208, 191 to 204, 223 to 266, 223 to 267, 227 to 269, 228 to 391, 228 to 396, 404, and 420 to 564 in the amino acid sequence represented by SEQ ID NO: 2;
(3) an extracellular domain of MARVELD3 isoform 1, which is any of amino acids 222 to 266, 223 to 269, 227 to 264, 227 to 268, 229 to 263, 231 to 265, 321 to 362, 322 to 357, 323 to 357, 324 to 357, and 324 to 358 in the amino acid sequence represented by SEQ ID NO: 3; and
(4) an extracellular domain of MARVELD3 isoform 2, which is any of amino acids 101 to 111, 163 to 198, 1 to 266, 1 to 270, 216 to 271, 222 to 269, 226 to 271, 227 to 268, 227 to 271, 248 to 271, 316 to 364, 322 to 360, 323 to 359, 324 to 360, 326 to 360, and 327 to 360 in the amino acid sequence represented by SEQ ID NO: 4.

[4A] The antibody of any one of [1], [2], [3](1), and [4](1), which can bind to XPR1, and is selected from the following:

(a1) an antibody comprising HVR-H1 consisting of the amino acid sequence of SEQ ID NO: 35, HVR-H2 consisting of the amino acid sequence of SEQ ID NO: 36, HVR-H3 consisting of the amino acid sequence of SEQ ID NO: 37, HVR-L1 consisting of the amino acid sequence of SEQ ID NO: 38, HVR-L2 consisting of the amino acid sequence of SEQ ID NO: 39, and HVR-L3 consisting of the amino acid sequence of SEQ ID NO: 40;
(a2) an antibody comprising the VH sequence of SEQ ID NO: 41 and the VL sequence of SEQ ID NO: 42;
(a3) an antibody that binds to the same epitope in XPR1 as the antibody of any one of (a1) to (a2);
(a4) an antibody that competes with the antibody of any one of (a1) to (a2) for binding to XPR1;
(a5) an antibody that blocks the binding of the antibody of any one of (a1) to (a2) to XPR1 by 50% or more in a competitive assay; and
(a6) an antibody comprising a VH sequence having at least 95% sequence identity with the amino acid sequence of SEQ ID NO: 41 and a VL sequence having at least 95% sequence identity with the amino acid sequence of SEQ ID NO: 42.

[4B] The antibody of any one of [1], [2], [3](2), and [4](2), which can bind to NOX1, and is selected from the following:

(b1) an antibody comprising HVR-H1 consisting of the amino acid sequence of SEQ ID NO: 67, HVR-H2 consisting of the amino acid sequence of SEQ ID NO: 68, HVR-H3 consisting of the amino acid sequence of SEQ ID NO: 69, HVR-L1 consisting of the amino acid sequence of SEQ ID NO: 70, HVR-L2 consisting of the amino acid sequence of SEQ ID NO: 71, and HVR-L3 consisting of the amino acid sequence of SEQ ID NO: 72;
(b2) an antibody comprising HVR-H1 consisting of the amino acid sequence of SEQ ID NO: 75, HVR-H2 consisting of the amino acid sequence of SEQ ID NO: 76, HVR-H3 consisting of the amino acid sequence of SEQ ID NO: 77, HVR-L1 consisting of the amino acid sequence of SEQ ID NO: 78, HVR-L2 consisting of the amino acid sequence of SEQ ID NO: 79, and HVR-L3 consisting of the amino acid sequence of SEQ ID NO: 80;
(b3) an antibody comprising the VH sequence of SEQ ID NO: 73 and the VL sequence of SEQ ID NO: 74;
(b4) an antibody comprising the VH sequence of SEQ ID NO: 81 and the VL sequence of SEQ ID NO: 82;
(b5) an antibody that binds to the same epitope in NOX1 as the antibody of any one of (b1) to (b4);
(b6) an antibody that competes with the antibody of any one of (b1) to (b4) for binding to NOX1;
(b7) an antibody that blocks the binding of the antibody of any one of (b1) to (b4) to NOX1 by 50% or more in a competitive assay;
(b8) an antibody comprising a VH sequence having at least 95% sequence identity with the amino acid sequence of SEQ ID NO: 73 and a VL sequence having at least 95% sequence identity with the amino acid sequence of SEQ ID NO: 74; and
(b9) an antibody comprising a VH sequence having at least 95% sequence identity with the amino acid sequence of SEQ ID NO: 81 and a VL sequence having at least 95% sequence identity with the amino acid sequence of SEQ ID NO: 82.

[4C] The antibody of any one of [1], [2], [3](3), [3](4), [4](3), and [4](4), which can bind to MARVELD3 isoform 1 and/or MARVELD3 isoform 2, and is selected from the following:

(c1) an antibody comprising HVR-H1 consisting of the amino acid sequence of SEQ ID NO: 43, HVR-H2 consisting of the amino acid sequence of SEQ ID NO: 44, HVR-H3 consisting of the amino acid sequence of SEQ ID NO: 45, HVR-L1 consisting of the amino acid sequence of SEQ ID NO: 46, HVR-L2 consisting of the amino acid sequence of SEQ ID NO: 47, and HVR-L3 consisting of the amino acid sequence of SEQ ID NO: 48;
(c2) an antibody comprising HVR-H1 consisting of the amino acid sequence of SEQ ID NO: 51, HVR-H2 consisting of the amino acid sequence of SEQ ID NO: 52, HVR-H3 consisting of the amino acid sequence of SEQ ID NO: 53, HVR-L1 consisting of the amino acid sequence of SEQ ID NO: 54, HVR-L2 consisting of the amino acid sequence of SEQ ID NO: 55, and HVR-L3 consisting of the amino acid sequence of SEQ ID NO: 56;
(c3) an antibody comprising the VH sequence of SEQ ID NO: 49 and the VL sequence of SEQ ID NO: 50;
(c4) an antibody comprising the VH sequence of SEQ ID NO: 57 and the VL sequence of SEQ ID NO: 58;
(c5) an antibody that binds to the same epitope in MARVELD3 isoform 1 and/or MARVELD3 isoform 2 as the antibody of any one of (c1) to (c4);
(c6) an antibody that competes with the antibody of any one of (c1) to (c4) for binding to MARVELD3 isoform 1 and/or MARVELD3 isoform 2;
(c7) an antibody that blocks the binding of the antibody of any one of (c1) to (c4) to MARVELD3 isoform 1 and/or MARVELD3 isoform 2 by 50% or more in a competitive assay; (c8) an antibody comprising a VH sequence having at least 95% sequence identity with the amino acid sequence of SEQ ID NO: 49 and a VL sequence having at least 95% sequence identity with the amino acid sequence of SEQ ID NO: 50; and
(c9) an antibody comprising a VH sequence having at least 95% sequence identity with the amino acid sequence of SEQ ID NO: 57 and a VL sequence having at least 95% sequence identity with the amino acid sequence of SEQ ID NO: 58.

[5] The antibody of any one of [1] to [4C], which has cytotoxic activity.
[6] The antibody of any one of [1] to [5], which has internalization activity.
[7] The antibody of any one of [1] to [6], which is a monoclonal antibody.
[8] The antibody of any one of [1] to [7], which is a human antibody, a humanized antibody, or a chimeric antibody.
[9] The antibody of any one of [1] to [8], which is an antibody fragment.
[10] The antibody of any one of [1] to [8], which is a full-length IgG antibody.
[11] The antibody of any one of [1] to [10], which is a multispecific antibody.

[12] The antibody of [11], further comprising a T cell receptor complex-binding domain.
[13] The antibody of [11] or [12], wherein the multispecific antibody comprises one binding domain for the protein.
[14] The antibody of any one of [11] to [13], comprising an Fc region with reduced Fcγ receptor-binding activity.
[15] The antibody of [14], wherein the Fc region has lower Fcγ receptor-binding activity than the Fc region of IgG1, IgG2, IgG3, or IgG4.
[16] The antibody of [12] or [15], wherein the antibody has cytotoxic activity, and the cytotoxic activity is a T cell-dependent cytotoxic activity.
[17] The antibody of any one of [12] to [16], wherein the T cell receptor complex-binding domain is a T cell receptor-binding domain having T cell receptor-binding activity.
[18] The antibody of any one of [12] to [16], wherein the T cell receptor complex-binding domain is a CD3-binding domain having CD3-binding activity.
[19] The antibody of [18], wherein the CD3-binding domain can bind to a CD3ε chain.
[20] The antibody of [18] or [19], wherein the CD3-binding domain comprises an antibody heavy chain variable region and an antibody light chain variable region.
[20A] The antibody of any one of [18] to [20], wherein the CD3-binding domain is selected from the following:

(d1) a domain comprising an antibody variable region having HVR-H1 consisting of the amino acid sequence of SEQ ID NO: 59, HVR-H2 consisting of the amino acid sequence of SEQ ID NO: 60, HVR-H3 consisting of the amino acid sequence of SEQ ID NO: 61, HVR-L1 consisting of the amino acid sequence of SEQ ID NO: 62, HVR-L2 consisting of the amino acid sequence of SEQ ID NO: 63, and HVR-L3 consisting of the amino acid sequence of SEQ ID NO: 64; (d2) a domain comprising an antibody variable region having the VH sequence of SEQ ID NO: 65 and the VL sequence of SEQ ID NO: 66;
(d3) a domain comprising an antibody variable region that binds to the same epitope in CD3 as the antibody variable region of any one of (d1) to (d2);
(d4) a domain comprising an antibody variable region that competes with the antibody variable region of any one of (d1) to (d2) for binding to CD3;
(d5) a domain comprising an antibody variable region that blocks the binding of the antibody variable region of any one of (d1) to (d2) to CD3 by 50% or more in a competitive assay; and
(d6) a domain comprising an antibody variable region comprising a VH sequence having at least 95% sequence identity with the amino acid sequence of SEQ ID NO: 65 and a VL sequence having at least 95% sequence identity with the amino acid sequence of SEQ ID NO: 66.

[21] The antibody of any one of [11] to [20A], wherein the multispecific antibody is a bispecific antibody.
[22] An isolated nucleic acid encoding the antibody of any one of [1] to [21].
[23] A host cell comprising the nucleic acid of [22].
[24] A method for producing the antibody of any one of [1] to [21], comprising culturing the host cell of [23] such that the antibody is produced.
[25] The method of [24], further comprising recovering the antibody from the host cell.
[26] An immunoconjugate comprising the antibody of any one of [1] to [21] and a cytotoxic agent.
[27] A pharmaceutical formulation comprising the antibody of any one of [1] to [21] and a pharmaceutically acceptable carrier.
[28] The antibody of any one of [1] to [21] for use as a pharmaceutical.
[29] The antibody of any one of [1] to [21] for use in treatment of cancer.
[30] The antibody of [29], wherein the cancer is lung cancer, and the antibody binds to any of XPR1, SPINT2, SEZ6L2, FLVCR1, SLC7A5, STEAP1, MMP14, TNFRSF21, and TMPRSS4.
[31] The antibody of [30], wherein the lung cancer does not have one or more selected from an EGFR driver mutation, a BRAF driver mutation, an ERBB2 driver mutation, ALK fusion gene expression, and RET/ROS1 fusion gene expression.
[32] The antibody of [30] or [31], wherein the lung cancer is lung adenocarcinoma.
[33] The antibody of [29], wherein the cancer is colorectal cancer, and the antibody binds to any of NOX1, MARVELD3 isoform 1, MARVELD3 isoform 2, SPINT2, MANSC1, SLC12A2, and CDCP1.
[34] The antibody of [33], wherein the colorectal cancer has a KRAS mutation.
[35] The antibody of any one of [1] to [21], which is for use in inducing cytotoxic activity.
[36] Use of the antibody of any one of [1] to [21] in the manufacture of a pharmaceutical.
[37] Use of the antibody of any one of [1] to [21] in the manufacture of a pharmaceutical for cancer treatment.
[38] The use of [37], wherein the cancer is lung cancer, and the antibody binds to any of XPR1, SPINT2, SEZ6L2, FLVCR1, SLC7A5, STEAP1, MMP14, TNFRSF21, and TMPRSS4.
[39] The use of [38], wherein the lung cancer does not have one or more selected from an EGFR driver mutation, a BRAF driver mutation, an ERBB2 driver mutation, ALK fusion gene expression, and RET/ROS1 fusion gene expression.

[40] The use of [38] or [39], wherein the lung cancer is lung adenocarcinoma.

[41] The use of [37], wherein the cancer is colorectal cancer, and the antibody binds to any of NOX1, MARVELD3 isoform 1, MARVELD3 isoform 2, SPINT2, MANSC1, SLC12A2, and CDCP1.

[42] The use of [41], wherein the colorectal cancer has a KRAS mutation.

[43] Use of the antibody of any one of [1] to [21] in the manufacture of a pharmaceutical that induces cytotoxic activity.

[44] A method for treating an individual having cancer, comprising administering an effective amount of the antibody of any one of [1] to [21] to the individual.

[45] The method of [44], wherein the cancer is lung cancer, and the antibody binds to any of XPR1, SPINT2, SEZ6L2, FLVCR1, SLC7A5, STEAP1, MMP14, TNFRSF21, and TMPRSS4.

[46] The method of [45], wherein the lung cancer does not have one or more selected from an EGFR driver mutation, a BRAF driver mutation, an ERBB2 driver mutation, ALK fusion gene expression, and RET/ROS1 fusion gene expression.

[47] The method of [45] or [46], wherein the lung cancer is lung adenocarcinoma.

[48] The method of [44], wherein the cancer is colorectal cancer, and the antibody binds to any of NOX1, MARVELD3 isoform 1, MARVELD3 isoform 2, SPINT2, MANSC1, SLC12A2, and CDCP1.

[49] The method of [48], wherein the colorectal cancer has a KRAS mutation.

[50] A method for inducing cytotoxic activity in an individual, comprising administering an effective amount of the antibody of any one of [1] to [21] to the individual to induce cytotoxic activity.

[51] A method for detecting the presence of XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, or TMPRSS4 in a biological sample, which comprises contacting the antibody of any one of [1] to [21] that can bind to a protein to be detected, with the biological sample under conditions permissive for binding of the antibody to the protein to be detected.

[52] A method for detecting a cancer cell in a biological sample, which comprises contacting the antibody of any one of [1] to [21] with the biological sample under conditions permissive for binding of the antibody to a protein that serves as an antigen of the antibody.

[53] A method for detecting a colorectal cancer cell in a biological sample, which comprises contacting the antibody of any one of [1] to [21] that can bind to NOX1, MARVELD3 isoform 1, MARVELD3 isoform 2, SPINT2, MANSC1, SLC12A2, or CDCP1, with the biological sample under conditions permissive for binding of the antibody to a protein that serves as an antigen of the antibody.

[54] A method for detecting a colorectal cancer cell having a KRAS mutation in a biological sample, which comprises contacting the antibody of any one of [1] to [21] that can bind to NOX1, MARVELD3 isoform 1, MARVELD3 isoform 2, SPINT2, MANSC1, SLC12A2, or CDCP1, with the biological sample under conditions permissive for binding of the antibody to a protein that serves as an antigen of the antibody.

[55] A method of detecting a lung cancer cell in a biological sample, which comprises contacting the antibody of any one of [1] to [21] that can bind to XPR1, SPINT2, SEZ6L2, FLVCR1, SLC7A5, STEAP1, MMP14, TNFRSF21, or TMPRSS4, with the biological sample under conditions permissive for binding of the antibody to a protein that serves as an antigen of the antibody.

[56] A method for detecting in a biological sample a lung cancer cell that does not have one or more selected from an EGFR driver mutation, a BRAF driver mutation, an ERBB2 driver mutation, ALK fusion gene expression, and RET/ROS1 fusion gene expression, wherein the method comprises contacting the antibody of any one of [1] to [21] that can bind to XPR1, SPINT2, SEZ6L2, FLVCR1, SLC7A5, STEAP1, MMP14, TNFRSF21, or TMPRSS4, with the biological sample under conditions permissive for binding of the antibody to a protein that serves as an antigen of the antibody.

[Brief Description of Drawings]

Fig. 1-1 shows the protein expression of each target antigen candidate. The vertical axis shows the name of the molecule and the quantification value of protein expression (iBAQ value), and the horizontal axis shows the analyzed tissues. All LC-MS data (N = 3) of all samples from each tissue were plotted. Closed circles (•) indicate colorectal cancer tissue samples with a KRAS mutation or lung cancer tissue samples with a driver mutation. Open circles (o) indicate samples without the above mutations.

Fig. 1-2: Refer to the description of Fig. 1-1.
Fig. 1-3: Refer to the description of Fig. 1-1.
Fig. 1-4: Refer to the description of Fig. 1-1.

Fig. 1-5: Refer to the description of Fig. 1-1.
Fig. 1-6: Refer to the description of Fig. 1-1.
Fig. 1-7: Refer to the description of Fig. 1-1.
Fig. 1-8: Refer to the description of Fig. 1-1.
Fig. 1-9: Refer to the description of Fig. 1-1.
Fig. 1-10: Refer to the description of Fig. 1-1.
Fig. 1-11: Refer to the description of Fig. 1-1.
Fig. 1-12: Refer to the description of Fig. 1-1.
Fig. 1-13: Refer to the description of Fig. 1-1.
Fig. 1-14: Refer to the description of Fig. 1-1.
Fig. 1-15: Refer to the description of Fig. 1-1.
Fig. 1-16: Refer to the description of Fig. 1-1.
Fig. 2 shows an alignment of the amino acid sequences of isoforms 1 and 2 of

MARVELD3 (SEQ ID NOs: 3 and 4, respectively). Extracellular regions are underlined based on Fig. 1A of Raleigh D.R. et.al., Mol. Biol. Cell 21, 2010.

Fig. 3-1: Comparison of the expression levels of MARVELD3 isoform 1- and 2-specific peptides in colorectal cancer samples. Fig. 3-1 shows the expression levels of MARVELD3 isoform 2, and Fig. 3-2 shows the expression levels of MARVELD3 isoform 1. Since EKPAEMFEF was identified as an isoform 2-specific peptide and QLDQQYTILR was identified as an isoform 1-specific peptide, the signal intensity of these peptides was used to compare the expression levels. The vertical axes show the signal intensity of the isoform 1- and 2-specific peptides, and the horizontal axes show the sample names of colorectal cancer tissues and adjacent normal tissues (each sample was analyzed by LC-MS at N=3). Black bars indicate samples with a KRAS mutation, and gray bars indicate samples without a KRAS mutation.
Fig. 3-2: Refer to the description of Fig. 3-1.
Fig. 4-1 shows the results of cell surface localization analyses of XPR1, NOX1, and MARVELD3 isoform 2 (SEQ ID NOs: 1, 2, and 4, respectively). In the full amino acid sequence of each protein, transmembrane regions are boxed with a single line and extracellular regions are boxed with a double line, according to the UniProt topology information. Dashed lines indicate peptides identified in overexpressing cells, and portions with black underlines and bold amino acid letters indicate peptides identified from the endogenous protein.
Fig. 4-2: Refer to the description of Fig. 4-1.
Fig. 4-3: Refer to the description of Fig. 4-1.
Fig. 5-1 shows the expression levels of XPR1 in NCI-H2227 cells and HLC-1 cells.
Fig. 5-2 shows the expression level of MARVELD3 isoform2 in Caco-2 cells.
Fig. 6 shows the Alexa488 staining of a fraction stained with CellTrace FarRed (cells transfected with an empty plasmid vector) and a fraction not stained with CellTrace FarRed (cells transfected with the human XPR1-Myc expression plasmid vector).
Fig. 7 shows the cell growth inhibition rate of an anti-human XPR1/anti-human CD3 bispecific antibody.
Fig. 8 shows the cell growth inhibition rate of an anti-human XPR1/anti-human CD3 bispecific antibody.
Fig. 9 shows the Alexa488 staining of a fraction stained with CellTrace FarRed (cells transfected with an empty plasmid vector) and a fraction not stained with CellTrace FarRed (cells transfected with a human MARVELD3 isoform2-expressing plasmid vector).
Fig. 10 shows the cell growth inhibition rate of an anti-human MARVELD3 isoform2/anti-human CD3 bispecific antibody.
Fig. 11 shows the Alexa488 staining of a fraction not stained with CellTrace FarRed and CellTrace Violet (cells transfected with human NOX1-Strep), a fraction stained with CellTrace FarRed and CellTrace Violet (cells transfected with NOX1_Nx1B_564-myc), and a fraction stained only with CellTrace FarRed (cells transfected with an empty plasmid vector).

[Description of Embodiments]

**I. DEFINITIONS**

The terms "anti-target protein antibody" and "an antibody that binds to a target protein" refer to an antibody that is capable of binding a target protein with sufficient affinity such that the antibody is useful as a diagnostic and/or therapeutic agent in targeting the target protein . In one embodiment, the extent of binding of an anti-target protein antibody to an unrelated, non-target protein is less than about 10% of the binding of the antibody to the target protein as measured, e.g., by a radioimmunoassay (RIA). In certain embodiments, an antibody that binds to a target protein has a dissociation constant (Kd) of 1 micro M or less, 100 nM or less, 10 nM or less, 1 nM or less, 0.1 nM or less, 0.01 nM or less, or 0.001 nM or less (e.g. $10^{-8}$ M or less, e.g. from $10^{-8}$ M to $10^{-13}$ M, e.g., from $10^{-9}$ M to $10^{-13}$ M). In certain embodiments, an anti-target protein antibody binds to an epitope of the target protein that is conserved among the target proteins from different species.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies; linear antibodies; single-chain antibody molecules (e.g. scFv); and multispecific antibodies formed from antibody fragments.

The terms "full length antibody," "intact antibody," and "whole antibody" are used herein interchangeably to refer to an antibody having a structure substantially similar to a native antibody structure or having heavy chains that contain an Fc region as defined herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity.

Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

A "functional Fc region" possesses an "effector function" of a native sequence Fc region. Exemplary "effector functions" include C1q binding; CDC; Fc receptor binding; ADCC; phagocytosis; down regulation of cell surface receptors (e.g. B cell receptor; BCR), etc. Such effector functions generally require the Fc region to be combined with a binding domain (e.g., an antibody variable domain) and can be assessed using various assays as disclosed, for example, in definitions herein.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called alpha, delta, epsilon, gamma, and mu, respectively.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "chimeric" antibody refers to an antibody in which a portion of the heavy and/or light chain is derived from a particular source or species, while the remainder of the heavy and/or light chain is derived from a different source or species.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

In an exemplary competition assay, an immobilized target antigen (XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, or TMPRSS4) is incubated in a solution comprising a first labeled antibody that binds to the target protein (e.g., XPB0062, NXA0125, NXA0164, MDA0279, or MDA0314) and a second unlabeled antibody that is being tested for its ability to compete with the first antibody for binding to the target protein. The second antibody may be present in a hybridoma supernatant. As a control, the immobilized target protein is incubated in a solution comprising the first labeled antibody but not the second unlabeled antibody. After incubation under conditions permissive for binding of the first antibody to the target protein, excess unbound antibody is removed, and the amount of label associated with the immobilized target protein is measured. If the amount of label associated with the immobilized target protein is substantially reduced in the test sample relative to the control sample, then that indicates that the second antibody is competing with the first antibody for binding to the target protein. See Harlow and Lane (1988) Antibodies: A Laboratory Manual ch.14 (Cold Spring Harbor Laboratory, Cold Spring Harbor, NY).

## Fc receptor

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, *e.g.*, Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.).

Binding to human FcRn *in vivo* and plasma half life of human FcRn high affinity binding polypeptides can be assayed, e.g., in transgenic mice or transfected human cell lines expressing human FcRn, or in primates to which the polypeptides with a variant Fc region are administered. WO 2000/42072 (Presta) describes antibody variants with increased or decreased binding to FcRs. See also, *e.g.,* Shields et al. J. Biol. Chem. 9(2):6591-6604 (2001).

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

## Antibody-dependent cell-mediated cytotoxicity

"Antibody-dependent cell-mediated cytotoxicity" or "ADCC" refers to a form of cytotoxicity in which the antibody Fc region bound onto Fc receptors (FcRs) present on certain cytotoxic cells (*e.g.* NK cells, neutrophils, and macrophages) enable these cytotoxic effector cells to bind specifically to an antigen-bearing target cell and subsequently kill the target cell with cytotoxins. The primary cells for mediating ADCC, NK cells, express Fc gamma RIII only, whereas monocytes express Fc gamma RI, Fc gamma RII, and Fc gamma RIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 or U.S. Patent No. 6,737,056 (Presta), may be performed. Useful effector cells for such assays include PBMC and NK cells. Alternatively, or additionally, ADCC activity of the molecule of interest may be assessed *in vivo*, *e.g.*, in an animal model such as that disclosed in Clynes et al. PNAS (USA) 95:652-656 (1998).

**Complement dependent cytotoxicity**

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, *e.g.,* as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, e.g., in US Patent No. 6,194,551 B1 and WO 1999/51642. See also, *e.g.,* Idusogie et al. J. Immunol. 164: 4178-4184 (2000).

**Human effector cells**

"Human effector cells" refer to leukocytes that express one or more FcRs and perform effector functions. In certain embodiments, the cells express at least Fc gamma RIII and perform ADCC effector function(s). Examples of human leukocytes which mediate ADCC include peripheral blood mononuclear cells (PBMC), natural killer (NK) cells, monocytes, cytotoxic T cells, and neutrophils. The effector cells may be isolated from a native source, e.g., from blood.

When a conjugate of the antibody of the present disclosure with a drug is incorporated into a cell, the conjugate-linked growth inhibitor or cytotoxic substance such as a toxic peptide can induce cell death of the cell that incorporated this antibody. Therefore, the antibody to which the growth inhibitor or the cytotoxic substance such as a toxic peptide is linked preferably also has internalization activity. In the present disclosure, "antibody having internalization activity" refers to an antibody that is transported into a cell (into the cytoplasm, vesicles, other organelles, and such) upon binding to a target protein on the cell surface. Whether or not an antibody has internalization activity can be confirmed using methods known to those skilled in the art. For example, the internalization activity can be confirmed by the method of contacting an antibody linked to a labeled substance with cells expressing its target protein and determining whether the labeled substance is incorporated into the cells, or the method of contacting an antibody linked to a growth inhibitor or a cytotoxic substance such as a toxic peptide with cells expressing its target protein and determining whether cell death is induced in the target protein-expressing cells.

An "immunoconjugate" is an antibody conjugated to one or more heterologous molecule(s), including but not limited to a cytotoxic agent.

The term "cytotoxic agent" as used herein refers to a substance that inhibits or prevents a cellular function and/or causes cell death or destruction. Cytotoxic agents include, but are not limited to, radioactive isotopes (e.g., $^{211}At$, $^{131}I$, $^{125}I$, $^{90}Y$, $^{186}Re$, $^{188}Re$, $^{153}Sm$, $^{212}Bi$, $^{32}P$, $^{212}Pb$ and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "naked antibody" refers to an antibody that is not conjugated to a heterologous moiety (e.g., a cytotoxic moiety) or radiolabel. The naked antibody may be present in a pharmaceutical formulation.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

An "effective amount" of an agent, e.g., a pharmaceutical formulation, refers to an amount effective, at dosages and for periods of time necessary, to achieve the desired therapeutic or prophylactic result.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the present disclosure are used to delay development of a disease or to slow the progression of a disease.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

**Cancer**

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

**Tumor**

The term "tumor" refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

Herein, the term "driver mutation" means a gene mutation that directly causes the occurrence or malignant transformation of cancer. For example, important driver mutations in lung cancer and colorectal cancer include gene mutations in ALK, RET/ROS1, KRAS, EGFR, BRAF, and ERBB2. Non-limiting examples of driver mutations include, for example, the mutations listed in Table 2.

In one embodiment, the antibodies of the present disclosure are useful for at least one of treatment, prevention, and diagnosis of lung cancer. In certain embodiments, the antibodies of the present disclosure are useful for at least one of treatment, prevention, and diagnosis of lung cancer that does not have one or more selected from an EGFR driver mutation, a BRAF driver mutation, an ERBB2 driver mutation, ALK fusion gene expression, and RET/ROS 1 fusion gene expression. In another embodiment, the antibodies of the present disclosure are useful in at least one of treatment, prevention, and diagnosis of colorectal cancer. In certain embodiments, the antibodies of the present disclosure are useful for at least one of treatment, prevention, and diagnosis of colorectal cancer with a KRAS mutation.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding" an antibody of the present disclosure refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

## II. COMPOSITIONS AND METHODS

In one aspect, the present disclosure is based, in part, on the discovery of proteins that are highly expressed specifically in cancer such as lung cancer and colorectal cancer. In certain embodiments, antibodies that bind to any of the following target proteins are provided: XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, and TMPRSS4. Antibodies of the present disclosure are useful, e.g., for the diagnosis or treatment of cancer such as lung cancer and colorectal cancer.

### Exemplary target proteins

The term "target protein," as used herein, refers to any native target protein from any vertebrate source, including mammals such as primates (e.g. humans) and rodents (e.g., mice and rats), unless otherwise indicated. The term encompasses a "full-length" unprocessed target protein as well as any form of target protein that results from processing in the cell. The term also encompasses naturally occurring variants of the target protein, e.g., splice variants or allelic variants. The amino acid sequences of exemplary target proteins of the present disclosure are shown in SEQ ID NO: 1 (XPR1), SEQ ID NO: 2 (NOX1), SEQ ID NO: 3 (MARVELD3 isoform 1), SEQ ID NO: 4 (MARVELD3 isoform 2), SEQ ID NO: 5 (SPINT2), SEQ ID NO: 7 (MANSC1), SEQ ID NO: 8 (SLC12A2), SEQ ID NO: 9 (CDCP1), SEQ ID NO: 12 (SEZ6L2), SEQ ID NO: 13 (FLVCR1), SEQ ID NO: 14 (SLC7A5), SEQ ID NO: 15 (STEAP1), SEQ ID NO: 16 (MMP14), SEQ ID NO: 17 (TNFRSF21), and SEQ ID NO: 18 (TMPRSS4). Explanations of exemplary target proteins of the present disclosure are provided below.

XPR1

XPR1 (xenotropic and polytropic retrovirus receptor) is a receptor for xenotropic/polytropic mouse leukemia viruses (X/P-MLVs) to infect and enter into cells. XPR1 consists of 696 amino acids, and has six asparagine-linked glycosylation sequences, eight cellular transmembrane domains, and four extracellular loops in this sequence (Proc Natl Acad Sci USA. 1999, 96:927-932. doi: 10.1073/pnas.96.3.927; Proc Natl Acad Sci USA. 1999, 96:1385-1390. doi: 10.1073/pnas.96.4.1385; and Nat Genet. 1999, 21:216-219). The inherent function of this protein in cells is unknown. No molecule highly homologous to XPR1 has been found in mammals, but in yeast, the SYG1 protein (J Biol Chem. 1995, 270:25435-25444), which is involved in the G protein-coupled division signaling, has been found to have a certain homology. The N-terminal hydrophilic region of XPR1 shows homology, although low, with NUC-2S of *Neurospora crassa,* and PHO81 and PHO85 of *S. cerevisiae* (Trends Genet. 1995, 11:209-211; Trends Biochem Sci. 1996, 21:383-387; and Mol Gen Genet. 1996, 252:709-716). Since these molecules are involved in the regulation of phosphate transport, it is considered that XPR1 may be involved in signal transduction and control of phosphate transport (Proc Natl Acad Sci USA. 1999, 96:1385-1390. doi: 10.1073/pnas.96.4.1385). In addition, it has been reported that RANKL stimulation increases the expression of XPR1 mRNA in bone marrow cells and macrophages, suggesting that the expression is regulated by the RANKL-RANK signal (Biochem Biophys Res Commun. 2010 Aug 20, 399(2):129-32).

XPR1 has four extracellular loops (ECL1-4), and ECL3 and ECL4 are especially important as receptors for virus entry. Amino acid mutations in these regions are known to influence differences in susceptibility to virus infection (J Virol. 2007 Oct, 81(19):10550-7; Retrovirology. 2009 Oct 7, 6():87; J Virol. 2010 Nov, 84(22):11970-80; J Virol. 1999 Nov, 73 (11):9362-8; Retrovirology. 2005 Dec 15, 2():76; Retrovirology. 2010 Nov30, 7:101; and Virology. 2016 Oct, 497: 53-58).

There have been no reports on the expression and function of XPR1 in cancer.

XPR1 [NP_004727.2] (SEQ ID NO: 1) consists of 696 amino acids. Based on analyses by various topology prediction programs (PSORT II, UniProt, Phobius, and PolyPhobius) and article information (Proc Natl Acad Sci USA. 1999, 96:927-932. doi: 10.1073/pnas.96.3.927; Proc Natl Acad Sci USA. 1999, 96:1385-1390. doi: 10.1073/pnas.96.4.1385; and Nat Genet. 1999, 21:216-219), the following amino acid regions may be epitopes for XPR1 isoform 1-recognizing TRAB antibodies: 244, 256-273, 257-270, 258-264, 258-268, 258-273, 260-270, 293-314, 336-343, 337-344, 338-341, 340-342, 340-344, 340-345, 368-372, 392-398, 397-401, 398-402, 420-442, 420-506, 465-479, 497-507, 498-508, 529-555, 529-570, and 582-586. On the other hand, two topology prediction programs (TMHMM and Tmpred) predict extracellular regions very differently, and the following amino acid regions may be epitopes for XPR1 isoform 1-recognizing TRAB antibodies: amino acid regions 1-234, 1-236, 293-318, 367-442, 369-473, 500-507, 529-696, and 589-696.

NOX1 (NADPH OXIDASE HOMOLOG 1, NOH1, MITOGENIC OXIDASE 1, MOX1, and GP91-2)

NADPH oxidase is a membrane-bound enzyme complex responsible for ROS production and is comprised of NOX family proteins which serve as catalytic domains and several other proteins. The NOX family is known to have seven family members, namely NOX 1 to 5 and DUOX1 and 2, and NOX1 is one of the family members (Physiol Rev. 2007, 87, 245-313; and Cell Mol. Life Sci. 2012, 69, 2327-2343).

ROS is generally considered to be involved in the development of various chronic diseases (arteriosclerosis, hypertension, and inflammation) by acting as a cytotoxic or mutagenic substance and damaging cells (Free Radical Biol. Med., 2007, 43, 332-347; and Cancer Sci., 2009, 100 (8), 1382-1388). On the other hand, elevated ROS levels have been observed in cancer, and ROS is considered to play an important role in the progression of cancer (Cancer. Lett., 2008, 266 (1), 37-52). In fact, an increase in NOX/DUOX expression has been observed in many cancer types (Anticancer Agents Med Chem. 2013, 13(3):502-14; and Clinical Science, 2015, 128, 863-875).

Increased expression of NOX1 was also observed in colorectal cancer, and in particular, K-Ras mutation has been reported to correspond with NOX1 expression (Int J Cancer. 2008, 123(1):100-7). KRas mutation-mediated activation of downstream signals induces senescence, and ROS produced by NOX1 reportedly plays an important role in inducing senescence (Genes Cells. 2013 Jan;18 (1):32-41).

NOX1 [NP_008983.2] (SEQ ID NO: 2) consists of 546 amino acids. Based on analyses by various topology prediction programs (PSORT II, UniProt, TMHMM, Tmpred, Phobius, and PolyPhobius) and article information (Gene. 2001, 16, 269(1-2):131-40; and Biochem Biophys Res Commun. 2014, 17;443(3):1060-5), the following amino acid regions may be epitopes for NOX1-recognizing TRAB antibodies: 1-4, 1-11, 18-55, 28-44, 31-44, 32-46, 34-50, 70-102, 70-103, 117-176, 120-172, 122-166, 122-172, 124-168, 190-208, 191-204, 223-266, 223-267, 227-269, 228-391, 228-396, 404, and 420-564.

MARVELD3 (MarvelD3, Marveld3, and marveld3)

The intercellular adhesion surface of epithelial cells and endothelial cells is called the tight junction (TJ) and is comprised of four different proteins: claudin, occludin, tricellulin, and marvelD3. Among them, occludin, tricellulin, and marvelD3 have a marvel domain and are therefore called the TJ-associated marvel protein (TAMP) family. These TJ proteins are all considered to be four-transmembrane proteins having intracellular N-terminal and C-terminal domains and two extracellular loops (Mol. Biol. Cell. 2010, 21, 1200-1213. doi:10.1091/mbc.E09-08-0734).

MarvelD3 has two isoforms (BMC Cell Biol. 2009, Dec 22, 10:95. doi: 10). Isoform 1 [NP_001017967.2] (SEQ ID NO: 3) encodes 410 amino acids, and isoform 2 [NP_443090.4] (SEQ ID NO: 4) encodes 401 amino acids. Amino acid sequence-based structure prediction using TMPRED also presumes that marvelD3 is, like other TJ proteins, a protein with intracellular N- and C-termini, four transmembrane domains, and two extracellular loops (http://www.ch.embnet.org/software/TMPRED_form.html).

In addition, comparison of the amino acid sequences of the two isoforms shows that both share common N-terminal 198 amino acids, but the subsequent C-terminal sequence is completely different between the two isoforms (BMC Cell Biol. 2009, Dec 22, 10:95. doi: 10). Expression of isoforms 1 and 2 at the gene level has been observed in cultured cells and various organs, but expression at the protein level has not been analyzed (BMC Cell Biol. 2009, Dec 22, 10:95. doi: 10). Furthermore, functional differences and such between the two isoforms are not known.

MarvelD3 has been shown to colocalize with occludin at the cell junction in both endogenous expression cell lines and forced expression cell lines (BMC Cell Biol. 2009, Dec 22, 10:95. doi: 10). Furthermore, on the cell membrane, marvelD3 has been shown to bind homophilically in *cis* and also heterophilically bind to both occludin and tricellulin in *cis,* indicating that marvelD3 is involved in TJ formation by interacting with other TAMPs (J Cell Sci 2013, 126: 554-564).

Little is known about the role of marvelD3 in cancer, except that its expression is reportedly reduced in undifferentiated pancreatic cancer cells and pancreatic cancer cells in which EMT is induced by snail (Exp Cell Res. 2011, Oct 1, 317(16): 2288-98).

Based on analyses by various topology prediction programs (PSORT II, UniProt, TMHMM, Tmpred, Phobius, and PolyPhobius) and article information (Mol. Biol. Cell. 2010, 21, 1200-1213. doi:10.1091/mbc.E09-08-0734; and BMC Cell Biol. 2009, Dec 22, 10:95. doi: 10), the following amino acid regions may be epitopes for MARVELD3 isoform 1-recognizing TRAB antibodies: 222-266, 223-269, 227-264, 227-268, 229-263, 231-265, 321-362, 322-357, 323-357, 324-357, and 324-358. Similarly, the following amino acid regions may be epitopes for MARVELD3 isoform 2-recognizing TRAB antibodies: 1-266, 1-270, 216-271, 222-269, 226-271, 227-268, 227-271, 248-271, 316-364, 322-360, 323-359, 324-360, 326-360, and 327-360. Meanwhile, some prediction programs give analysis results with three transmembrane domains, in contrast to what is mentioned above.

SPINT2

SPINT2 (Kunitz-type protease inhibitor 2) [NP_066925.1] (SEQ ID NO: 5) is a transmembrane protein having two extracellular Kunitz domains that inhibit serine proteases (https://www.ncbi.nlm.nih.gov/protein/NP_066925.1).

MANSC1

MANSC1 (MANSC domain-containing protein 1) is a protein represented by [NP_060520.2] (SEQ ID NO: 7) (https://www.ncbi.nlm.nih.gov/protein/NP_060520.2).

SLC12A2

SLC12A2 (solute carrier family 12 member 2) [NP_001037.1] (SEQ ID NO: 8) is a membrane protein involved in the secretion and uptake of sodium and chlorides, and is said to have an important role in regulating ionic balance and cell volume (https://www.ncbi.nlm.nih.gov/protein/NP_001037.1).

CDCP1

CDCP1 (CUB domain-containing protein 1) [NP_073753.3] (SEQ ID NO: 9) is a transmembrane protein having three extracellular domains and is known to be phosphorylated by Src family kinases (https://www.ncbi.nlm.nih.gov/protein/NP_073753.3).

SEZ6L2

SEZ6L2 (Seizure 6-like protein 2) [NP_963869.2] (SEQ ID NO: 12) is a protein localized in the cell membrane, and is said to be possibly involved in endoplasmic reticulum function in nerve cells (https://www.uniprot.org/uniprot/Q6UXD5; and https://www.ncbi.nlm.nih.gov/protein/NP_963869.2).

FLVCR1

FLVCR1 (feline leukemia virus subgroup C receptor-related protein 1) [NP_054772.1] (SEQ ID NO: 13) is a heme transporter and is considered to play an important role in the formation of red blood cells (https://www.ncbi.nlm.nih.gov/protein/NP_054772.1).

SLC7A5

SLC7A5 (solute carrier family 7 member 5) is a protein represented by [NP_003477.4] (SEQ ID NO: 14) and is also called large neutral amino acids transporter small subunit 1 (https://www.ncbi.nlm.nih.gov/protein/NP_003477.4).

STEAP1

STEAP1 (metalloreductase STEAP1) is a protein represented by [NP_036581.1] (SEQ ID NO: 15), and has the ability to reduce $Fe^{3+}$ to $Fe^{2+}$ and $Cu^{2+}$ to $Cu^{1+}$ as a metalloreductase (https://www.uniprot.org/uniprot/Q9UHE8; and https://www.ncbi.nlm.nih.gov/protein/NP_036581.1).

MMP14

MMP14 (matrix metalloproteinase-14) is a protein represented by [NP_004986.1] (SEQ ID NO: 16), and has endopeptidase activity for degrading extracellular matrices and such (https://www.ncbi.nlm.nih.gov/protein/NP_004986.1).

TNFRSF21

TNFRSF21 (tumor necrosis factor receptor superfamily member 21) is a protein represented by [NP_055267.1] (SEQ ID NO: 17), and is considered to induce apoptosis by activating nuclear factor kappa-B and mitogen-activated protein kinase 8. It is also called Death receptor 6 (DR6) (https://www.ncbi.nlm.nih.gov/protein/NP_055267.1).

TMPRSS4

TMPRSS4 (transmembrane protease serine 4) is a protein represented by [NP_063947.1] (SEQ ID NO: 18), and it may be a serine protease (https://www.ncbi.nlm.nih.gov/protein/NP_063947.1).

**A. Exemplary Antibodies**

In one aspect, the present disclosure provides isolated antibodies that bind to any of the following target proteins: XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, and TMPRSS4. In certain embodiments, an antibody of the present disclosure binds to an extracellular domain of the above-mentioned target protein. In certain embodiments, an antibody of the present disclosure has cytotoxic activity. In certain embodiments, an antibody of the present disclosure has internalization activity.

In one aspect, the present disclosure provides an antibody that can bind to XPR1 (XPR1-binding antibody). In certain embodiments, the epitope of the XPR1-binding antibody of the present disclosure is the sequence of any of the following in the XPR1 amino acid sequence represented by SEQ ID NO: 1:
amino acid positions 1 to 108, 111 to 122, 159 to 171, 177 to 216, 423 to 448, 473 to 502, 660 to 670, 674 to 696, 177 to 190, 428 to 448, 660 to 670, 244, 256 to 273, 257 to 270, 258 to 264, 258 to 268, 256 to 270, 258 to 273, 260 to 270, 293 to 314, 336 to 343, 337 to 344, 338 to 341, 340 to 342, 340 to 344, 343 to 344, 340 to 345, 368 to 372, 392 to 398, 397 to 401, 398 to 402, 420 to 442, 420 to 506, 465 to 479, 497 to 507, 498 to 508, 529 to 555, 529 to 570, 582 to 586, 1 to 234, 1 to 236, 293 to 318, 367 to 442, 369 to 473, 500 to 507, 529 to 696, and 589 to 696.

In certain embodiments, the XPR1-binding antibody of the present disclosure is any one of (a1) to (a6):

(a1) an antibody comprising HVR-H1 consisting of the amino acid sequence of SEQ ID NO: 35, HVR-H2 consisting of the amino acid sequence of SEQ ID NO: 36, HVR-H3 consisting of the amino acid sequence of SEQ ID NO: 37, HVR-L1 consisting of the amino acid sequence of SEQ ID NO: 38, HVR-L2 consisting of the amino acid sequence of SEQ ID NO: 39, and HVR-L3 consisting of the amino acid sequence of SEQ ID NO: 40;
(a2) an antibody comprising the VH sequence of SEQ ID NO: 41 and the VL sequence of SEQ ID NO: 42;
(a3) an antibody that binds to the same epitope in XPR1 as the antibody of any one of (a1) to (a2);
(a4) an antibody that competes with the antibody of any one of (a1) to (a2) for binding to XPR1;
(a5) an antibody that blocks the binding of the antibody of any one of (a1) to (a2) to XPR1 by 50% or more in a competitive assay; and
(a6) an antibody comprising a VH sequence having at least 95% sequence identity with the amino acid sequence of SEQ ID NO: 41 and a VL sequence having at least 95% sequence identity with the amino acid sequence of SEQ ID NO: 42.

In one aspect, the present disclosure provides an antibody that can bind to NOX1 (NOX1-binding antibody). In certain embodiments, the epitope of the NOX1-binding antibody of the present disclosure is the sequence of any of the following in the amino acid sequence represented by SEQ ID NO: 2:
amino acid positions 44 to 54, 131 to 161, 242 to 258, 1 to 4, 1 to 11, 18 to 55, 28 to 44, 31 to 44, 32 to 46, 34 to 50, 70 to 102, 70 to 103, 117 to 176, 120 to 172, 122 to 166, 122 to 172, 124 to 168, 190 to 208, 191 to 204, 223 to 266, 223 to 267, 227 to 269, 228 to 391, 228 to 396, 404, and 420 to 564.

In certain embodiments, the NOX1-binding antibody of the present disclosure is any one of (b1) to (a9):

(b1) an antibody comprising HVR-H1 consisting of the amino acid sequence of SEQ ID NO: 67, HVR-H2 consisting of the amino acid sequence of SEQ ID NO: 68, HVR-H3 consisting of the amino acid sequence of SEQ ID NO: 69, HVR-L1 consisting of the amino acid sequence of SEQ ID NO: 70, HVR-L2 consisting of the amino acid sequence of SEQ ID NO: 71, and HVR-L3 consisting of the amino acid sequence of SEQ ID NO: 72;
(b2) an antibody comprising HVR-H1 consisting of the amino acid sequence of SEQ ID NO: 75, HVR-H2 consisting of the amino acid sequence of SEQ ID NO: 76, HVR-H3 consisting of the amino acid sequence of SEQ ID NO: 77, HVR-L1 consisting of the amino acid sequence of SEQ ID NO: 78, HVR-L2 consisting of the amino acid sequence of SEQ ID NO: 79, and HVR-L3 consisting of the amino acid sequence of SEQ ID NO: 80;
(b3) an antibody comprising the VH sequence of SEQ ID NO: 73 and the VL sequence of SEQ ID NO: 74;
(b4) an antibody comprising the VH sequence of SEQ ID NO: 81 and the VL sequence of SEQ ID NO: 82;
(b5) an antibody that binds to the same epitope in NOX1 as the antibody of any one of (b1) to (b4);
(b6) an antibody that competes with the antibody of any one of (b1) to (b4) for binding to NOX1;
(b7) an antibody that blocks the binding of the antibody of any one of (b1) to (b4) to NOX1 by 50% or more in a competitive assay;
(b8) an antibody comprising a VH sequence having at least 95% sequence identity with the amino acid sequence of SEQ ID NO: 73 and a VL sequence having at least 95% sequence identity with the amino acid sequence of SEQ ID NO: 74; and
(b9) an antibody comprising a VH sequence having at least 95% sequence identity with the amino acid sequence of SEQ ID NO: 81 and a VL sequence having at least 95% sequence identity with the amino acid sequence of SEQ ID NO: 82.

In one aspect, the present disclosure provides an antibody that can bind to MARVELD3 isoform1 (MARVELD3 isoform1-binding antibody). In certain embodiments, the epitope of the MARVELD3 isoform1-binding antibody of the present disclosure is the sequence of any of the following in the amino acid sequence represented by SEQ ID NO: 3: amino acid positions 222 to 266, 223 to 269, 227 to 264, 227 to 268, 229 to 263, 231 to 265, 321 to 362, 322 to 357, 323 to 357, 324 to 357, and 324 to 358.

In one aspect, the present disclosure provides an antibody that can bind to MARVELD3 isoform2 (MARVELD3 isoform2-binding antibody). In certain embodiments, the epitope of the MARVELD3 isoform2-binding antibody of the present disclosure is the sequence of any of the following in the amino acid sequence represented by SEQ ID NO: 4: amino acid positions 101 to 111, 163 to 198, 1 to 266, 1 to 270, 216 to 271, 222 to 269, 226 to 271, 227 to 268, 227 to 271, 248 to 271, 316 to 364, 322 to 360, 323 to 359, 324 to 360, 326 to 360, and 327 to 360.

In certain embodiments, the MARVELD3 isoform2-binding antibody of the present disclosure is any one of (c1) to (c9):

(c1) an antibody comprising HVR-H1 consisting of the amino acid sequence of SEQ ID NO: 43, HVR-H2 consisting of the amino acid sequence of SEQ ID NO: 44, HVR-H3 consisting of the amino acid sequence of SEQ ID NO: 45, HVR-L1 consisting of the amino acid sequence of SEQ ID NO: 46, HVR-L2 consisting of the amino acid sequence of SEQ ID NO: 47, and HVR-L3 consisting of the amino acid sequence of SEQ ID NO: 48;
(c2) an antibody comprising HVR-H1 consisting of the amino acid sequence of SEQ ID NO: 51, HVR-H2 consisting of the amino acid sequence of SEQ ID NO: 52, HVR-H3 consisting of the amino acid sequence of SEQ ID NO: 53, HVR-L1 consisting of the amino acid sequence of SEQ ID NO: 54, HVR-L2 consisting of the amino acid sequence of SEQ ID NO: 55, and HVR-L3 consisting of the amino acid sequence of SEQ ID NO: 56;
(c3) an antibody comprising the VH sequence of SEQ ID NO: 49 and the VL sequence of SEQ ID NO: 50;
(c4) an antibody comprising the VH sequence of SEQ ID NO: 57 and the VL sequence of SEQ ID NO: 58;
(c5) an antibody that binds to the same epitope in MARVELD3 isoform 1 and/or MARVELD3 isoform 2 as the antibody of any one of (c1) to (c4);
(c6) an antibody that competes with the antibody of any one of (c1) to (c4) for binding to MARVELD3 isoform 1 and/or MARVELD3 isoform 2;
(c7) an antibody that blocks the binding of the antibody of any one of (c1) to (c4) to MARVELD3 isoform 1 and/or MARVELD3 isoform 2 by 50% or more in a competitive assay;
(c8) an antibody comprising a VH sequence having at least 95% sequence identity with the amino acid sequence of SEQ ID NO: 49 and a VL sequence having at least 95% sequence identity with the amino acid sequence of SEQ ID NO: 50; and
(c9) an antibody comprising a VH sequence having at least 95% sequence identity with the amino acid sequence of SEQ ID NO: 57 and a VL sequence having at least 95% sequence identity with the amino acid sequence of SEQ ID NO: 58.

In a further aspect of the present disclosure, an anti-target protein antibody according to any of the above embodiments is a monoclonal antibody, including a chimeric, humanized or human antibody. In one embodiment, an anti-target protein antibody is an antibody fragment, e.g., a Fv, Fab, Fab', scFv, diabody, or $F(ab')_2$ fragment. In another embodiment, the antibody is, for example, a full length IgG antibody or a full length antibody of other antibody class or isotype as defined herein.

In a further aspect, an anti-target protein antibody according to any of the above embodiments may incorporate any of the features, singly or in combination, as described in Sections 1-7 below:

### *1. Antibody Affinity*

In certain embodiments, an antibody provided herein has a dissociation constant (Kd) of 1 micro M or less, 100 nM or less, 10 nM or less, 1 nM or less, 0.1 nM or less, 0.01 nM or less, or 0.001 nM or less (e.g. $10^{-8}$ M or less, e.g. from $10^{-8}$ M to $10^{-13}$ M, e.g., from $10^{-9}$ M to $10^{-13}$ M).

In one embodiment, Kd is measured by a radiolabeled antigen binding assay (**RIA**). In one embodiment, an RIA is performed with the Fab version of an antibody of interest and its antigen. For example, solution binding affinity of Fabs for antigen is measured by equilibrating Fab with a minimal concentration of ($^{125}I$)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER (registered trademark) multi-well plates (Thermo Scientific) are coated overnight with 5 micro g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23 degrees C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20 (registered trademark)) in PBS. When the plates have dried, 150 micro l/well of scintillant (MICROSCINT-20 ™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using a BIACORE (registered trademark) surface plasmon resonance assay. For example, an assay using a BIACORE (registered trademark)-2000 or a BIACORE(registered trademark)-3000 (BIAcore, Inc., Piscataway, NJ) is performed at 25 degrees C with immobilized antigen CM5 chips at ~10 response units (RU). In one embodiment, carboxymethylated dextran biosensor chips (CM5, BIACORE, Inc.) are activated with *N*-ethyl-*N*'- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 micro g/ml (~0.2 micro M) before injection at a flow rate of 5 micro 1/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25 degrees C at a flow rate of approximately 25 micro l/min. Association rates ($k_{on}$) and dissociation rates ($k_{off}$) are calculated using a simple one-to-one Langmuir binding model (BIACORE (registered trademark) Evaluation Software version 3.2) by simultaneously fitting the association and dissociation sensorgrams. The equilibrium dissociation constant (Kd) is calculated as the ratio $k_{off}/k_{on}$. See, e.g., Chen et al., J. Mol. Biol. 293:865-881 (1999). If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 degrees C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO™ spectrophotometer (ThermoSpectronic) with a stirred cuvette.

### *2. Antibody Fragments*

In certain embodiments, an antibody provided herein is an antibody fragment. Antibody fragments include, but are not limited to, Fab, Fab', Fab'-SH, $F(ab')_2$, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and $F(ab')_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### *3. Chimeric and Humanized Antibodies*

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

***4. Human Antibodies***

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HuMab (registered trademark) technology; U.S. Patent No. 7,041,870 describing K-M MOUSE (registered trademark) technology, and U.S. Patent Application Publication No. US 2007/0061900, describing VelociMouse (registered trademark) technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

***5. Library-Derived Antibodies***

Antibodies of the present disclosure may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352:

624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### *6. Multispecific Antibodies*

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for a target protein and the other is for any other antigen. In certain embodiments, bispecific antibodies may bind to two different epitopes of a target protein. Bispecific antibodies may also be used to localize cytotoxic agents to cells which express a target protein. Bispecific antibodies can be prepared as full length antibodies or antibody fragments.

In certain embodiments, a multispecific antibody provided herein is a T cell-redirecting antibody (TRAB). Such antibodies interact with two or more of the target proteins of the present disclosure, that is, proteins that are highly expressed specifically in cancer cells, and a protein expressed in T cells. Thus, they have the effect of enhancing antitumor activities by crosslinking T cells having cytotoxic activity with cancer cells.

Blinatumomab, which is a BiTE molecule, and Catumaxomab are known as bispecific antibodies that recognize a protein expressed on T cells (CD3ε or TCR) and a protein expressed on cancer cells (a cancer antigen). These molecules can bind to a cancer antigen and the CD3ε chain expressed on a T cell with each of their two antigen-binding domains (scFv or Fab), and form intercellular crosslinks between the T cells and the cancer antigen-expressing cells. This way, such T cell-redirecting antibodies can use T cells as effector cells to induce strong cytotoxic activity against cancer antigen-expressing cells.

In certain embodiments, a multispecific antibody of the present disclosure comprises a binding domain for a target protein of the present disclosure and a T cell receptor complex-binding domain and optionally has T cell dependent cytotoxic activity. Such a multispecific antibody of the present disclosure can redirect T cells to cells that express the target protein of the present disclosure on the cell surface, thereby inducing T cell-mediated cytotoxic activity against the cells. In certain embodiments, a multispecific antibody of the present disclosure comprises an Fc region that has reduced Fcγ receptor-binding activity, and optionally comprises an Fc region that has a lower Fcγ receptor-binding activity than the Fc region of IgG1, IgG2, IgG3, or IgG4. In certain embodiments, a multispecific antibody of the present disclosure comprises one binding domain for a target protein of the present disclosure. The T cell receptor complex-binding domain contained in the multispecific antibody of the present disclosure is, in one embodiment, a T cell receptor-binding domain having activity to bind to a T cell receptor, or in another embodiment, a CD3-binding domain having CD3-binding activity. In certain embodiments, the CD3 binding domain contained in the multispecific antibody of the present disclosure is a domain that is capable of binding to the CD3ε chain and optionally comprises an antibody heavy chain variable region and an antibody light chain variable region.

In certain embodiments, the CD3-binding domain contained in a multispecific antibody of the present disclosure is any one of the following:

(d1) a domain comprising an antibody variable region having HVR-H1 consisting of the amino acid sequence of SEQ ID NO: 59, HVR-H2 consisting of the amino acid sequence of SEQ ID NO: 60, HVR-H3 consisting of the amino acid sequence of SEQ ID NO: 61, HVR-L1 consisting of the amino acid sequence of SEQ ID NO: 62, HVR-L2 consisting of the amino acid sequence of SEQ ID NO: 63, and HVR-L3 consisting of the amino acid sequence of SEQ ID NO: 64;

(d2) a domain comprising an antibody variable region having the VH sequence of SEQ ID NO: 65 and the VL sequence of SEQ ID NO: 66;
(d3) a domain comprising an antibody variable region that binds to the same epitope in CD3 as the antibody variable region of any one of (d1) to (d2);
(d4) a domain comprising an antibody variable region that competes with the antibody variable region of any one of (d1) to (d2) for binding to CD3;
(d5) a domain comprising an antibody variable region that blocks the binding of the antibody variable region of any one of (d1) to (d2) to CD3 by 50% or more in a competitive assay; and
(d6) a domain comprising an antibody variable region that has a VH sequence having at least 95% sequence identity with the amino acid sequence of SEQ ID NO: 65 and a VL sequence having at least 95% sequence identity with the amino acid sequence of SEQ ID NO: 66.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see,e.g. Gruber et al., J. Immunol., 152:5368 (1994)); and preparing trispecific antibodies as described, e.g., in Tutt et al. J. Immunol. 147: 60 (1991).
Engineered antibodies with three or more functional antigen binding sites, including "Octopus antibodies," are also included herein (see, e.g. US 2006/0025576A1).
The antibody or fragment herein also includes a "Dual Acting Fab" or "DAF" comprising an antigen binding site that binds to a target protein as well as another, different antigen (see, US 2008/0069820, for example).

***7. Antibody Variants***

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

**a) Substitution. Insertion, and Deletion Variants**

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

[Table 1]

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |

(continued)

| Original Residue | Exemplary Substitutions | Preferred Substitutions |
|---|---|---|
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex may be analyzed to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion of an enzyme (e.g. for ADEPT) or a polypeptide which increases the plasma half-life of the antibody to the N- or C-terminus of the antibody.

**b) Glycosylation variants**

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the present disclosure may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about +/- 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

c) **Fc region variants**

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region represented by SEQ ID NO: 6, 19, 20, or 21, respectively) comprising an amino acid modification (e.g. a substitution) at one or more amino acid positions.

In certain embodiments, the present disclosure contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACT1™ non-radioactive cytotoxicity assay for flow cytometry (CellTechnology, Inc. Mountain View, CA; and CytoTox 96 (registered trademark) non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo*, *e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and increased binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which increase binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

d) **Cysteine engineered antibody variants**

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

## B. Antibody Production Methods

Methods for producing antibodies having the desired binding activity are known to those skilled in the art, and the antibodies may be obtained as polyclonal or monoclonal antibodies. Monoclonal antibodies derived from mammals may be suitably produced as the antibodies of the present disclosure. Such mammalian-derived monoclonal antibodies include antibodies produced by hybridomas and antibodies produced by host cells transformed with an expression vector carrying an antibody gene by genetic engineering techniques.

There is no particular limitation on the mammal to be immunized for obtaining antibodies. It is preferable to select the mammal by considering its compatibility with the parent cells to be used in cell fusion for hybridoma production. In general, rabbits, monkeys, and rodents such as mice, rats, and hamsters are suitably used.

The above animals are immunized with a sensitizing antigen by known methods. Generally performed immunization methods include, for example, intraperitoneal or subcutaneous injection of a sensitizing antigen into mammals. Specifically, a sensitizing antigen is appropriately diluted with Phosphate-Buffered Saline (PBS), physiological saline, or the like. If desired, a conventional adjuvant such as Freund's complete adjuvant is mixed with the antigen, and the mixture is emulsified. Then, the sensitizing antigen is administered to a mammal several times at 4- to 21-day intervals. Appropriate carriers may be used in immunization with the sensitizing antigen. In particular, when a low-molecular-weight partial peptide is used as the sensitizing antigen, it is sometimes desirable to couple the sensitizing antigen peptide to a carrier protein such as albumin or keyhole limpet hemocyanin for immunization.

Alternatively, hybridomas producing a desired antibody can be prepared using DNA immunization as mentioned below. DNA immunization is an immunization method that confers immunostimulation by expressing a sensitizing antigen in an animal immunized as a result of administering a vector DNA constructed to allow expression of an antigen protein-encoding gene in the animal. As compared to conventional immunization methods in which a protein antigen is administered to animals to be immunized, DNA immunization is expected to be superior in that:

- immunostimulation can be provided while retaining the structure of a membrane protein; and
- there is no need to purify the antigen for immunization.

In order to prepare a monoclonal antibody of the present disclosure using DNA immunization, first, a DNA expressing an antigen protein is administered to an animal to be immunized. The antigen protein-encoding DNA can be synthesized by known methods such as PCR. The obtained DNA is inserted into an appropriate expression vector, and then this is administered to an animal to be immunized. Preferably used expression vectors include, for example, commercially-available expression vectors such as pcDNA3.1. Vectors can be administered to an organism using conventional methods. For example, DNA immunization is performed by using a gene gun to introduce expression vector-coated gold particles into cells in the body of an animal to be immunized.

After immunizing a mammal as described above, an increase in the titer of an antigen-binding antibody is confirmed in the serum. Then, immune cells are collected from the mammal, and then subjected to cell fusion. In particular, splenocytes are preferably used as immune cells.

A mammalian myeloma cell is used as a cell to be fused with the above-mentioned immune cells. The myeloma cells preferably comprise a suitable selection marker for screening. A selection marker confers characteristics to cells for their survival (or death) under a specific culture condition. Hypoxanthine-guanine phosphoribosyltransferase deficiency (hereinafter abbreviated as HGPRT deficiency) and thymidine kinase deficiency (hereinafter abbreviated as TK deficiency) are known as selection markers. Cells with HGPRT or TK deficiency have hypoxanthine-aminopterin-thymidine sensitivity (hereinafter abbreviated as HAT sensitivity). HAT-sensitive cells cannot synthesize DNA in a HAT selection medium, and are thus killed. However, when the cells are fused with normal cells, they can continue DNA synthesis using the salvage pathway of the normal cells, and therefore they can grow even in the HAT selection medium.

HGPRT-deficient and TK-deficient cells can be selected in a medium containing 6-thioguanine, 8-azaguanine (hereinafter abbreviated as 8AG), or 5'-bromodeoxyuridine. Normal cells are killed because they incorporate these pyrimidine analogs into their DNA. Meanwhile, cells that are deficient in these enzymes can survive in the selection medium, since they cannot incorporate these pyrimidine analogs. In addition, a selection marker referred to as G418 resistance provided by the neomycin-resistant gene confers resistance to 2-deoxystreptamine antibiotics (gentamycin analogs). Various types of myeloma cells that are suitable for cell fusion are known.

For example, myeloma cells including the following cells can be preferably used:

P3(P3×63Ag8.653) (J. Immunol. (1979) 123 (4), 1548-1550);
P3×63Ag8U.1 (Current Topics in Microbiology and Immunology (1978)81, 1-7);
NS-1 (C. Eur. J. Immunol. (1976)6 (7), 511-519);
MPC-11 (Cell (1976) 8 (3), 405-415);
SP2/0 (Nature (1978) 276 (5685), 269-270);
FO (J. Immunol. Methods (1980) 35 (1-2), 1-21);
S194/5.XX0.BU.1 (J. Exp. Med. (1978) 148 (1), 313-323);
R210 (Nature (1979) 277 (5692), 131-133), etc.

Cell fusions between the immunocytes and myeloma cells are essentially carried out using known methods, for example, a method by Kohler and Milstein et al. (Methods Enzymol. (1981) 73: 3-46).

More specifically, cell fusion can be carried out, for example, in a conventional culture medium in the presence of a cell fusion-promoting agent. The fusion-promoting agents include, for example, polyethylene glycol (PEG) and Sendai virus (HVJ). If required, an auxiliary substance such as dimethyl sulfoxide is also added to improve fusion efficiency.

The ratio of immunocytes to myeloma cells may be arbitrarily set, preferably, for example, one myeloma cell for every one to ten immunocytes. Culture media to be used for cell fusions include, for example, media that are suitable for the growth of myeloma cell lines, such as RPMI1640 medium and MEM medium, and other conventional culture medium used for this type of cell culture. In addition, serum supplements such as fetal calf serum (FCS) may be preferably added to the culture medium.

For cell fusion, predetermined amounts of the above immune cells and myeloma cells are mixed well in the above culture medium. Then, a PEG solution (for example, the average molecular weight is about 1,000 to 6,000) prewarmed to about 37°C is added thereto at a concentration of generally 30% to 60% (w/v). The mixed solution is gently mixed to produce desired fusion cells (hybridomas). Then, an appropriate culture medium mentioned above is gradually added to the cells, and this is repeatedly centrifuged to remove the supernatant. Thus, cell fusion agents and such which are unfavorable to hybridoma growth can be removed.

The hybridomas thus obtained can be selected by culture using a conventional selective medium, for example, HAT medium (a culture medium containing hypoxanthine, aminopterin, and thymidine). Culture is continued in the above medium using the HAT medium for a period of time sufficient to kill cells other than the desired hybridomas (non-fused cells). Typically, the period is several days to several weeks. Then, hybridomas producing the desired antibody are screened and singly cloned by conventional limiting dilution methods.

The hybridomas thus obtained can be selected using a selection medium based on the selection marker possessed by the myeloma used for cell fusion. For example, HGPRT- or TK-deficient cells can be selected by culture using the HAT medium (a culture medium containing hypoxanthine, aminopterin, and thymidine). Specifically, when HAT-sensitive myeloma cells are used for cell fusion, cells successfully fused with normal cells can selectively proliferate in the HAT medium. Culture is continued in the above medium using the HAT medium for a period of time sufficient to kill cells other than the desired hybridomas (non-fused cells). Specifically, desired hybridomas can be selected by culture for generally several days to several weeks. Then, hybridomas producing the desired antibody are screened and singly cloned by conventional limiting dilution methods.

Screening and single cloning of desired antibodies can be suitably performed by screening methods based on known antigen-antibody reaction. For example, a desired antibody can be selected by screening using fluorescence activated cell sorting (FACS). FACS is a system that enables measurement of the binding of an antibody to cell surface by analyzing cells contacted with a fluorescent antibody using laser beam, and measuring the fluorescence emitted from individual cells.

To screen for hybridomas that produce a monoclonal antibody of the present disclosure by FACS, cells that express the antigen bound by the produced antibody are first prepared. Preferred cells used for screening are mammalian cells that are forced to express the antigen. By using mammalian cells that are used as the host cell but have not been transformed as a control, the activity of an antibody to bind to the cell-surface antigen can be selectively detected. Specifically, hybridomas producing a desired monoclonal antibody can be obtained by selecting hybridomas that produce an antibody which binds to cells forced to express the antigen but not to the host cell.

Alternatively, cells expressing the antigen of interest are immobilized and the activity of an antibody to bind to the antigen-expressing cells can be assessed based on the principle of ELISA. For example, antigen-expressing cells are immobilized to the wells of an ELISA plate. Culture supernatants of hybridomas are contacted with the immobilized cells in the wells, and antibodies that bind to the immobilized cells are detected. When the monoclonal antibodies are derived from mouse, antibodies bound to the cells can be detected using an anti-mouse immunoglobulin antibody. Hybridomas producing a desired antibody having the antigen-binding ability are selected by the above screening, and they can be cloned by a limiting dilution method or the like.

Monoclonal antibody-producing hybridomas thus prepared can be passaged in a conventional culture medium. The hybridomas can be stored in liquid nitrogen for a long period.

The above hybridomas are cultured by a conventional method, and desired monoclonal antibodies can be obtained from the culture supernatants. Alternatively, the hybridomas are administered to and grown in compatible mammals, and monoclonal antibodies can be obtained from the ascites. The former method is suitable for obtaining antibodies with high purity.

Antibodies that are encoded by antibody genes cloned from antibody-producing cells such as the above hybridomas can also be preferably used. A cloned antibody gene is inserted into an appropriate vector, and this is introduced into a host to express the antibody encoded by the gene. Methods for isolating antibody genes, inserting the genes into vectors, and transforming host cells have already been established, for example, by Vandamme et al. (Eur. J. Biochem. (1990) 192(3), 767-775). Methods for producing recombinant antibodies are also known as described below.

Generally, to obtain a cDNA encoding the antibody variable region (V region), total RNA is first extracted from hybridomas. For example, the following methods can be used as methods for extracting mRNAs from cells:

- the guanidine ultracentrifugation method (Biochemistry (1979) 18(24), 5294-5299), and
- the AGPC method (Anal. Biochem. (1987) 162(1), 156-159).

Extracted mRNAs can be purified using the mRNA Purification Kit (GE Healthcare Bioscience) or such. Alternatively, kits for extracting total mRNA directly from cells, such as the QuickPrep mRNA Purification Kit (GE Healthcare Bioscience), are also commercially available. mRNAs can be prepared from hybridomas using such kits. cDNAs encoding the antibody V region can be synthesized from the prepared mRNAs using a reverse transcriptase. cDNAs can be synthesized using the AMV Reverse Transcriptase First-strand cDNA Synthesis Kit (Seikagaku Corporation) or such. Furthermore, the SMART RACE cDNA amplification kit (Clontech) and the PCR-based 5'-RACE method (Proc. Natl. Acad. Sci. USA (1988) 85(23), 8998-9002; Nucleic Acids Res. (1989) 17(8), 2919-2932) can be appropriately used to synthesize and amplify cDNAs. In such a cDNA synthesis process, appropriate restriction enzyme sites described below may be introduced into both ends of a cDNA.

The cDNA fragment of interest is purified from the resulting PCR product, and then this is ligated to a vector DNA. A recombinant vector is thus constructed, and introduced into *E. coli* or such. After colony selection, the desired recombinant vector can be prepared from the colony-forming *E. coli*. Then, whether the recombinant vector has the cDNA nucleotide sequence of interest is tested by a known method such as the dideoxy nucleotide chain termination method.

The 5'-RACE method which uses primers to amplify the variable region gene is conveniently used for isolating the gene encoding the variable region. First, a 5'-RACE cDNA library is constructed by cDNA synthesis using RNAs extracted from hybridoma cells as a template. A commercially available kit such as the SMART RACE cDNA amplification kit is appropriately used to synthesize the 5'-RACE cDNA library.

The antibody gene is amplified by PCR using the prepared 5'-RACE cDNA library as a template. Primers for amplifying the mouse antibody gene can be designed based on known antibody gene sequences. The nucleotide sequences of the primers vary depending on the immunoglobulin subclass. Therefore, it is preferable that the subclass is determined in advance using a commercially available kit such as the Iso Strip mouse monoclonal antibody isotyping kit (Roche Diagnostics).

Specifically, for example, primers that allow amplification of genes encoding γ1, y2a, y2b, and γ3 heavy chains and κ and λ light chains are used to isolate mouse IgG-encoding genes. In general, a primer that anneals to a constant region site close to the variable region is used as a 3'-side primer to amplify an IgG variable region gene. Meanwhile, a primer attached to a 5' RACE cDNA library construction kit is used as a 5'-side primer.

Immunoglobulins composed of a combination of heavy and light chains may be reshaped using the thus amplified PCR products. A desired antibody can be selected by screening using the antigen-binding activity of a reshaped immunoglobulin as an indicator. The screening can be carried out, for example, by the following steps:

(1) contacting a desired antigen-expressing cell with an antibody comprising the V region encoded by a cDNA obtained from a hybridoma;
(2) detecting the binding of the antibody to the antigen-expressing cell; and
(3) selecting an antibody that binds to the antigen-expressing cell.

Methods for detecting the binding of an antibody to the antigen-expressing cells are known. Specifically, the binding of an antibody to the antigen-expressing cells can be detected by the above-described techniques such as FACS. Fixed samples of the antigen-expressing cells may be appropriately used to assess the binding activity of an antibody.

For antibody screening methods that use the binding activity as an indicator, panning methods that use phage vectors can also be used suitably. Screening methods using phage vectors are advantageous when the antibody genes are obtained from a polyclonal antibody-expressing cell population as heavy-chain and light-chain subclass libraries. Genes encoding the heavy-chain and light-chain variable regions can be linked by an appropriate linker sequence to form a single-chain Fv (scFv). Phages expressing scFv on their surface can be produced by inserting a scFv-encoding gene into a phage vector. The phages are contacted with an antigen of interest. Then, a DNA encoding scFv having the binding activity of interest can be isolated by collecting phages bound to the antigen. This process can be repeated as necessary to enrich scFv having the binding activity of interest.

After isolation of the cDNA encoding the V region of the antibody of interest, the cDNA is digested with restriction enzymes that recognize the restriction sites introduced into both ends of the cDNA. Preferred restriction enzymes recognize and cleave a nucleotide sequence that occurs in the nucleotide sequence of the antibody gene at a low frequency. Furthermore, a restriction site for an enzyme that produces a sticky end is preferably introduced into a vector to insert a single-copy digested fragment in the correct orientation. The cDNA encoding the V region of the antibody is digested as described above, and this is inserted into an appropriate expression vector to construct an antibody expression vector. In this case, if a gene encoding the antibody constant region (C region) and a gene encoding the above V region are fused in-frame, a chimeric antibody is obtained. Herein, a "chimeric antibody" means that the origin of the constant region is different from that of the variable region. Thus, in addition to mouse/human heterochimeric antibodies, human/human allochimeric antibodies are included in the chimeric antibodies of the present disclosure. A chimeric antibody expression vector can be constructed by inserting the above V region gene into an expression vector that already has the constant region. Specifically, for example, a recognition sequence for a restriction enzyme that excises the above V region gene can be appropriately placed on the 5' side of an expression vector carrying a DNA that encodes a desired antibody constant region (C region). A chimeric antibody expression vector is constructed by fusing in-frame two genes digested with the same combination of restriction enzymes.

To produce a monoclonal antibody, antibody genes are inserted into an expression vector so that the genes are expressed under the control of an expression regulatory region. The expression regulatory region for antibody expression includes, for example, enhancers and promoters. Furthermore, an appropriate signal sequence may be attached to the amino terminus so that the expressed antibody is secreted to the outside of cells. The signal sequence is cleaved from the carboxyl terminus of the expressed polypeptide, and the resulting antibody can be secreted to the outside of cells. Then, appropriate host cells are transformed with the expression vector, and recombinant cells expressing the antibody-encoding DNA can be obtained.

DNAs encoding the antibody heavy chain (H chain) and light chain (L chain) are separately inserted into different expression vectors to express the antibody gene. An antibody molecule having the H and L chains can be expressed by co-transfecting the same host cell with vectors inserted with the H chain and L chain. Alternatively, host cells can be transformed with a single expression vector into which DNAs encoding the H and L chains are inserted (see WO 94/11523).

There are many known combinations of host cells and expression vectors for antibody preparation by introducing isolated antibody genes into appropriate hosts. All these expression systems are applicable to isolation of the domains that bind to the target proteins of the present disclosure described above and T cell receptor complex-binding domain.

Appropriate eukaryotic cells used as host cells include animal cells, plant cells, and fungal cells. Specifically, the animal cells include, for example, the following cells.

(1) mammalian cells: CHO, COS, myeloma, baby hamster kidney (BHK), HeLa, Vero, or such;
(2) amphibian cells: Xenopus oocytes, or such; and
(3) insect cells: sf9, sf21, Tn5, or such.

In addition, as a plant cell, an antibody gene expression system using cells derived from the *Nicotiana* genus such as *Nicotiana tabacum* is known. Callus cultured cells can be appropriately used to transform plant cells.

Furthermore, the following cells can be used as fungal cells:

yeasts: the *Saccharomyces* genus such as *Saccharomyces cerevisiae,* and the *Pichia* genus such as *Pichia pastoris;* and

filamentous fungi: the *Aspergillus* genus such as *Aspergillus niger.*

Furthermore, antibody gene expression systems that utilize prokaryotic cells are also known. For example, when using bacterial cells, *E. coli* cells, *Bacillus subtilis* cells, and such can suitably be utilized. Expression vectors carrying the antibody genes of interest are introduced into these cells by transfection. The transfected cells are cultured *in vitro,* and the desired antibody can be prepared from the culture of transformed cells.

In addition to the above-described host cells, transgenic animals can also be used to produce a recombinant antibody. That is, the antibody can be obtained from an animal into which the gene encoding the antibody of interest is introduced. For example, the antibody gene can be constructed as a fusion gene by inserting in frame into a gene that encodes a protein produced specifically in milk. Goat β-casein or such can be used, for example, as the protein secreted in milk. DNA fragments containing the fused gene inserted with the antibody gene is injected into a goat embryo, and then this embryo is introduced into a female goat. Desired antibodies can be obtained as a protein fused with the milk protein from milk produced by the transgenic goat born from the embryo-recipient goat (or progeny thereof). In addition, to increase the volume of milk containing the desired antibody produced by the transgenic goat, hormones can be administered to the transgenic goat as necessary (Bio/Technology (1994) 12 (7), 699-702).

When an antigen-binding molecule described herein is administered to human, an antigen-binding domain derived from a genetically recombinant antibody that has been artificially modified to reduce the heterologous antigenicity against human and such, can be appropriately used as the various binding domains in the molecule when domains comprising an antibody variable region are used. Such genetically recombinant antibodies include, for example, humanized antibodies. These modified antibodies are appropriately produced by known methods.

An antibody variable region used to produce the various binding domains of antigen-binding molecules described herein is generally formed by three complementarity-determining regions (CDRs) that are separated by four framework regions (FRs). CDR is a region that substantially determines the binding specificity of an antibody. The amino acid sequences of CDRs are highly diverse. On the other hand, the FR-forming amino acid sequences often have high identity even among antibodies with different binding specificities. Therefore, generally, the binding specificity of a certain antibody can be introduced into another antibody by CDR grafting.

A humanized antibody is also called a reshaped human antibody. Specifically, humanized antibodies prepared by grafting the CDR of a non-human animal antibody such as a mouse antibody to a human antibody and such are known. Common genetic engineering techniques for obtaining humanized antibodies are also known. Specifically, for example, overlap extension PCR is known as a method for grafting a mouse antibody CDR to a human FR. In overlap extension PCR, a nucleotide sequence encoding a mouse antibody CDR to be grafted is added to primers for synthesizing a human antibody FR. Primers are prepared for each of the four FRs. It is generally considered that when grafting a mouse CDR to a human FR, selecting a human FR that has high identity to a mouse FR is advantageous for maintaining the CDR function. That is, it is generally preferable to use a human FR comprising an amino acid sequence which has high identity to the amino acid sequence of the FR adjacent to the mouse CDR to be grafted.

Nucleotide sequences to be ligated are designed so that they will be connected to each other in frame. Human FRs are individually synthesized using the respective primers. As a result, products in which the mouse CDR-encoding DNA is attached to the individual FR-encoding DNAs are obtained. Nucleotide sequences encoding the mouse CDR of each product are designed so that they overlap with each other. Then, complementary strand synthesis reaction is conducted to anneal the overlapping CDR regions of the products synthesized using a human antibody gene as template. Human FRs are ligated *via* the mouse CDR sequences by this reaction.

The full length V region gene, in which three CDRs and four FRs are ultimately ligated, is amplified using primers that anneal to its 5'- or 3'-end, which are added with suitable restriction enzyme recognition sequences. An expression vector for humanized antibody can be produced by inserting the DNA obtained as described above and a DNA that encodes a human antibody C region into an expression vector so that they will ligate in frame. After the recombinant vector is transfected into a host to establish recombinant cells, the recombinant cells are cultured, and the DNA encoding the humanized antibody is expressed to produce the humanized antibody in the cell culture (see, European Patent Publication No. EP 239400 and International Patent Publication No. WO 1996/002576).

By qualitatively or quantitatively measuring and evaluating the antigen-binding activity of the humanized antibody produced as described above, one can suitably select human antibody FRs that allow CDRs to form a favorable antigen-binding site when ligated through the CDRs. Amino acid residues in FRs may be substituted as necessary, so that the CDRs of a reshaped human antibody form an appropriate antigen-binding site. For example, amino acid sequence mutations can be introduced into FRs by applying the PCR method used for grafting a mouse CDR into a human FR. More specifically, partial nucleotide sequence mutations can be introduced into primers that anneal to the FR. Nucleotide sequence mutations are introduced into the FRs synthesized by using such primers. Mutant FR sequences having the desired characteristics can be selected by measuring and evaluating the activity of the amino acid-substituted mutant antibody to bind to the antigen by the above-mentioned method (Sato, K. et al., Cancer Res. (1993) 53: 851-856).

Alternatively, desired human antibodies can be obtained by immunizing transgenic animals having the entire repertoire of human antibody genes (see WO 1993/012227; WO 1992/003918; WO 1994/002602; WO 1994/025585; WO 1996/034096; WO 1996/033735) by DNA immunization.

Furthermore, techniques for preparing human antibodies by panning using human antibody libraries are also known. For example, the V region of a human antibody is expressed as a single-chain antibody (scFv) on phage surface by the phage display method. Phages expressing a scFv that binds to the antigen can be selected. The DNA sequence encoding the human antibody V region that binds to the antigen can be determined by analyzing the genes of selected phages. The DNA sequence of the scFv that binds to the antigen is determined. An expression vector is prepared by fusing the V region sequence in frame with the C region sequence of a desired human antibody, and inserting this into an appropriate expression vector. The expression vector is introduced into cells appropriate for expression such as those described above. The human antibody can be produced by expressing the human antibody-encoding gene in the cells. These methods are already known (see WO 1992/001047; WO 1992/020791; WO 1993/006213; WO 1993/011236; WO 1993/019172; WO 1995/001438; WO 1995/015388).

In addition to the phage display method, techniques that use a cell-free translation system, techniques for displaying antigen-binding molecules on the surface of viruses or cells, and techniques that use emulsions are also known as techniques for obtaining human antibodies by panning using human antibody libraries. For example, the ribosome display method where a complex is formed between the translated protein and mRNA *via* the ribosome by removing the stop codon and such, the cDNA display method or the mRNA display method where a genetic sequence and the translated protein are covalently linked using a compound such as puromycin, the CIS display method where a complex is formed between the gene and the translated protein using a nucleic acid-binding protein, or such may be used as techniques of using a cell-free translation system. For the technique of presenting antigen-binding molecules on the surface of cells or viruses, besides the phage display method, the *E. coli* display method, Gram-positive bacteria display method, yeast display method, mammalian cell display method, virus display method, and such may be used. As a technique that uses emulsions, the *in vitro* virus display method which involves incorporating genes and translation-related molecules into an emulsion, and such may be used. These methods are already publicly known (Nat Biotechnol. 2000 Dec;18(12):1287-92; Nucleic Acids Res. 2006;34(19): e127; Proc Natl Acad Sci U S A. 2004 Mar 2;101(9):2806-10; Proc Natl Acad Sci U S A. 2004 Jun 22;101(25):9193-8; Protein Eng Des Sel. 2008 Apr;21(4):247-55; Proc Natl Acad Sci U S A. 2000 Sep 26;97(20):10701-5; MAbs. 2010 Sep-Oct;2(5):508-18; and Methods Mol Biol. 2012, 911:183-98).

## C. Assays

Anti-target protein antibodies provided herein may be identified, screened for, or characterized for their physical/chemical properties and/or biological activities by various assays known in the art.

### *1. Binding assays and other assays*

In one aspect, an antibody of the present disclosure is tested for its antigen binding activity, e.g., by known methods such as ELISA, Western blot, etc.

### *2. Activity assays*

In one aspect, assays are provided for identifying antibodies of the present disclosure thereof having biological activity. Biological activity may include, e.g., cytotoxic activity (ADCC activity, CDC activity, and such) and internalization activity. Antibodies having such biological activity in vivo and/or in vitro are also provided.

## D. Immunoconjugates

The present disclosure also provides immunoconjugates comprising an antibody of the present disclosure conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, saponaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include $^{211}At$, $^{131}I$, $^{125}I$, $^{90}Y$, $^{186}Re$, $^{188}Re$, $^{153}Sm$, $^{212}Bi$, $^{32}P$, $^{212}Pb$ and radioactive isotopes of Lu. When the radioconjugate is used for detection, it may comprise a radioactive atom for scintigraphic studies, for example Tc-99m or $^{123}I$, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionuclide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

## E. Methods and Compositions for Diagnostics and Detection

In certain embodiments, any of the anti-target protein antibodies provided herein is useful for detecting the presence of a target protein in a biological sample. The term "detecting" as used herein encompasses quantitative or qualitative detection. In certain embodiments, a biological sample comprises a cell or tissue, such as a cell or tissue of heart, liver, lung, kidney, spleen, large intestine, or bone marrow.

In one embodiment, an antibody of the present disclosure for use in a method of diagnosis or detection is provided. In a further aspect, a method of detecting the presence of a target protein in a biological sample is provided. In certain embodiments, the method comprises contacting the biological sample with an antibody as described herein under conditions permissive for binding of the antibody as described herein to the target protein, and detecting whether a complex is formed between the antibody and the target protein. Such method may be an *in vitro* or *in vivo* method. In one embodiment, an antibody of the present disclosure is used to select subjects eligible for therapy with an antibody of the present disclosure, e.g. where the target protein is a biomarker for selection of patients.

Exemplary disorders that may be diagnosed using an antibody of the present disclosure include cancers such as lung cancer (e.g. lung adenocarcinoma) and colorectal cancer.

In certain embodiments, labeled anti-target protein antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}P$, $^{14}C$, $^{125}I$, $^{3}H$, and $^{131}I$, fluorophores such as rare earth chelates or fluorescein and its derivatives, rhodamine and its derivatives, dansyl, umbelliferone, luciferases, e.g., firefly luciferase and bacterial luciferase (U.S. Patent No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, those coupled with an enzyme that employs hydrogen peroxide to oxidize a dye precursor such as HRP, lactoperoxidase, or microperoxidase, biotin/avidin, spin labels, bacteriophage labels, stable free radicals, and the like.

## F. Pharmaceutical Formulations

In one aspect, the present disclosure provides pharmaceutical formulations of an anti-target protein antibody as described herein. In certain embodiments, a pharmaceutical formulation of the present disclosure is an agent for treating, preventing, or diagnosing cancer. Such formulations are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX (registered trademark), Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated or prevented, preferably those with complementary activities that do not adversely affect each other. For example, it may be desirable to further provide a cytotoxic agent. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

## G. Therapeutic Methods and Compositions

Any of the antibodies provided herein may be used in therapeutic methods.

In one aspect, an antibody of the present disclosure for use as a medicament is provided. In further aspects, an antibody of the present disclosure for use in treating cancer is provided. In certain embodiments, an antibody of the present disclosure for use in a method of treatment is provided. In certain embodiments, the present disclosure provides an antibody of the present disclosure for use in a method of treating an individual having cancer (e.g. lung cancer or colorectal cancer) comprising administering to the individual an effective amount of the antibody of the present disclosure. In one such embodiment, the method further comprises administering to the individual an effective amount of at least one additional therapeutic agent, e.g., as described below. In further embodiments, the present disclosure provides an antibody of the present disclosure for use in inducing cytotoxic activity. In certain embodiments, the present disclosure provides an antibody of the present disclosure for use in a method of inducing cytotoxic activity in an individual comprising administering to the individual an effective of the antibody of the present disclosure to induce cytotoxic activity. An "individual" according to any of the above embodiments is preferably a human.

In a further aspect, the present disclosure provides the use of an antibody of the present disclosure in the manufacture or preparation of a medicament. In one embodiment, the medicament is for treatment of cancer (e.g. lung cancer or colorectal cancer). In a further embodiment, the medicament is for use in a method of treating cancer (e.g. lung cancer or colorectal cancer) comprising administering to an individual having cancer (e.g. lung cancer or colorectal cancer) an effective amount of the medicament. In one such embodiment, the method further comprises administering to the individual an effective amount of at least one additional therapeutic agent, e.g., as described below. In a further embodiment, the medicament is for inducing cytotoxic activity. In a further embodiment, the medicament is for use in a method of inducing cytotoxic activity in an individual comprising administering to the individual an amount effective of the medicament to induce cytotoxic activity. An "individual" according to any of the above embodiments may be a human.

In one embodiment of the above-mentioned aspect, in one embodiment of the aspect, the present disclosure provides an antibody or a pharmaceutical of the present disclosure for use in treatment of lung cancer, wherein the antibody or pharmaceutical is for administration to a lung cancer patient who has been selected by the following: assessing the presence or absence of one or more selected from an EGFR driver mutation, a BRAF driver mutation, an ERBB2 driver mutation, ALK fusion gene expression, and RET/ROS1 fusion gene expression in a biological sample obtained from a lung cancer patient, and selecting a lung cancer patient who does not have one or more selected from an EGFR driver mutation, a BRAF driver mutation, an ERBB2 driver mutation, ALK fusion gene expression, and RET/ROS1 fusion gene expression, as a responder to treatment with the antibody or pharmaceutical of the present disclosure. In another embodiment, the present disclosure provides an antibody or a pharmaceutical of the present disclosure for use in treatment of colorectal cancer, wherein the antibody or pharmaceutical is for administration to a colorectal cancer patient who has been selected by the following: assessing the presence or absence of a KRAS mutation in a biological sample obtained from a colorectal cancer patient, and selecting a colorectal cancer patient with a KRAS mutation as a responder to treatment with the antibody or pharmaceutical of the present disclosure. Methods for assessing the presence or absence of EGFR driver mutations, BRAF driver mutations, ERBB2 driver mutations, ALK fusion gene expression, RET/ROS 1 fusion gene expression, and KRAS mutations are known in the art, and examples include the methods described in the Examples herein.

In a further aspect, the present disclosure provides a method for treating cancer (e.g. lung cancer and colorectal cancer) (synonymous with a method for treating an individual having cancer). In one embodiment, the method comprises administering to an individual having such cancer (e.g. lung cancer or colorectal cancer) an effective amount of an antibody of the present disclosure. In one such embodiment, the method further comprises administering to the individual an effective amount of at least one additional therapeutic agent, as described below. An "individual" according to any of the above embodiments may be a human.

In an embodiment of the above-mentioned aspect, the above method comprises assessing the presence or absence of one or more selected from an EGFR driver mutation, a BRAF driver mutation, an ERBB2 driver mutation, ALK fusion gene expression, and RET/ROS1 fusion gene expression in a biological sample obtained from an individual who has lung cancer; selecting an individual who does not have one or more selected from an EGFR driver mutation, a BRAF driver mutation, an ERBB2 driver mutation, ALK fusion gene expression, and RET/ROS 1 fusion gene expression as a responder to treatment with the antibody of the present disclosure; and administering the antibody of the present disclosure to the selected individual. In another embodiment, the above method comprises assessing the presence or absence of a KRAS mutation in a biological sample obtained from an individual who has colorectal cancer; selecting an individual with a KRAS mutation as a responder to treatment with the antibody of the present disclosure; and administering the antibody of the present disclosure to the selected individual.

In a further aspect, the present disclosure provides a method for inducing cytotoxic activity in an individual. In one embodiment, the method comprises administering to the individual an effective amount of an antibody of the present disclosure to include cytotoxic activity. In one embodiment, an "individual" is a human.

In a further aspect, the present disclosure provides pharmaceutical formulations comprising any of the antibodies provided herein, e.g., for use in any of the above therapeutic methods. In one embodiment, a pharmaceutical formulation comprises any of the antibodies provided herein and a pharmaceutically acceptable carrier. In another embodiment, a pharmaceutical formulation comprises any of the antibodies provided herein and at least one additional therapeutic agent, e.g., as described below.

Antibodies of the present disclosure can be used either alone or in combination with other agents in a therapy. For instance, an antibody of the present disclosure may be co-administered with at least one additional therapeutic agent. In certain embodiments, an additional therapeutic agent is a cytotoxic agent.

Such combination therapies noted above encompass combined administration (where two or more therapeutic agents are included in the same or separate formulations), and separate administration, in which case, administration of the antibody of the present disclosure can occur prior to, simultaneously, and/or following, administration of the additional therapeutic agent or agents. In one embodiment, administration of the antibody of the present disclosure and administration of an additional therapeutic agent occur within about one month, or within about one, two or three weeks, or within about one, two, three, four, five, or six days, of each other. Antibodies of the present disclosure can also be used in combination with radiation therapy.

An antibody of the present disclosure (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the present disclosure would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the present disclosure (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 micro g/kg to 15 mg/kg (e.g. 0.1mg/kg-10mg/kg) of antibody can be an initial candidate dosage for administration to the patient, whether, for example, by one or more separate administrations, or by continuous infusion. One typical daily dosage might range from about 1 micro g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the present disclosure in place of or in addition to an antibody of the present disclosure.

### H. Articles of Manufacture

In another aspect of the present disclosure, an article of manufacture containing materials useful for the treatment, prevention and/or diagnosis of the disorders described above is provided. The article of manufacture comprises a container and a label on or a package insert associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active ingredient in the composition is an antibody of the present disclosure. The label or package insert indicates that the composition is used for treating the condition of choice. Moreover, the article of manufacture may comprise (a) a first container with a composition contained therein, wherein the composition comprises an antibody of the present disclosure; and (b) a second container with a composition contained therein, wherein the composition comprises a further cytotoxic or otherwise therapeutic agent. The article of manufacture in this embodiment of the present disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the present disclosure in place of or in addition to an antibody of the present disclosure.

[Examples]

(Example 1) Human tissues and gene analyses thereof

(1-1) Obtaining colorectal cancer tissues, lung cancer tissues, adjacent normal tissues, and normal tissues

Human tissues to be analyzed were purchased from Asterand, ILS Bio, and All Cells. For colorectal cancer tissues and normal tissues adjacent to colorectal cancer tissue, 12 pairs of a colorectal cancer tissue and a normal tissue adjacent to the colorectal cancer tissue derived from the same donors (stage III or IV) (from 12 donors, a total of 24 specimens) were obtained. Additionally, two specimens of cancer tissue that had metastasized to the lymph node were obtained from each of two of the 12 donors. In total, 28 specimens were obtained.

For lung cancer tissues and normal tissues adjacent to lung cancer tissue, 14 specimens of lung adenocarcinoma tissues and 10 specimens of normal tissues adjacent to lung adenocarcinoma were obtained. Of these two sets of a cancer tissue and an adjacent normal tissue were derived from the same donors, and the others were not.

For normal tissues, three specimens of normal or adjacent normal tissues each from the heart, liver, lung, kidney, spleen, colon, and bone marrow were obtained.

(1-2) Analysis of ALK, RET fusion gene in human lung cancer tissues (RNA-seq analysis)

RNA-seq analysis (Takara Bio) was performed to examine whether the lung cancer tissues obtained in Example 1-1 had a driver fusion gene (ALK, RET/ROS1). Total RNA was extracted from two to three tissue pieces of approximately 3 mm in size using NucleoSpinTissue (Macherey-Nagel) by the method recommended by the manufacturer. A DNA library was prepared from the total RNA sample using TruSeq RNA Sample Prep Kit v2 (Illumina) by the method recommended by the manufacturer. The prepared DNA libraries were mixed, and single-lane, 100-base paired-end sequencing was performed using Illumina sequencer HiSeq, and nucleotide sequences (read sequences) were obtained by the software supplied with the sequencer. Fusion gene analyses were performed using STAR-Fusion (https://github.com/STAR-Fusion/STAR-Fusion).

The results revealed that among the 14 lung cancer tissue samples obtained, none had a RET/ROS1 fusion gene, and one had an ALK fusion gene (485475RF) (Table 2).

(1-3) Driver mutation analysis of human colorectal cancer tissue and lung cancer tissue (Exome-seq analysis)

Exome-seq analysis (Takara Bio) was performed to examine whether the cancer tissues obtained in Example 1-1 had driver mutations (KRAS, EGFR, BRAF, ERBB2). Genomic DNA was extracted from two to three tissue pieces of approximately 3 mm in size using NucleoSpinTissue (Macherey-Nagel) by the method recommended by the manufacturer. A DNA library was prepared from the genomic DNA sample using SureSelect XT Human All Exon V6 (Agilent) by the method recommended by the manufacturer. The prepared DNA libraries were mixed, and 100-base paired-end analysis was performed using Illumina sequencer HiSeq, and nucleotide sequences (read sequences) were obtained by the software supplied with the sequencer. Mutation analysis was performed using CLC Genomics Server (QIAGEN).

The results showed that, for the colorectal cancer tissues, 7 out of the 12 donor-derived samples (1170614F, 1171659F, 39694FT, 39697FT, 39698FT, 39705FT, and 40644FT) were found to have a KRAS driver mutation. For the lung cancer tissues, three samples (1191924F, 1160304F, and 1245371F) were found to have an EGFR driver mutation, two samples (1186142F and 1209913F) a BRAF driver mutation, and three samples (1177695F, 1185682F, and 463102XF) a KRAS driver mutation (Table 2).

[Table 2]

| Summary of results of driver-mutation and fusion-gene analyses on colorectal cancer and lung cancer tissues. | | | | | | |
|---|---|---|---|---|---|---|
| ID | Tissue | KRAS | EGFR | BRAF | ERBB2 | Fusion gene |
| 1119061F | colorectal cancer | | | | | |
| 1136277F | colorectal cancer | | | | | |

(continued)

| Summary of results of driver-mutation and fusion-gene analyses on colorectal cancer and lung cancer tissues. | | | | | | |
|---|---|---|---|---|---|---|
| ID | Tissue | KRAS | EGFR | BRAF | ERBB2 | Fusion gene |
| 1145808F | colorectal cancer | | | | | |
| 1170614F | colorectal cancer | Gly12Asp | | | | |
| 1171659F | colorectal cancer | Gly12Val | | | | |
| ILS39694FT1 | colorectal cancer | Gly12Cys | | | | |
| ILS39697FT1 | colorectal cancer | Gln61Arg | | | | |
| ILS39698FT1 | colorectal cancer | Gly13Asp | | | | |
| ILS39705FT1 | colorectal cancer | Gly12Ser | | | | |
| ILS40183FT4 | colorectal | | | | | |
| | cancer | | | | | |
| ILS40636FT4 | colorectal cancer | | | | | |
| ILS40636FM1 | colorectal cancer, metastasis to lymph node | | | | | |
| ILS40636FM2 | colorectal cancer, metastasis to lymph node | | | | | |
| ILS40644FT1 | colorectal cancer | Gly12Val | | | | |
| ILS40644FM1 | colorectal cancer, metastasis to lymph node | Gly12Val | | | | |
| ILS40644FM2 | colorectal cancer, metastasis to lymph node | Gly12Val | | | | |
| 463118ZF | lung cancer | | | | | |
| 1081042F | lung cancer | | | | | |
| 1177695F | lung cancer | Gly12Cys | | | | |
| 1184871F | lung cancer | | | | | |
| 1185682F | lung cancer | Gly12Cys | | | | |
| 1204668F | lung cancer | | | | | |
| 1245371F | lung cancer | | Gly719Ala | | | |
| 463102XF | lung cancer | Gly12Cys | | | | |
| ILS31537 D3 | lung cancer | | | | | |
| 485475RF | lung cancer | | | | | ALK |
| 1186142F | lung cancer | | | Gly466Ala | | |
| 1191924F | lung cancer | | Glu746_Ala750del | | | |
| 1160304F | lung cancer | | Leu858Arg | | | |
| 1209913F | lung cancer | | | Val600Glu | | |

The detected driver mutations are summarized in Table 2. ILS40636FM1, ILS40636FM2, ILS40644FM1, and ILS40644FM2 are results from cancer tissues having metastasized to the lymph node, and the others are results from colorectal cancer and lung cancer tissues.

(Example 2) Protein expression analysis of human tissues (Proteomics analysis)

The tissue block obtained in Example 1-1 in a frozen state was placed in a mortar containing liquid nitrogen, and broken into tissue pieces of about 3 mm in size by hitting with a pestle. Two to three tissue pieces were transferred to a tube, and finely ground using a multi-bead shocker (Yasui Kikai). A tissue lysis buffer (2% deoxycholic acid/ 100 mM ammonium bicarbonate, Protease inhibitor cocktail (Roche), and PhosSTOP (Roche)) was added to the ground tissue, and the tissue was ultrasonically disrupted using an acoustic solubilizer (Covaris) or Bioruptor (Sonicbio), and centrifuged at 15,000 rpm for 30 minutes to remove insoluble materials. Then, the supernatant was collected as a tissue extract. For the bone marrow, the tissue was washed with PBS, and the tissue lysis buffer was added to precipitated cells. Then, a tissue extract was obtained in the same manner as the other tissues.

Proteins in the tissue extract were reduced with dithiothreitol, alkylated with iodoacetamide, and then precipitated by TCA/acetone precipitation. The proteins were dissolved in 8 M urea/400 mM ammonium bicarbonate solution, and the protein solution was diluted four times with distilled water. The diluted protein solution was subjected to protein digestion with lysyl endopeptidase and trypsin using Rapid Enzyme Digestion System (REDS) (AMR) to obtain a peptide solution. The peptide solution was desalted using Monospin C18 (GL Science) by the method recommended by the manufacturer. Thereafter, the desalted peptide solution was fractionated using Pierce™ High pH Reversed-Phase Peptide Fractionation Kit (ThermoFisher Scientific) or AssayMAP Bravo cartridge RP-S (Agilent) by the method recommended by the manufacturer, and each fraction was analyzed by an LC-MS analysis system in which Q-Exactive (ThermoFisher Scientific) was coupled to a nano-LC system (Ultimate3000, Dionex).

Raw data after the LC-MS analyses were used for database search using MaxQuant (http://www.biochem.mpg.de/5111795/maxquant) to identify proteins and obtain quantification values (iBAQ). The database search was performed with the following parameters:
Taxonomy: uniprot human, Fixed modification: carbamidomethylation (C), and Variable modification: oxidation (M); deamidation (NQ), Acetyl (protein N-term), and FDR (protein, peptide) <1%.

The quantification values were calculated by performing the following processing on the MaxQuant output file "proteinGroups.txt":

1. Sum up the signal intensities of protein groups assigned to the same gene to calculate the intensity at the gene level.
2. For each gene, normalize the intensity with the number of fragments that can be generated by trypsinization to calculate the iBAQ score.
3. For each sample, perform normalization such that the score average is 1,000,000.

A total of more than 10,000 proteins were identified, and an iBAQ value was obtained for each protein. Cell surface proteins that showed high protein expression in cancer and low expression in adjacent normal tissues and normal tissues — 7 proteins from colorectal cancer (SPINT2, MARVELD3, MANSC1, NOX1, SLC12A2, CDCP1, and CEACAM5) and 10 proteins from lung cancer (SPINT2, PVRL4, SEZ6L2, XPR1, FLVCR1, SLC7A5, STEAP1, MMP14, TNFRSF21, and TMPRSS4) — were selected as target antigen candidates.

The expression of the selected candidate proteins is shown in Figures 1-1 to 1-16. In analyzing the colorectal cancer samples, CEACAM5 (SEQ ID NO: 10), which is an antigen currently undergoing clinical trials, serves as an index for comparing antigen expression profiles. CEACAM5 showed certain levels of expression in the normal tissues and the adjacent normal tissues, but even higher expression in almost all cases of cancer. NOX1, MARVELD3, and MANSC1 showed a different type of expression profile from that of CEACAM5, as their protein expression was not observed in normal tissues but was high (above the median value) in three to four cases of colorectal cancer tissue with a KRAS mutation, which showed large differences from the expression levels in the normal tissues adjacent to colorectal cancer tissue. SPINT2, SLC12A2, and CDCP1 showed a similar expression profile to that of CEACAM5, as they showed certain levels of expression in the normal tissues and the normal tissues adjacent to colorectal cancer tissue but even higher expression in the colorectal cancer tissues. In analyzing the lung cancer samples, PVRL4 (SEQ ID NO: 11), which is under ongoing clinical trials, serves as an index for comparing antigen expression profiles. PVRL4 and XPR1 were very highly cancer-specific antigens, with no protein expression observed in the normal tissues but high expression in the lung cancer tissues. TMPRSS4 and TNFRSF21 were not expressed in the normal tissues, but showed moderate expression levels in the lung cancer tissues. SLC7A5, STEAP1, FLVCR1, SEZ6L2, MMP14 and SPINT2 showed certain levels of expression in the normal tissues and the normal tissues adjacent to lung cancer tissue, but even higher expression in the lung cancer tissues.

(Example 3) Isoform expression analysis of target antigen candidate MARVELD3 from colorectal cancer

MARVELD3 is known to have isoforms 1 and 2, which have very similar intracellular region amino acid sequences but largely different extracellular regions (Fig. 2).

To determine which isoform is highly expressed in cancer tissue, the LC-MS peak intensities of the MARVELD3 peptide fragments identified in the proteomics analysis were examined. For the list of identified peptides and quantification values, one of the MaxQuant output files analyzed in Example 1-2, ModificationSpecificPeptides.txt, was used. In addition to the peptide fragments common to isoforms 1 and 2, the isoform 2-specific fragment was detected in four out of the seven colorectal cancer tissue specimens with a KRAS mutation (in two or more of three LC-MS runs), but in none of the adjacent normal tissues in two or more of three runs (Fig. 3-1). The isoform 1-specific fragment was detected in two out of the seven colorectal cancer tissue specimens with a KRAS mutation (Fig. 3-2). Therefore, it was presumed that isoform 2 is highly expressed in more colorectal cancer tissues with a KRAS mutation.

(Example 4) Cell surface localization analysis of target antigen candidates

To examine the cell surface localization of XPR-1, MARVELD3 (isoform2), andNOX1, cell surface proteomics analysis was performed using Expi293 cells (ThermoFisher Scientific) made to overexpress myc-tag-added XPR-1 and MARVELD3 (isoform2) using ExpiFectamine (ThermoFisher Scientific) by the method recommended by the manufacturer, and C2BBe1, SW403, SW1463, DMS79 (ATCC), and HLC-1 cells (Riken), which presumably highly express these three proteins. After washing the cells with PBS, the cells were incubated with a cell membrane-impermeable biotin reagent (Sulfo-NHS-LC-biotin, ThermoFisher Scientific) to biotinylate the amino groups of the N-terminus and lysine side chains of cell surface proteins. The collected cells were ultrasonically disrupted using Covaris acoustic solubilizer or Bioruptor (Sonicbio) to obtain a cell extract.

Proteins in the cell extract were precipitated by the methanol/chloroform method and dissolved in an 8 M urea/400 mM ammonium bicarbonate solution. After reduction with dithiothreitol and alkylation with iodoacetamide, the proteins were digested with trypsin to obtain a peptide solution. The peptide solution was mixed with Neutravidin FG beads (Tamagawa Seiki), washed, and then eluted to purify biotinylated peptides.

The purified biotinylated peptides were analyzed using an LC-MS analysis system in which Q-Exactive (ThermoFisher Scientific) or Orbitrap fusion Lumos (ThermoFisher Scientific) was coupled to a nano-LC system (Ultimate 3000, Dionex).

Raw data after the LC-MS analyses were used for database search using MaxQuant (http://www.biochem.mpg.de/5111795/maxquant) to identify biotinylated peptides and proteins and obtain quantification values. The database search was performed with the following parameters: Taxonomy: uniprot human, Fixed modification: carbamidomethylation (C), Variable modification: oxidation (M), Acetyl (Protein N-term), NHS-LC-biotin (N-term); NHS-LC-biotin (K), FDR (protein, peptide) <1%. For the list of identified peptides and quantification values, one of the MaxQuant output files, ModificationSpecificPeptides.txt, was used. The sequences and expression levels of the biotinylated peptides detected by this method enable determination of whether or not the protein to be analyzed is expressed on the cell membrane, and also enable identification of its extracellular region.

From cells overexpressing XPR1, biotinylated peptides of amino acid numbers 1-108, 111-122, 159-171, 177-216, 423-448, 473-502, 660-670, and 674-696 were detected (Fig. 4). In addition, biotinylated peptides of amino acid numbers 177-190, 428-448, and 660-670 were detected from the endogenous protein of HLC-1 or DMS-79 cells (Fig. 4-1). This confirmed that XPR1 is localized on the cell surface, and suggested that regions considered to be intracellular regions according to uniprot may be extracellular regions.

NOX1 was confirmed to be localized on the cell surface because biotinylated peptides of amino acid numbers 44-54, 131-161, and 242-258 were detected from C2BBe1, SW1463, or SW403 cells (Fig. 4-2).

For MARVELD3 isoform 2, biotinylated peptides of amino acid numbers 101-111 and 163-198 were detected from the overexpressing cells. However, no biotinylated peptide of the endogenous protein was detected from the analyzed cells (Fig. 4-3). The small number of lysin to be biotinylated in the putative extracellular region of MARVELD3 isoform 2 might make the detection of biotinylated peptides difficult.

(Example 5) Protein expression analysis of cell lines (proteomics analysis)

HLC-1 cells (Riken), NCI-H2227 cells (ATCC), and Caco-2 cells (Riken) were suspended in a cell lysis buffer (1% NP-40, 50 mM Tris-Cl (pH7.5), 150 mM NaCl, Protease inhibitor cocktail (Roche)), and ultrasonically disrupted with an acoustic solubilizer (Covaris) to obtain cell extracts.

Proteins in the cell extracts were reduced with dithiothreitol, alkylated with iodoacetamide, and then precipitated by methanol/chloroform precipitation. The precipitated proteins were dissolved in 8 M urea/400 mM ammonium bicarbonate solution, diluted 4 times with distilled water, and then digested with lysyl endopeptidase and trypsin to obtain peptide solutions. The peptide solutions were desalted using Monospin C18 (GL Science) by the method recommended by the manufacturer. Thereafter, the desalted peptide solutions were fractionated using AssayMAP Bravo cartridge RP-S (Agilent) by the method recommended by the manufacturer, and each fraction was analyzed by an LC-MS analysis system in which Q-Exactive (ThermoFisher Scientific) was coupled to a nano-LC system (ThermoFisher Scientific).

Raw data after the LC-MS analyses were used for database search using MaxQuant (http://www.biochem.mpg.de/5111795/maxquant) to identify proteins and obtain quantification values (iBAQ, Intensity Based Absolute Quantification). The database search was performed with the following parameters:

Taxonomy: uniprot human, Fixed modification: carbamidomethylation (C), Variable modification: oxidation (M); deamidation (NQ), Acetyl (protein N-term), and FDR (protein, peptide) <1%.

The iBAQ values for XPR1 and MARVELD3 set forth in the MaxQuant output file "proteinGroups.txt" are shown in Fig. 5-1 and Fig. 5-2.

(Example 6) Preparation of anti-XPR1 antibody

A plasmid vector expressing full-length human XPR1 (SEQ ID NO: 1) with a TT peptide sequence (FNNFTVSFWLRVPKVSASHLE, SEQ ID NO: 22) added to the C-terminus (human XPR1-TT, nucleotide SEQ ID NO: 23, amino acid SEQ ID NO: 24) was prepared. Four New Zealand White rabbits (Kitayama Labes) were immunized with the prepared plasmid vector six times by a gene gun method and an *in vivo* electroporation method. Peripheral blood mononuclear cells and spleen cells were harvested from the rabbits one week after the final immunization. Surface IgG-positive B cells were concentrated from the harvested rabbit-derived cells by MACS using PE-labeled anti-rabbit IgG antibody (Southern biotech) and cultured. B cells secreting an antibody binding to human XPR1 were identified from the cultured B cells using a fluorescence microscope attached to the micromanipulator CellCelector (ALS) and collected in a microwell plate using the micromanipulator CellCelector (ALS). Genes constituting the variable regions of rabbit immunoglobulin were cloned from the collected B cells by RT-PCR. These rabbit immunoglobulin variable region DNA fragments were inserted into an expression vector containing the IgG constant regions to prepare a recombinant antibody. The anti-human XPR1 antibody was prepared by a method known to those skilled in the art. The prepared anti-human XPR1 antibody is shown in Table 3.

[Table 3]

| Name of antibody | XPB0062 |
|---|---|
| HVR_H1 | SEQ ID NO: 35 |
| HVR_H2 | SEQ ID NO: 36 |
| HVR_H3 | SEQ ID NO: 37 |
| HVR_L1 | SEQ ID NO: 38 |
| HVR_L2 | SEQ ID NO: 39 |
| HVR_L3 | SEQ ID NO: 40 |
| VH | SEQ ID NO: 41 |
| VL | SEQ ID NO: 42 |

As an anti-human CD3 antibody, CE115TR (heavy chain variable region SEQ ID NO: 65, light chain variable region SEQ ID NO: 66), which binds to the CD3ε chain constituting the T cell receptor and induces an activation signal of the T cell receptor, was prepared by a method known to those skilled in the art. The present inventors prepared a bispecific antibody consisting of an anti-human XPR1 antibody and an anti-CD3 antibody by a method known to those skilled in the art. The prepared anti-human XPR1/anti-human CD3 bispecific antibody is shown in Table 4. The heavy chain constant region sequence on the anti-human XPR1 side of the bispecific antibody is the sequence shown in SEQ ID NO: 83, the light chain constant region sequence on the anti-human XPR1 side is the sequence shown in SEQ ID NO: 85, the heavy chain constant region sequence on the anti-human CD3 side is the sequence shown in SEQ ID NO: 84, and the light chain constant region sequence on the anti-human CD3 side is the sequence shown in SEQ ID NO: 86.

[Table 4]

| Name of Antibody | | TR01H113//XPB0062HCa |
|---|---|---|
| Anti-human XPR1 | HVR_H1 | SEQ ID NO: 35 |
| | HVR_H2 | SEQ ID NO: 36 |
| | HVR_H3 | SEQ ID NO: 37 |
| | HVR_L1 | SEQ ID NO: 38 |
| | HVR_L2 | SEQ ID NO: 39 |
| | HVR_L3 | SEQ ID NO: 40 |
| | VH | SEQ ID NO: 41 |
| | VL | SEQ ID NO: 42 |
| Anti-human CD3 | HVR_H1 | SEQ ID NO: 59 |
| | HVR_H2 | SEQ ID NO: 60 |
| | HVR_H3 | SEQ ID NO: 61 |
| | HVR_L1 | SEQ ID NO: 62 |
| | HVR_L2 | SEQ ID NO: 63 |
| | HVR_L3 | SEQ ID NO: 64 |
| | VH | SEQ ID NO: 65 |
| | VL | SEQ ID NO: 66 |

(Example 7) Confirmation of binding of anti-human XPR1 antibody to human XPR1

A plasmid vector expressing in mammalian cells full-length human XPR1 (amino acid SEQ ID NO: 1) with a Myc tag sequence (EQKLISEEDL, SEQ ID NO: 25) added to the C-terminus (human XPR1-Myc, nucleotide SEQ ID NO: 26, amino acid SEQ ID NO: 27) was constructed. The protein was transiently expressed in Expi293 cells (Thermo Fisher Scientific) by introducing the constructed plasmid vector into Expi293 cells. At the same time, Expi293 cells transfected with an empty plasmid vector were also prepared as a control. The Expi293 cells transfected with an empty plasmid vector were stained with CellTrace FarRed (Thermo Fisher Scientific) and then mixed with the Expi293 cells made to express human XPR1-Myc, and subsequently reacted with the anti-human XPR1 antibody prepared in Example 6. Then, anti-human IgG Fc cross-adsorbed Alexa488 (Invitrogen) was reacted to stain the anti-human XPR1 antibody. Dead cells were stained with DAPI (sigma-aldrich). The cells reacted with the antibody were measured by FACS Aria III (Becton, Dickinson and Company). The obtained data were analyzed by FlowJo ver.10. The live cell fraction, which was not stained with DAPI fluorescence, was separated into a fraction that was stained with CellTrace FarRed (cells transfected with the empty plasmid vector) and a fraction that was not stained with CellTrace FarRed (cells transfected with the human XPR1-Myc expression plasmid vector), and Alexa488 staining for each fraction was examined. As a result, more intense Alexa488 staining was observed in the fraction that was not stained with CellTrace FarRed (cells transfected with the human XPR1-Myc expression plasmid vector) than in the fraction that was stained with CellTrace FarRed (cells transfected with an empty plasmid vector) (Fig. 6). This result confirmed that the anti-human XPR1 antibody prepared in Example 6 binds to human XPR1 expressed on cells.

(Example 8) Cytotoxic activity assay of anti-human XPR1/anti-human CD3 bispecific antibody LDH cytotoxicity assay using human peripheral blood mononuclear cells (PBMC)

The cytotoxic activity of the anti-human XPR1/anti-human CD3 bispecific antibody prepared in Example 6 was evaluated by the LDH release method (LDH Cytotoxicity Detection Kit: TAKARA). First, the antibody solutions at various concentrations (0.04, 0.004, and 0.0004 mg/mL) diluted with the culture solution (RPMI1640 solution supplemented with 10% FBS) to make 4 times of the final concentration were added to a 96-well U-bottom plate at 50 $\mu$L per well. Next, as target cells, HLC-1 was adjusted to $1 \times 10^5$ cells/mL, NCI-H2227 was adjusted to $2 \times 10^5$ cells/mL. The cells were seeded at 100 $\mu$L/well or 50 $\mu$L/well, respectively, so that the final number of cells would be $1 \times 10^4$ cells/well, and the U-bottom plate was allowed to stand at room temperature for 15 minutes. As effector cells, human PBMCs (peripheral blood mononuclear cells) were adjusted to $4 \times 10^6$ cells/mL or $2 \times 10^6$ cells/mL. The cells of $4 \times 10^6$ cells/mL were

added to the wells seeded with HLC-1 cells, and the cells of $2 \times 10^6$ cells/mL to the wells seeded with NCI-H2227 cells, at 50 μL per well. Subsequently, the U-bottom plate was allowed to stand at 37°C for approximately 24 hours in a 5% carbon dioxide gas incubator, and then centrifuged. In the U-bottom plate, in addition to the wells to which the above target cells, the effector cells, and the antibody were added, wells to which only the culture solution was added, wells to which only the target cells and the effector cells were added (no antibody added), and wells to which the target cells, the effector cells, and Triton X-100 were added (no antibody added), were also prepared.

One hundred μL of culture supernatant in each well of the U bottom plate was transferred to a 96-well flat bottom plate. The catalyst solution was dissolved in 1 mL of $H_2O$ and mixed with the dye solution to a catalyst solution to dye solution ratio of 1:45. The mixture of the catalyst solution and the dye solution was dispensed into the 96-well flat bottom plate to which the culture supernatant had been transferred, and the flat bottom plate was allowed to stand at room temperature for 15 to 30 minutes.

The absorbance at 492 nm was measured for each well, and the absorbance at the control wavelength of 620 nm measured at the same time was used to calculate 492-620 nm absorbance. The 492-620 nm absorbance of each well was applied to the following formula to calculate the cell growth inhibition rate (Cytotoxicity).

$$\text{Cell growth inhibition rate (\%)} = \frac{(A-D)-(B-D)}{(C-D)-(B-D)} \times 100$$

A: 492-620 nm absorbance of wells to which target cells, effector cells, and antibody were added
B: 492-620 nm absorbance of wells to which only target cells and effector cells were added
C: 492-620 nm absorbance of wells to which target cells, effector cells, and Triton X-100 were added
D: mean 492-620 nm absorbance of wells containing only the culture medium (blank)

The anti-human XPR1/anti-human CD3 bispecific antibody showed a dose-dependent cell growth inhibitory activity (Figs. 7 and 8).

(Example 9) Preparation of anti-human MARVELD3 isoform2 antibody

A plasmid vector expressing human MARVELD3 isoform2 (nucleotide SEQ ID NO: 28, amino acid SEQ ID NO: 4) was constructed. Four New Zealand White rabbits (Kitayama Labes) were immunized with the constructed plasmid vector six times by a gene gun method and an in vivo electroporation method, and peripheral blood mononuclear cells and spleen cells were harvested from the rabbits one week after the final immunization. Surface IgG-positive B cells were concentrated from the harvested rabbit-derived cells by MACS using PE-labeled anti-rabbit IgG antibody (Southern biotech), and cultured. B cells secreting antibodies binding to human MARVELD3 isoform2 were identified from the cultured B cells using a fluorescence microscope attached to the micromanipulator CellCelector (ALS) and collected in a microwell plate using the micromanipulator CellCelector (ALS). Genes constituting the variable regions of rabbit immunoglobulin were cloned from the collected B cells by RT-PCR. These rabbit immunoglobulin variable region DNA fragments were inserted into an expression vector containing the IgG constant regions to prepare recombinant antibodies. The anti-human MARVELD3 isoform2 antibodies were prepared by a method known to those skilled in the art. The prepared anti-human MARVELD3 isoform2 antibodies are shown in Table 5.

[Table 5]

| Name of antibody | MDA0279 | MDA0314 |
|---|---|---|
| HVR_H1 | SEQ ID NO: 43 | SEQ ID NO: 51 |
| HVR_H2 | SEQ ID NO: 44 | SEQ ID NO: 52 |
| HVR_H3 | SEQ ID NO: 45 | SEQ ID NO: 53 |
| HVR_L1 | SEQ ID NO: 46 | SEQ ID NO: 54 |
| HVR_L2 | SEQ ID NO: 47 | SEQ ID NO: 55 |
| HVR_L3 | SEQ ID NO: 48 | SEQ ID NO: 56 |
| VH | SEQ ID NO: 49 | SEQ ID NO: 57 |
| VL | SEQ ID NO: 50 | SEQ ID NO: 58 |

As an anti-human CD3 antibody, CE115TR (heavy chain variable region SEQ ID NO: 65, light chain variable region SEQ ID NO: 66), which binds to the CD3ε chain constituting the T cell receptor and induces an activation signal of the T cell receptor, was prepared by a method known to those skilled in the art. The present inventors prepared bispecific antibodies consisting of an anti-human MARVELD3 isoform2 antibody and an anti-CD3 antibody by a method known to those skilled in the art. The prepared anti-human MARVELD3 isoform2/anti-human CD3 bispecific antibodies are shown in Table 6. The heavy chain constant region sequence on the anti-human MARVELD3 isoform2 side of the bispecific antibodies is the sequence shown in SEQ ID NO: 83, the light chain constant region sequence on the anti-human MARVELD3 isoform2 side is the sequence shown in SEQ ID NO: 85, the heavy chain constant region sequence on the anti-human CD3 side is the sequence shown in SEQ ID NO: 84, and the light chain constant region sequence of the anti-human CD3 side is the sequence shown in SEQ ID NO: 86.

[Table 6]

| Name of Antibody | | TR01H113//MDA0279 | TR01H113//MDA0314 |
|---|---|---|---|
| Anti-human MARVELD3 isoform2 | HVR_H1 | SEQ ID NO: 43 | SEQ ID NO: 51 |
| | HVR_H2 | SEQ ID NO: 44 | SEQ ID NO: 52 |
| | HVR_H3 | SEQ ID NO: 45 | SEQ ID NO: 53 |
| | HVR_L1 | SEQ ID NO: 46 | SEQ ID NO: 54 |
| | HVR_L2 | SEQ ID NO: 47 | SEQ ID NO: 55 |
| | HVR_L3 | SEQ ID NO: 48 | SEQ ID NO: 56 |
| | VH | SEQ ID NO: 49 | SEQ ID NO: 57 |
| | VL | SEQ ID NO: 50 | SEQ ID NO: 58 |
| Anti-human CD3 | HVR_H1 | SEQ ID NO: 59 | |
| | HVR_H2 | SEQ ID NO: 60 | |
| | HVR_H3 | SEQ ID NO: 61 | |
| | HVR_L1 | SEQ ID NO: 62 | |
| | HVR_L2 | SEQ ID NO: 63 | |
| | HVR_L3 | SEQ ID NO: 64 | |
| | VH | SEQ ID NO: 65 | |
| | VL | SEQ ID NO: 66 | |

(Example 10) Confirmation of binding of anti-human MARVELD3 isoform2 antibodies to MARVELD3 isoform2

A plasmid vector that expresses, in mammalian cells, a molecule (Myc-human MARVELD3, nucleotide SEQ ID NO: 29, amino acid SEQ ID NO: 30) in which the Myc tag sequence (EQKLISEEDL, SEQ ID NO: 25) has been added after the N-terminal methionine of full-length human MARVELD3 isoform2 (amino acid SEQ ID NO: 4), was introduced into Expi293 cells (Thermo Fisher Scientific), and the protein was transiently expressed in the Expi293 cells. At the same time, Expi293 cells transfected with an empty plasmid vector were also prepared as a control. The Expi293 cells transfected with an empty plasmid vector were stained with CellTrace FarRed (Thermo Fisher Scientific) and then mixed with the Expi293 cells made to express human MARVELD3 isoform2, and subsequently reacted with an anti-human MARVELD3 isoform2 antibody prepared in Example 9. Then, anti-human IgG Fc cross-adsorbed Alexa488 (Invitrogen) was reacted to stain the anti-human MARVELD3 isoform2 antibody. Dead cells were stained with DAPI (sigma-aldrich). The cells reacted with the antibody were measured by FACS Aria III (Becton, Dickinson and Company). The obtained data were analyzed by FlowJo ver.10. The live cell fraction, which was not stained with DAPI fluorescence, was separated into a fraction that was stained with CellTrace FarRed (cells transfected with the empty plasmid vector) and a fraction that was not stained with CellTrace FarRed (cells transfected with the human XPR1-Myc expression plasmid vector), and Alexa488 staining for each fraction was examined. As a result, more intense Alexa488 staining was observed in the fraction that was not stained with CellTrace FarRed (cells transfected with the human MARVELD3 isoform2 expression plasmid vector) than in the fraction that was stained with CellTrace FarRed (cells transfected with the empty plasmid vector) (Fig. 9). This result confirmed that the anti-human MARVELD3 isoform2 antibodies prepared in Example 9 bind to human MARVELD3 isoform2 expressed on cells.

(Example 11) Cytotoxic activity assay of anti-human MARVELD3 isoform2/anti-human CD3 bispecific antibody

Cytotoxic activity assay using human peripheral blood mononuclear cells

The cytotoxic activity of the anti-human MARVELD3 isoform2/anti-human CD3 bispecific antibodies prepared in Example 9 was evaluated based on cell growth inhibition rates determined using xCELLigence Real-Time Cell Analyzer (Roche Diagnostics). The target cells used were Caco-2 cells. First, 50 μL of the medium was added to each well of the E-Plate 96 (Roche Diagnostics) plate. Caco-2 cells were detached from the dish and seeded at $1.3 \times 10^3$ cells/well (50 μl/well). The live cell assay was then initiated using the xCELLigence Real-Time Cell Analyzer. On the following day, the plate was removed from the xCELLigence Real-Time Cell Analyzer and 50 μL of each antibody prepared at various concentrations (0.1, 1.0, and 10 μg/mL) was added to each well of the plate. In addition, 50 μL of human PBMC suspension ($5 \times 10^4$ cells/well) was added. The plate was placed again on the xCELLigence Real-Time Cell Analyzer and live cell assay was initiated. The reaction was carried out at 37°C under 5% carbon dioxide gas. The cell growth inhibition rate (%) was determined according to the following equation using cell index values 72 hours after addition of human PBMC. The cell index values used for the calculation were normalized such that the cell index value immediately before addition of the antibody was 1.

$$\text{Cell growth inhibition rate (\%)} = \frac{A-B}{A-1} \times 100$$

A represents the mean cell index value of antibody-free wells (only target cells and human PBMC), and B represents the mean cell index value of each well. The measurement was repeated three times.

The cytotoxic activity of the anti-KLH/CD3 bispecific antibodies, TR01H113//MDA0279 and TR01H113//MDA0314, was measured using peripheral blood mononuclear cells (PBMC). TR01H113//MDA0279 and TR01H113//MDA0314 showed high cell growth inhibition rates in comparison to the control anti-KLH/CD3 bispecific antibody (Fig. 10).

(Example 12) Preparation of human NOX1 protein

A gene encoding the full-length human NOX1 sequence (amino acid SEQ ID NO: 2) with a Twin-Strep tag sequence (WSHPQFEKGGGSGGGSGGSAWSHPQFEK, SEQ ID NO: 31) added to the C-terminus (human NOX1-Strep, amino acid SEQ ID NO: 34) was synthesized, and cloned into a mammalian cell expression vector. The protein was transiently expressed by introducing this expression vector into Expi293 cells (Thermo Fisher Scientific).

The obtained cells were collected by centrifugation, and Fos-choline 14 and cholesterol hemisuccinate were added at final concentrations of 1% and 0.2%, respectively, to solubilize the human NOX1--Strep protein. The solubilized protein sample was purified by affinity chromatography using Strep-Tactin XT Superflow (IBA GmbH). Fractions containing the human NOX1--Strep protein were collected and Amphipol A8-35 solution (Anatrace) was added at a final concentration of 2 mg/mL. Immediately after that, Bio-Beads SM-2 Adsorbent Media (BIO-RAD) was added and incubated at 4°C for 12-16 hours to remove Fos-choline 14/ cholesterol hemisuccinate and reconstitute the human NOX1--Strep protein into Amphipol A8-35. The reconstituted human NOX1--Strep protein sample was purified by gel filtration chromatography using a Superdex 200 increase 10/300 column (GE Healthcare). Fractions containing the human NOX1--Strep protein were collected and concentrated using a 50 kDa MWCO ultrafiltration membrane. To the concentrated sample, Amphipol A8-35 was added at a final concentration of 2 mg/mL to obtain a final preparation.

(Example 13) Preparation of anti-human NOX1 antibodies

Two New Zealand White rabbits (Kitayama Labes) were immunized four times using the human NOX1-Strep protein prepared in Example 12, and peripheral blood mononuclear cells and spleen cells were harvested from the rabbits 6 days after the final immunization. A gene encoding amino acids 1 to 564 of human NOX1 (SEQ ID NO: 2) with the signal sequence and the transmembrane region of Neurexin IB-delta fused to the N-terminus (Cell. 2018 Nov 1, 175(4):1131-1140.e11.) and a Myc tag sequence (EQKLISEEDL) added to the C-terminus (NOX1_Nx1B_564-myc, nucleotide SEQ ID NO: 32, amino acid SEQ ID NO: 33) was synthesized, and this was cloned into a mammalian cell plasmid expression vector. Using the constructed plasmid vector, eight New Zealand White rabbits (Kitayama Labes) were immunized six times by a gene gun method and a Tropis (PharmaJet) device, and spleen cells were harvested from the rabbits six or seven days after the final immunization. Surface IgG-positive B cells were concentrated from the harvested rabbit-derived cells by MACS and FACS using PE-labeled anti-rabbit IgG antibody (Southern biotech), and cultured. B cells secreting an antibody that binds to human NOX1 were identified from the cultured B cells using a fluorescence microscope attached to the micromanipulator CellCelector (ALS) and collected in a microwell plate using the micromanipulator CellCelector (ALS). Genes constituting the variable regions of rabbit immunoglobulin were cloned from the collected B cells by RT-PCR. These rabbit immunoglobulin variable region DNA fragments were inserted into an expression vector containing IgG constant regions to prepare recombinant antibodies. The anti-human NOX1 antibodies were prepared by a method known to those skilled in the art. The prepared anti-human NOX1 antibodies are shown in Table 7.

[Table 7]

| Name of antibody | NXA0125 | NXA0164 |
|---|---|---|
| HVR_H1 | SEQ ID NO: 67 | SEQ ID NO: 75 |
| HVR_H2 | SEQ ID NO: 68 | SEQ ID NO: 76 |
| HVR_H3 | SEQ ID NO: 69 | SEQ ID NO: 77 |
| HVR_L1 | SEQ ID NO: 70 | SEQ ID NO: 78 |
| HVR_L2 | SEQ ID NO: 71 | SEQ ID NO: 79 |
| HVR_L3 | SEQ ID NO: 72 | SEQ ID NO: 80 |
| VH | SEQ ID NO: 73 | SEQ ID NO: 81 |
| VL | SEQ ID NO: 74 | SEQ ID NO: 82 |

As an anti-human CD3 antibody, CE115TR (heavy chain variable region SEQ ID NO: 65, light chain variable region SEQ ID NO: 66), which binds to the CD3ε chain constituting the T cell receptor and induces an activation signal of the T cell receptor, was prepared by a method known to those skilled in the art. The present inventors prepared bispecific antibodies consisting of an anti-human NOX1 antibody and an anti-CD3 antibody by a method known to those skilled in the art. The prepared anti-human NOX1/anti-human CD3 bispecific antibodies are shown in Table 8. The heavy chain constant region sequence on the anti-human NOX1 side of the bispecific antibodies is the sequence shown in SEQ ID NO: 83, the light chain constant region sequence on the anti-human XPR1 side is the sequence shown in SEQ ID NO: 85, the heavy chain constant region sequence on the anti-human CD3 side is the sequence shown in SEQ ID NO: 84, and the light chain constant region sequence on the anti-human CD3 side is the sequence shown in SEQ ID NO: 86.

[Table 8]

| Name of Antibody | | TR01H113//NXA0125 | TR01H113//NXA0164 |
|---|---|---|---|
| Anti-human NOX1 | HVR_H1 | SEQ ID NO: 67 | SEQ ID NO: 75 |
| | HVR_H2 | SEQ ID NO: 68 | SEQ ID NO: 76 |
| | HVR_H3 | SEQ ID NO: 69 | SEQ ID NO: 77 |
| | HVR_L1 | SEQ ID NO: 70 | SEQ ID NO: 78 |
| | HVR_L2 | SEQ ID NO: 71 | SEQ ID NO: 79 |
| | HVR_L3 | SEQ ID NO: 72 | SEQ ID NO: 80 |
| | VH | SEQ ID NO: 73 | SEQ ID NO: 81 |
| | VL | SEQ ID NO: 74 | SEQ ID NO: 82 |

(continued)

| Name of Antibody | | TR01H113//NXA0125 | TR01H113//NXA0164 |
|---|---|---|---|
| Anti-human CD3 | HVR_H1 | SEQ ID NO: 59 | |
| | HVR_H2 | SEQ ID NO: 60 | |
| | HVR_H3 | SEQ ID NO: 61 | |
| | HVR_L1 | SEQ ID NO: 62 | |
| | HVR_L2 | SEQ ID NO: 63 | |
| | HVR_L3 | SEQ ID NO: 64 | |
| | VH | SEQ ID NO: 65 | |
| | VL | SEQ ID NO: 66 | |

(Example 14) Confirmation of binding of anti-human NOX1 antibodies to human NOX1

A plasmid vector expressing human NOX1-Strep (amino acid SEQ ID NO: 34) or a plasmid vector expressing NOX1_Nx1B_564-myc (amino acid SEQ ID NO: 33) was introduced into Expi293 cells (Thermo Fisher Scientific) to express the protein transiently. At the same time, Expi293 cells transfected with an empty plasmid vector were also prepared as a control. These cells were treated with 4% paraformaldehyde and 1% digitonin. The Expi293 cells transfected with an empty plasmid vector were stained with CellTrace FarRed (Thermo Fisher Scientific), the Expi293 cells transfected with the plasmid vector expressing human NOX1-Strep were stained with CellTrace FarRed and CellTrace Violet (Thermo Fisher Scientific), and then mixed with the Expi293 cells made to express NOX1_Nx1B_564-myc. Subsequently, the cells were reacted with an anti-human NOX1 antibody prepared in Example 13.

Then, anti-human IgG Fc cross-adsorbed Alexa488 (Invitrogen) was reacted to stain the anti-human NOX1 antibody. The cells reacted with the antibody were measured by FACS Aria III (Becton, Dickinson and Company). The obtained data were analyzed by FlowJo ver.10. Fractions stained with CellTrace FarRed (cells transfected with the empty plasmid vector), not stained (cells transfected with human NOX1-Strep), and stained with CellTrace FarRed and CellTrace Violet (cells transfected with NOX1_Nx1B_564-myc) were separated, and Alexa488 staining for each fraction was examined. More intense staining was observed in the unstained fraction (cells transfected with human NOX1-Strep) and the fraction stained with CellTrace FarRed and CellTrace Violet (cells transfected with NOX1_Nx1B_564-myc) than in the fraction stained with CellTrace FarRed (cells transfected with the empty plasmid vector) (Fig. 11). This result confirmed that the anti-human NOX1 antibodies prepared in Example 13 bind to human NOX1 expressed on cells.

Although the foregoing invention has been described in some detail by way of illustration and example for purposes of clarity of understanding, the descriptions and examples should not be construed as limiting the scope of the present disclosure. The disclosures of all patent and scientific literature cited herein are expressly incorporated in their entirety by reference.

[Industrial Applicability]

The antibodies of the present disclosure have at least one of cytotoxic activity and internalization activity, and are useful for either or both treatment and prevention of cancer (for example, lung cancer and colorectal cancer).

## Claims

**1.** An isolated antibody that binds to any of the following proteins: XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, and TMPRSS4.

**2.** The antibody of claim 1, which binds to an extracellular domain of the protein.

**3.** The antibody of claim 1 or 2, which binds to any of:

(1) XPR1 represented by SEQ ID NO: 1,
(2) NOX1 represented by SEQ ID NO: 2,
(3) MARVELD3 isoform 1 represented by SEQ ID NO: 3,
(4) MARVELD3 isoform 2 represented by SEQ ID NO: 4,
(5) SPINT2 represented by SEQ ID NO: 5,
(6) MANSC1 represented by SEQ ID NO: 7,
(7) SLC12A2 represented by SEQ ID NO: 8,
(8) CDCP1 represented by SEQ ID NO: 9,
(9) SEZ6L2 represented by SEQ ID NO: 12,
(10) FLVCR1 represented by SEQ ID NO: 13,
(11) SLC7A5 represented by SEQ ID NO: 14,
(12) STEAP1 represented by SEQ ID NO: 15,
(13) MMP14 represented by SEQ ID NO: 16,
(14) TNFRSF21 represented by SEQ ID NO: 17, and
(15) TMPRSS4 represented by SEQ ID NO: 18.

**4.** The antibody of claim 1 or 2, which binds to any of:

(1) an extracellular domain of XPR1, which is any of amino acids 1 to 108, 111 to 122, 159 to 171, 177 to 216, 423 to 448, 473 to 502, 660 to 670, 674 to 696, 177 to 190, 428 to 448, 660 to 670, 244, 256 to 273, 257 to 270, 258 to 264, 258 to 268, 256 to 270, 258 to 273, 260 to 270, 293 to 314, 336 to 343, 337 to 344, 338 to 341, 340 to 342, 340 to 344, 343 to 344, 340 to 345, 368 to 372, 392 to 398, 397 to 401, 398 to 402, 420 to 442, 420 to 506, 465 to 479, 497 to 507, 498 to 508, 529 to 555, 529 to 570, 582 to 586, 1 to 234, 1 to 236, 293 to 318, 367 to 442, 369 to 473, 500 to 507, 529 to 696, and 589 to 696 in the amino acid sequence represented by SEQ ID NO: 1;
(2) an extracellular domain of NOX1, which is any of amino acids 44 to 54, 131 to 161, 242 to 258, 1 to 4, 1 to 11, 18 to 55, 28 to 44, 31 to 44, 32 to 46, 34 to 50, 70 to 102, 70 to 103, 117 to 176, 120 to 172, 122 to 166, 122 to 172, 124 to 168, 190 to 208, 191 to 204, 223 to 266, 223 to 267, 227 to 269, 228 to 391, 228 to 396, 404, and 420 to 564 in the amino acid sequence represented by SEQ ID NO: 2;

(3) an extracellular domain of MARVELD3 isoform 1, which is any of amino acids 222 to 266, 223 to 269, 227 to 264, 227 to 268, 229 to 263, 231 to 265, 321 to 362, 322 to 357, 323 to 357, 324 to 357, and 324 to 358 in the amino acid sequence represented by SEQ ID NO: 3; and
(4) an extracellular domain of MARVELD3 isoform 2, which is any of amino acids 101 to 111, 163 to 198, 1 to 266, 1 to 270, 216 to 271, 222 to 269, 226 to 271, 227 to 268, 227 to 271, 248 to 271, 316 to 364, 322 to 360, 323 to 359, 324 to 360, 326 to 360, and 327 to 360 in the amino acid sequence represented by SEQ ID NO: 4.

**5.** The antibody of any one of claims 1 to 4, which has cytotoxic activity.

**6.** The antibody of any one of claims 1 to 5, which is a multispecific antibody.

**7.** The antibody of claim 6, further comprising a T cell receptor complex-binding domain.

**8.** The antibody of claim 6 or 7, comprising an Fc region with reduced Fcγ receptor-binding activity.

**9.** The antibody of claim 7 or 8, wherein the antibody has cytotoxic activity, and the cytotoxic activity is a T cell-dependent cytotoxic activity.

**10.** The antibody of any one of claims 7 to 9, wherein the T cell receptor complex-binding domain is a T cell receptor-binding domain having T cell receptor-binding activity.

**11.** The antibody of any one of claims 7 to 9, wherein the T cell receptor complex-binding domain is a CD3-binding domain having CD3-binding activity.

**12.** A pharmaceutical formulation comprising the antibody of any one of claims 1 to 11 and a pharmaceutically acceptable carrier.

**13.** The antibody of any one of claims 1 to 11 for use in treatment of cancer.

**14.** The antibody of claim 13, wherein the cancer is lung cancer, and the antibody binds to any of XPR1, SPINT2, SEZ6L2, FLVCR1, SLC7A5, STEAP1, MMP14, TNFRSF21, and TMPRSS4.

**15.** The antibody of claim 13, wherein the cancer is colorectal cancer, and the antibody binds to any of NOX1, MARVELD3 isoform 1, MARVELD3 isoform 2, SPINT2, MANSC1, SLC12A2, and CDCP1.

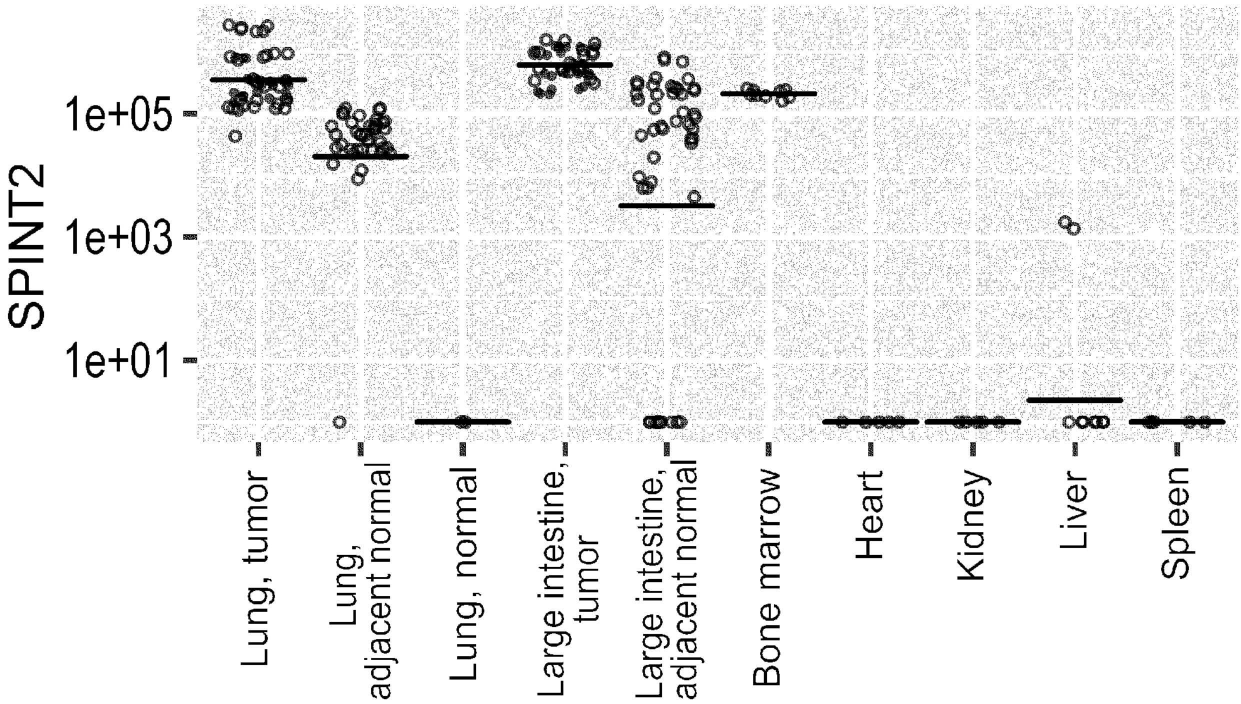
SPINT2
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-1

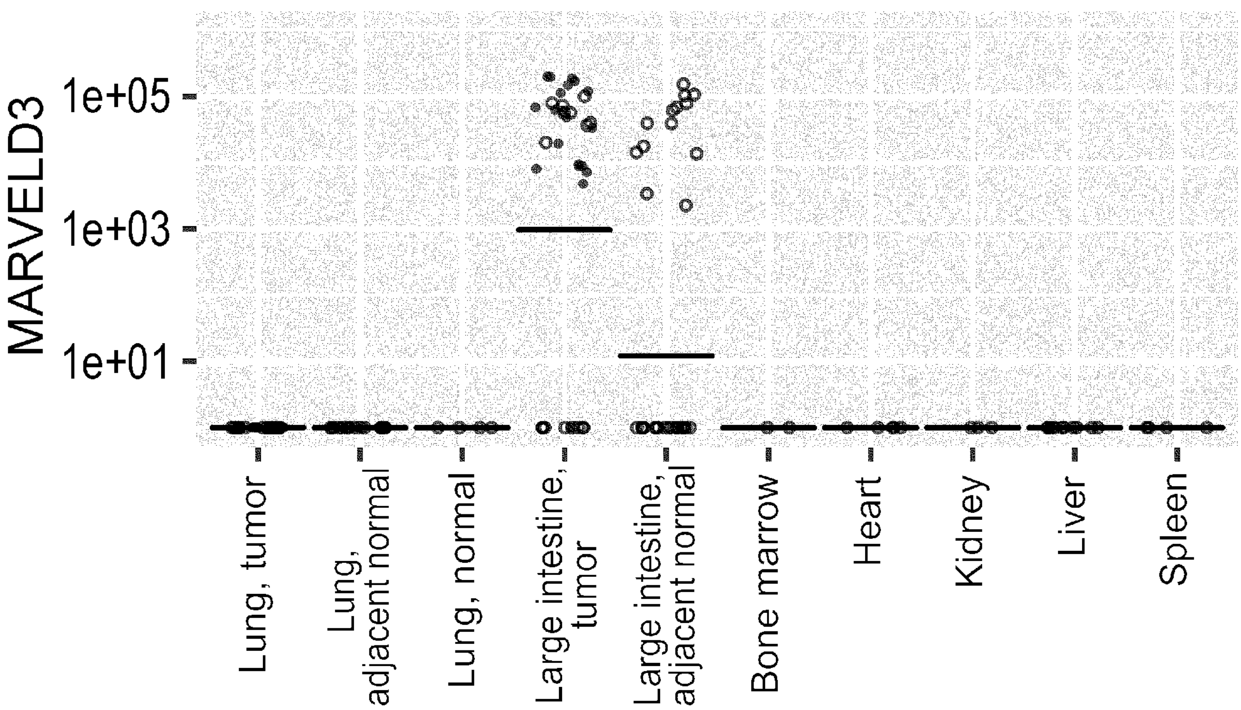
MARVELD3
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-2

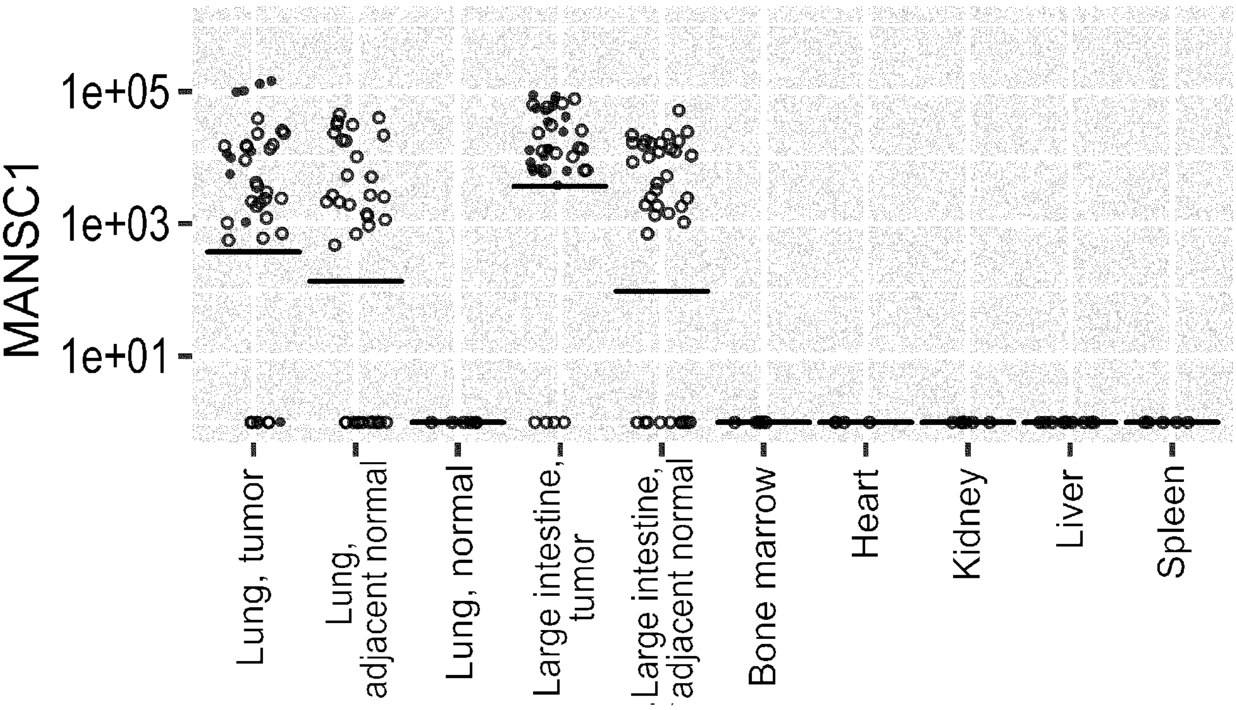
MANSC1
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-3

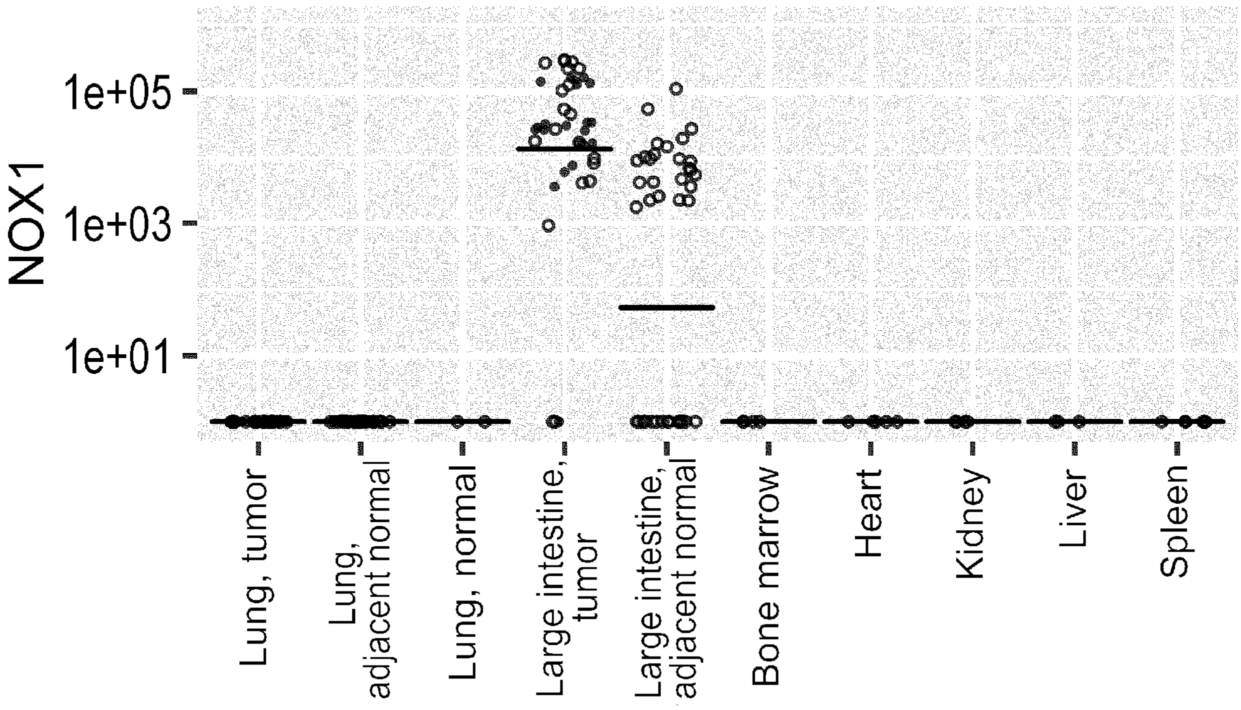
NOX1
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-4

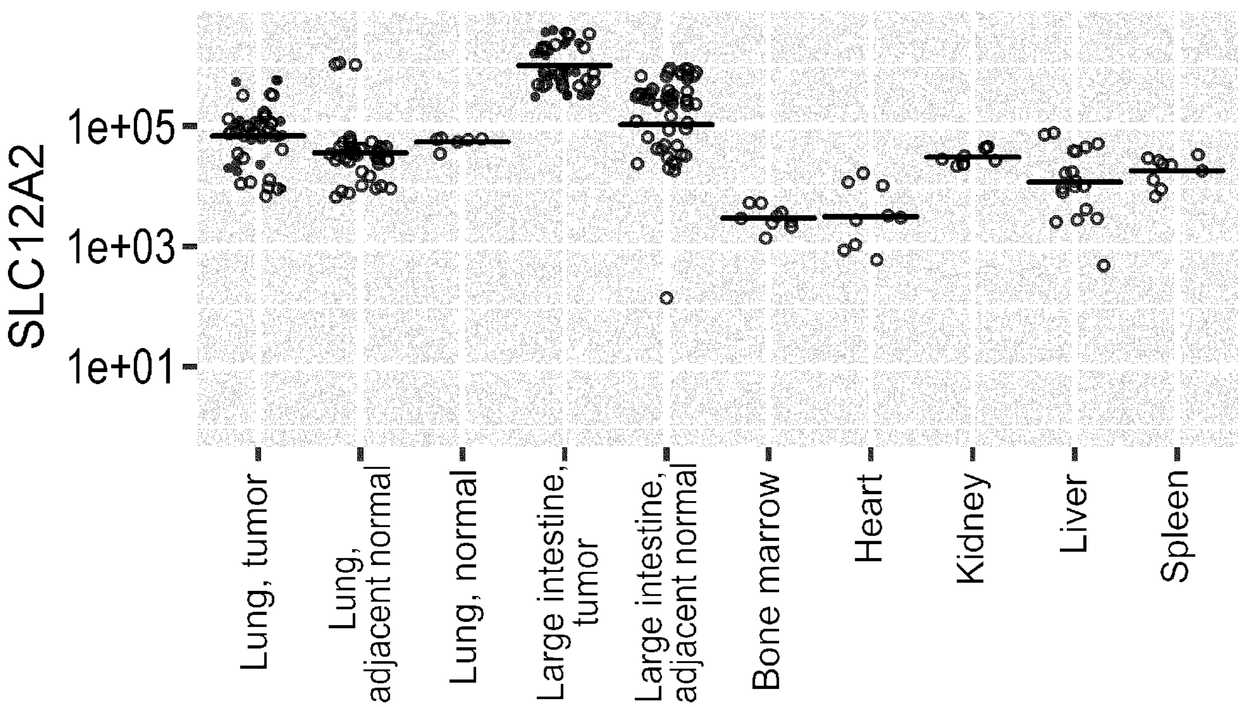
SLC12A2
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-5

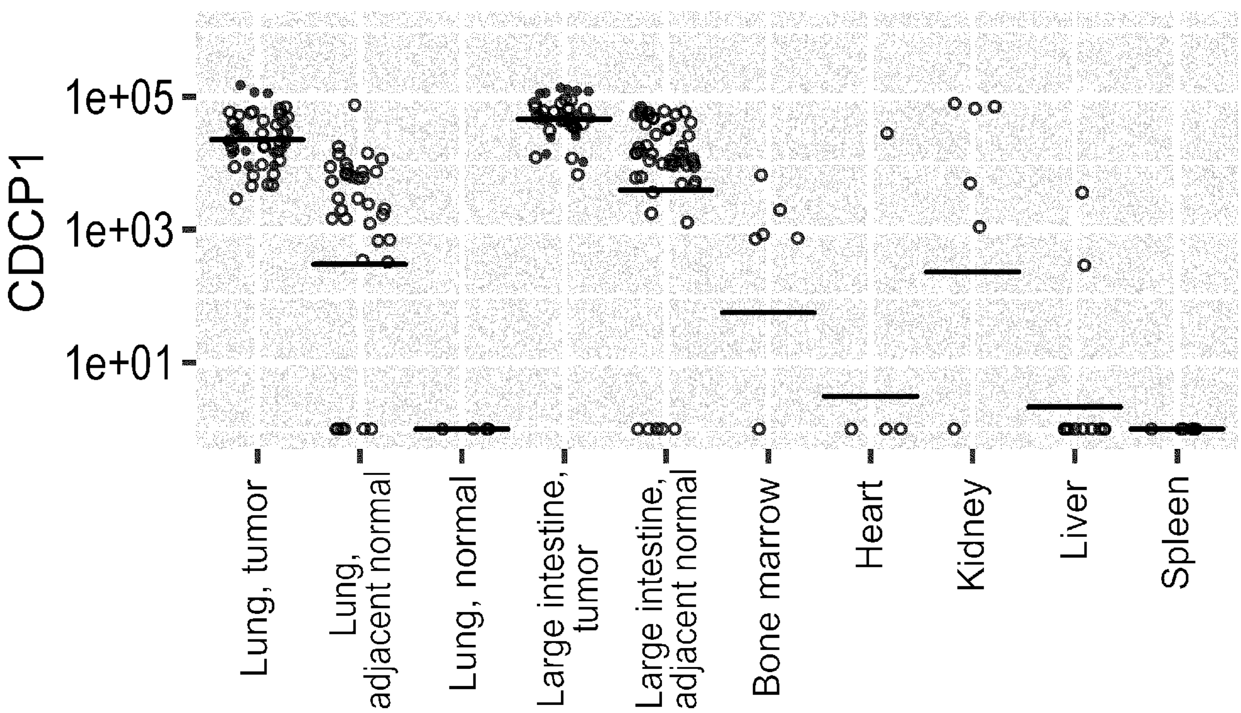
CDCP1
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-6

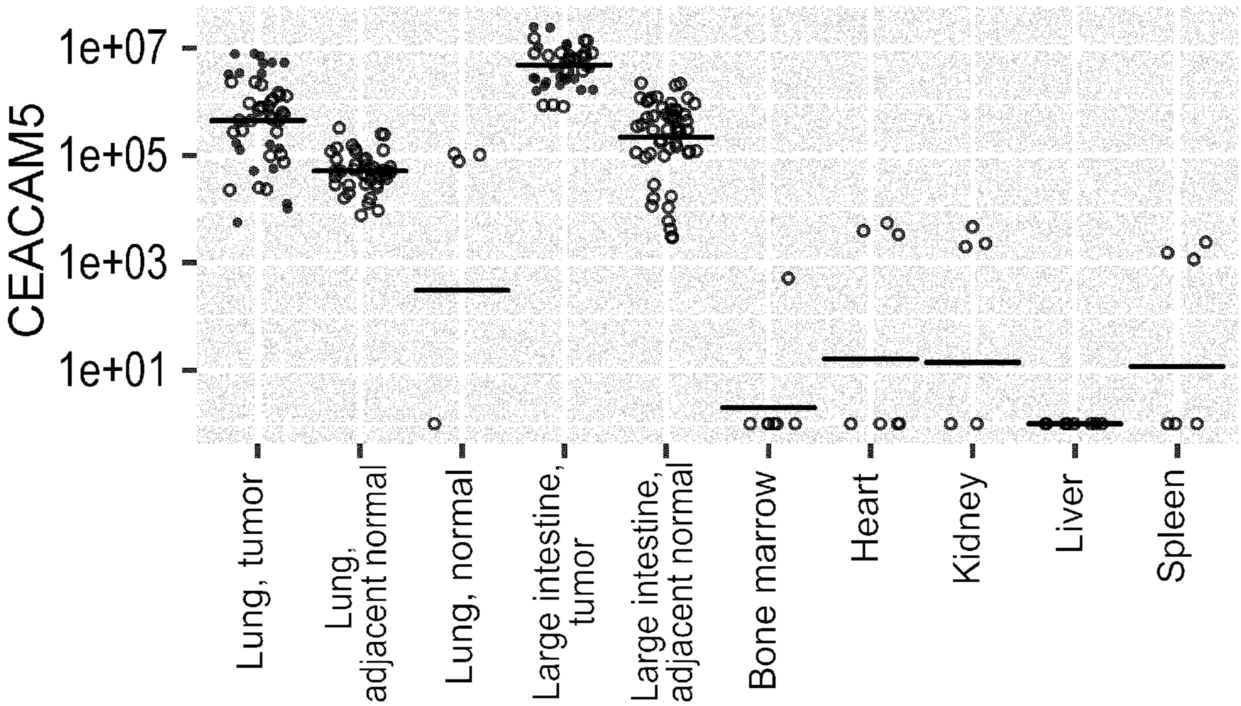
CEACAM5
1e+07
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-7

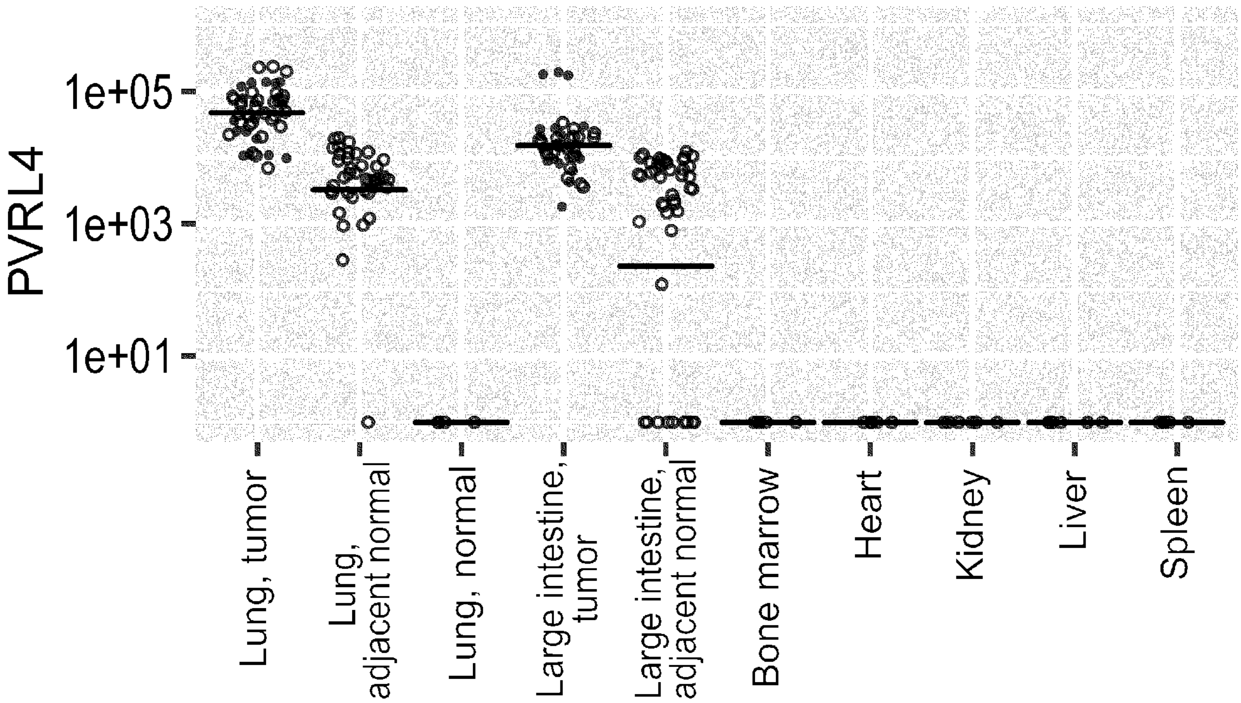
PVRL4
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-8

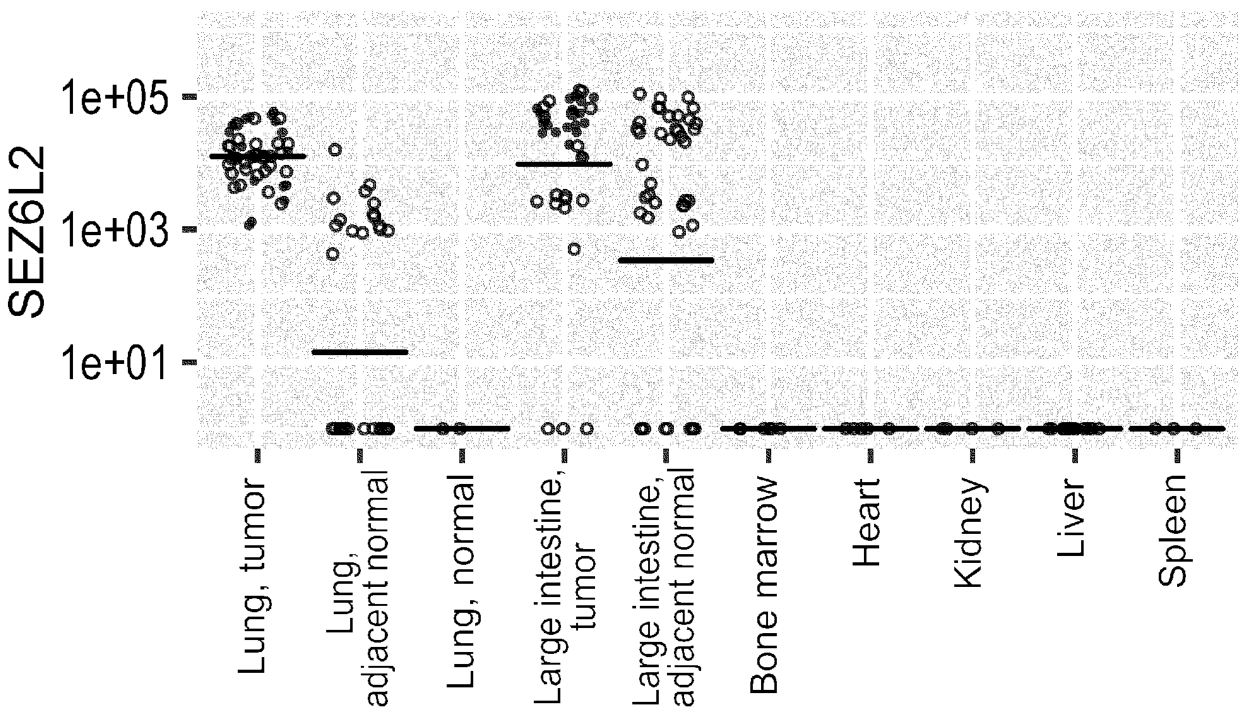
SEZ6L2
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-9

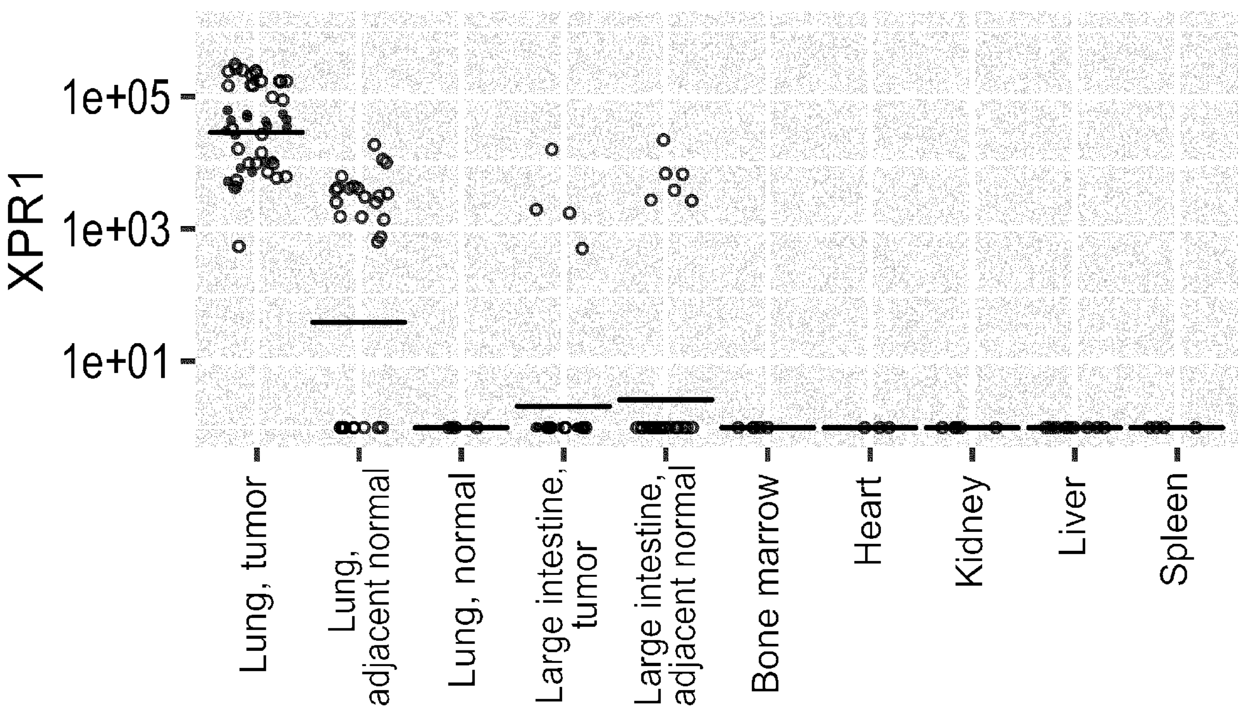
XPR1
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-10

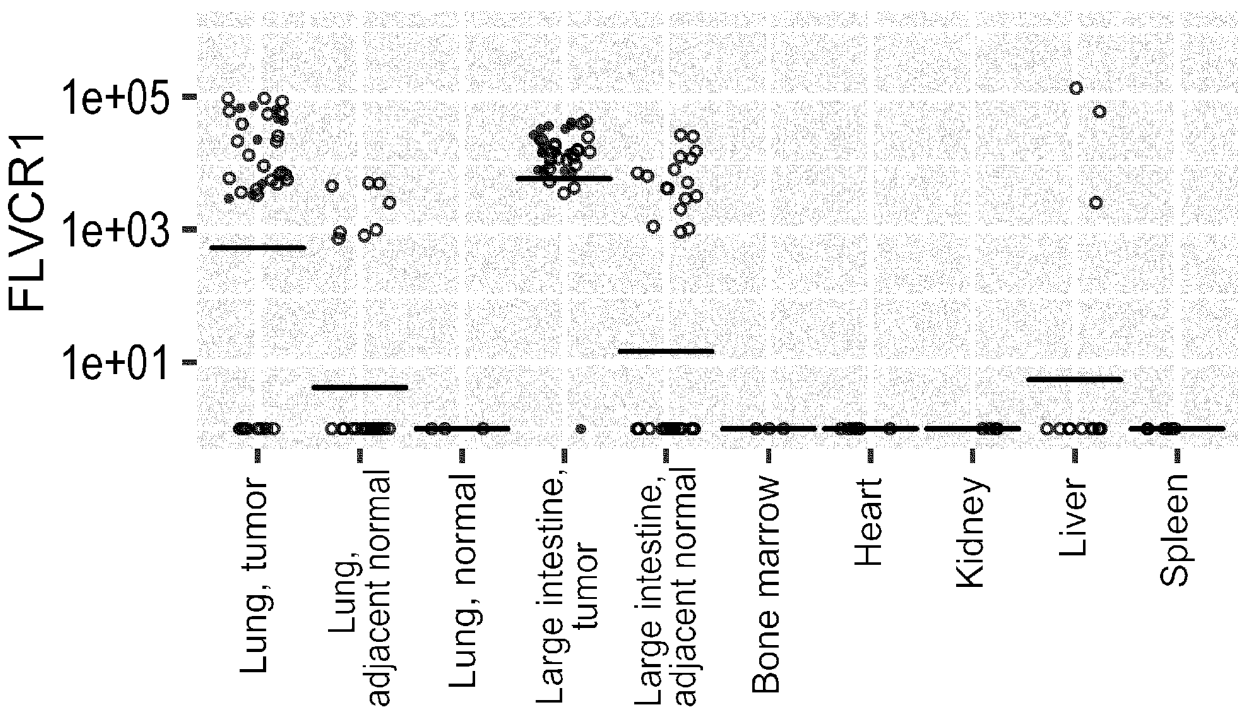
FLVCR1
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-11

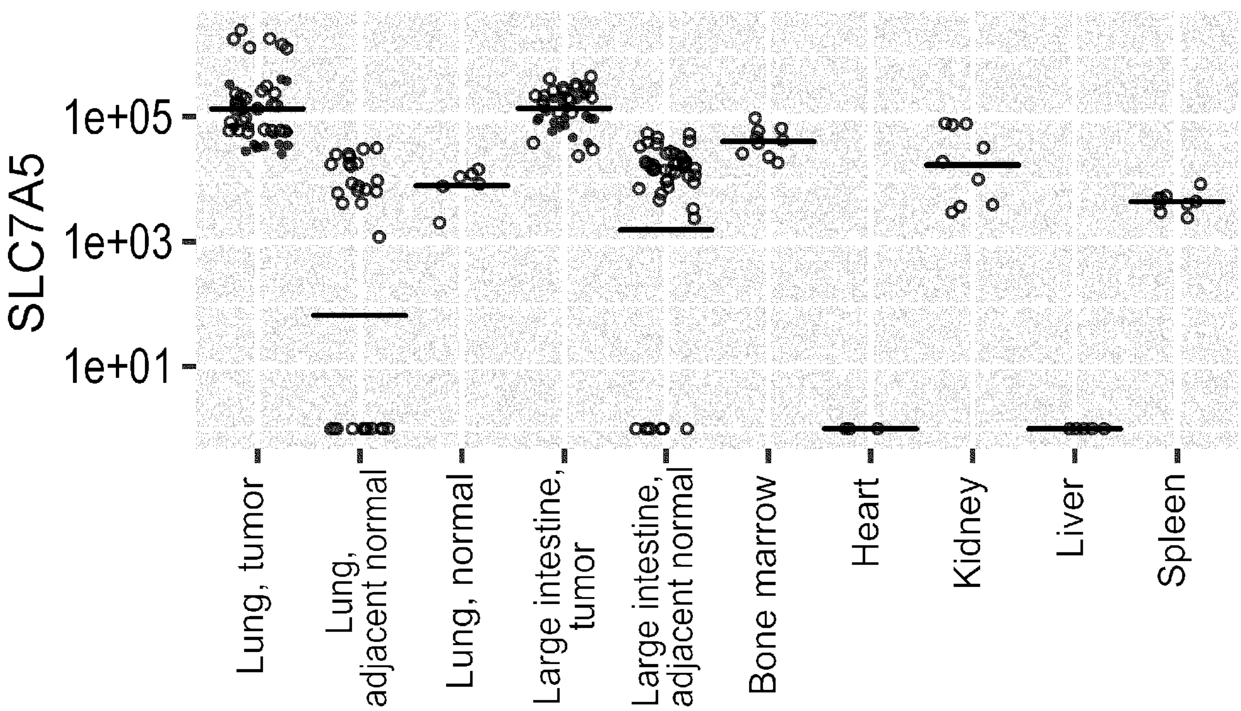

SLC7A5
1e+05
1e+03
1e+01
Lung, tumor
Lung, adjacent normal
Lung, normal
Large intestine, tumor
Large intestine, adjacent normal
Bone marrow
Heart
Kidney
Liver
Spleen


FIG. 1-12

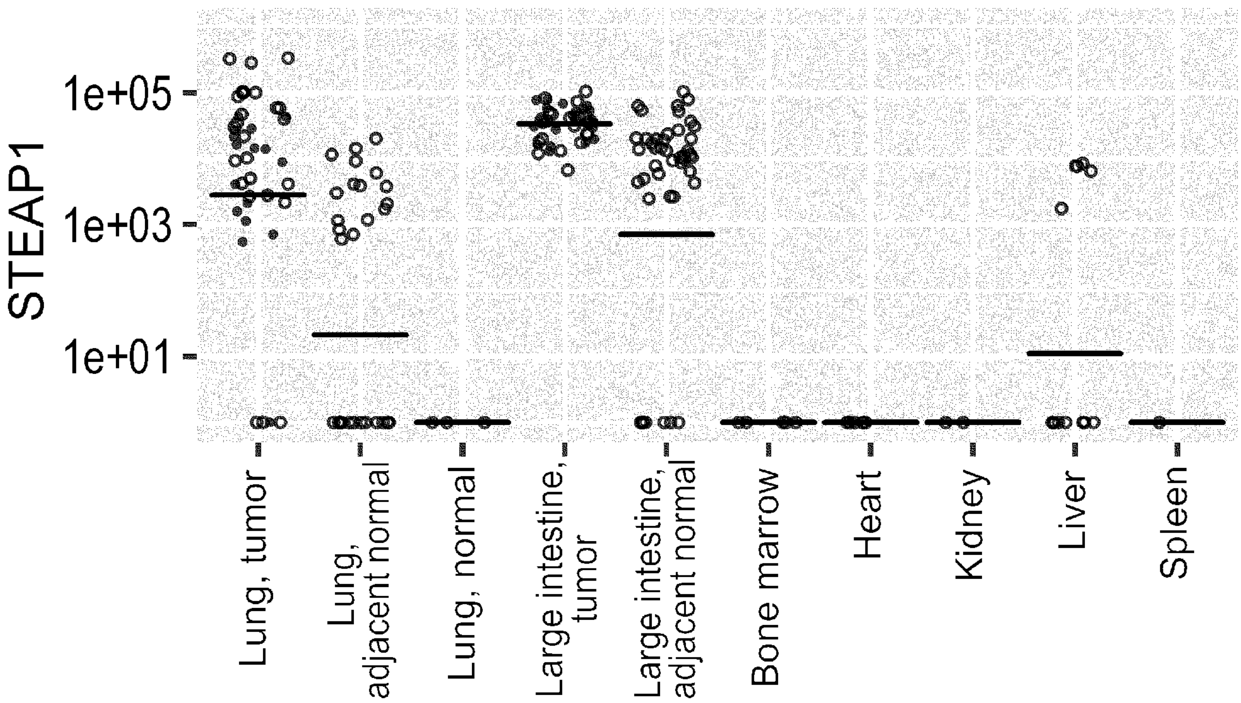
STEAP1
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-13

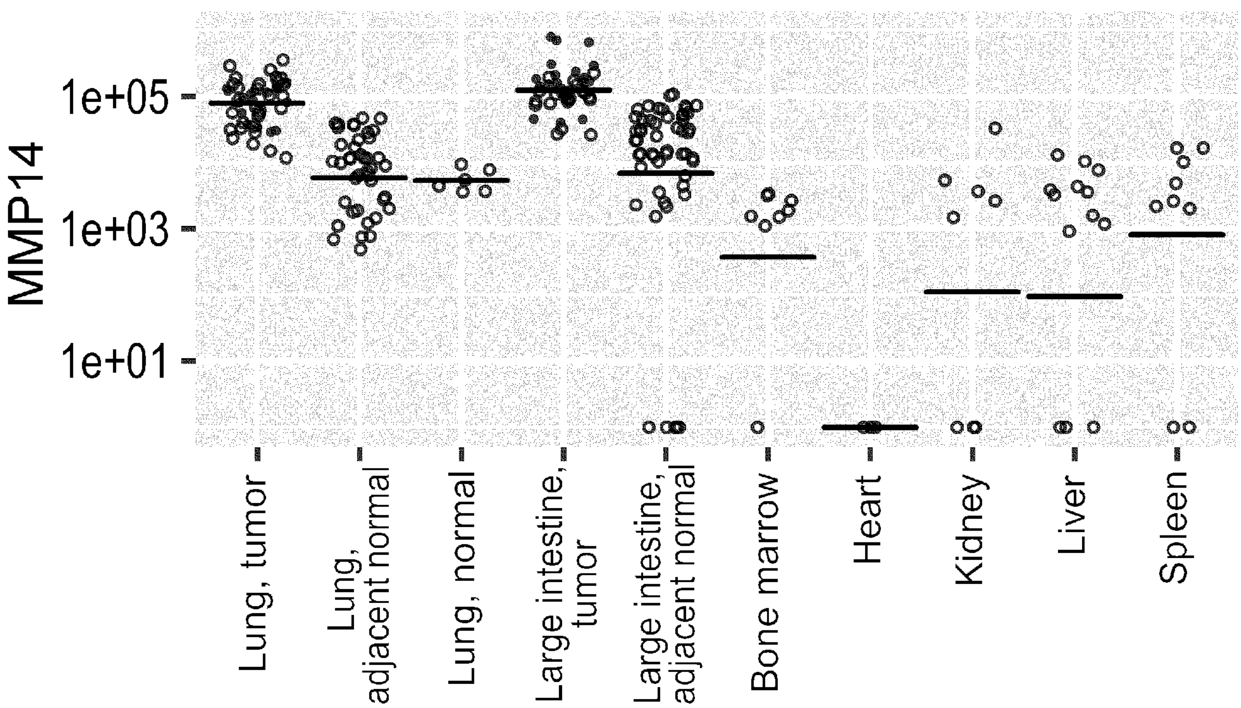
MMP14
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-14

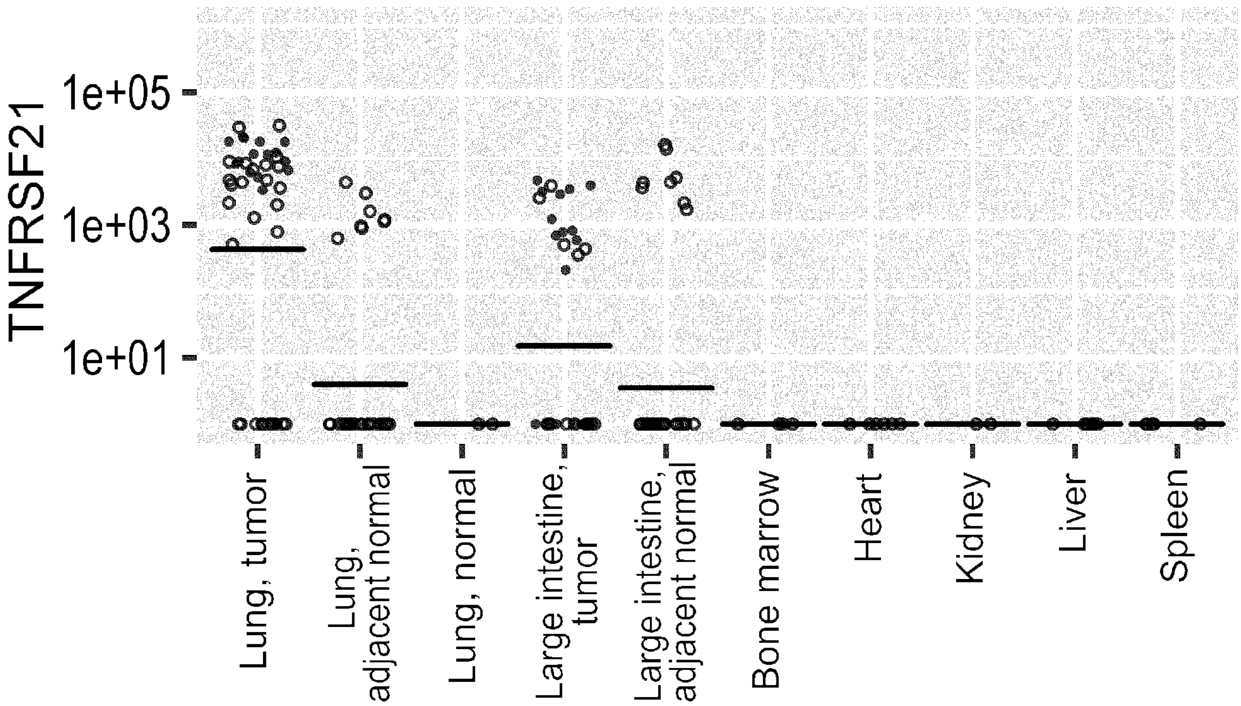
TNFRSF21
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-15

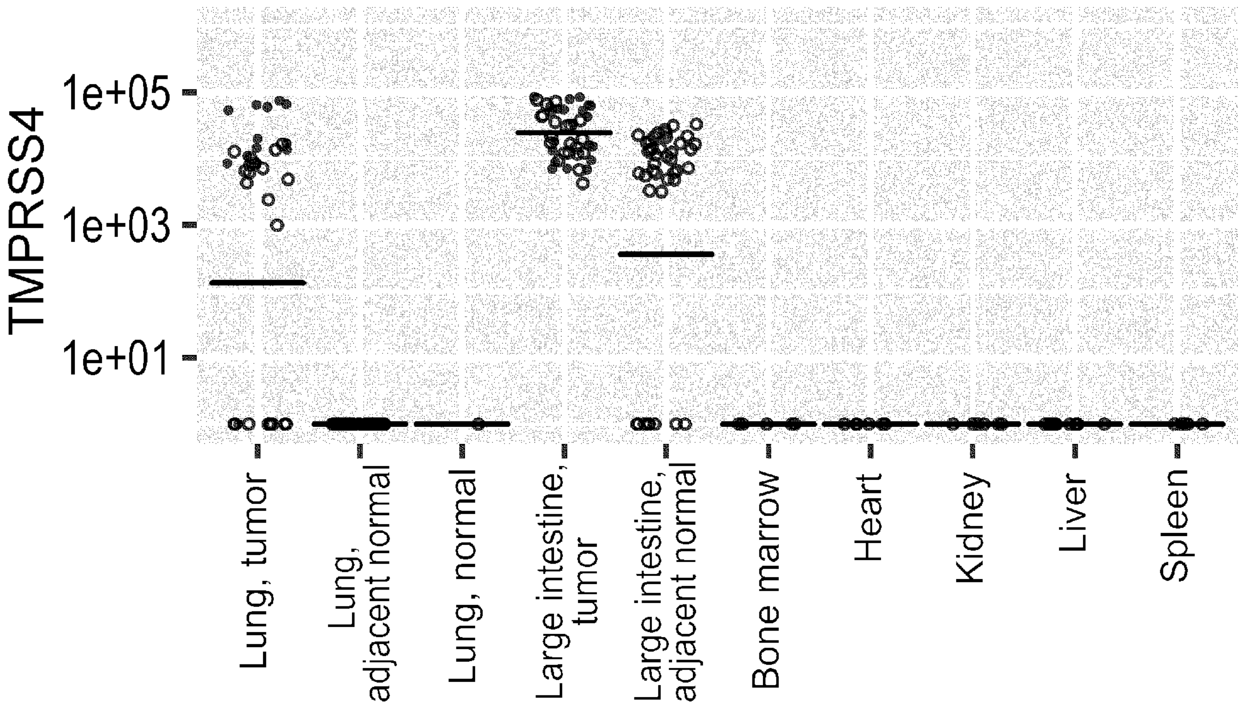
TMPRSS4
1e+05
1e+03
1e+01
Lung, tumor
Lung, adjacent normal
Lung, normal
Large intestine, tumor
Large intestine, adjacent normal
Bone marrow
Heart
Kidney
Liver
Spleen

FIG. 1-16

```
hMARVELD3 isoform1    1 MEDPSGAREPRARPRERDPGRRPHPDQGRTHDRPRDRPGDPRRKRSSDGNRRRDGDRDPERDQERDGNRD 70
hMARVELD3 isoform2    1 ...................................................................... 70

hMARVELD3 isoform1   71 RNRDRERERERERDPDRGPRRDTHRDAGPRAGEHGVWEKPRQSRTRDGARGLTWDAAAPPGPAPWEAPEP 140
hMARVELD3 isoform2   71 ...................................................................... 140

hMARVELD3 isoform1  141 PQPQRKGDPGRRRPESEPPSERYLPSTPRPGREEVEYYQSEAEGLLECHKCKYLCTGRGVVQIVEVVLNG 210
hMARVELD3 isoform2  141 ..........................................................ACC.ML..L..L 210

hMARVELD3 isoform1  211 MVLICIVASYFVLAGFSASFSSGGGFGNNY---YSPFEGTELEQVRQLDQQYTILRSPLIYGGVAVSLGL 277
hMARVELD3 isoform2  211 LI.A.SSV..SSTG.YTGIT.L..IYYYQFGGA..G.D.ADG.KAQ...V.FYQ.KL.MVTVAM.C.GA. 280

hMARVELD3 isoform1  278 GVLTMGVLLQGAKSRTMLSGKWLLTEAAFSLLAAVGYCTGIGVYLHVALQINSTDTCKTRERLYARKGLT 347
hMARVELD3 isoform2  281 TA.CCLFVAM.VLRVPWHCPLL.V..GLLDM.I.G..IPALYF.F.YLSAAYGSPV..E.QA..QS..YS 350

hMARVELD3 isoform1  348 WMDCQLAGTDGAAATFACLLVIMYGASVVLALRSYREQKRYKGSREQPGSYSDAPEYLWSGTL        410
hMARVELD3 isoform2  351 GFG.SFH.A.IG.GI..A.GIVVFALGA...IKG..KVRKL.EKPAEMFEF------------        401
```

FIG. 2

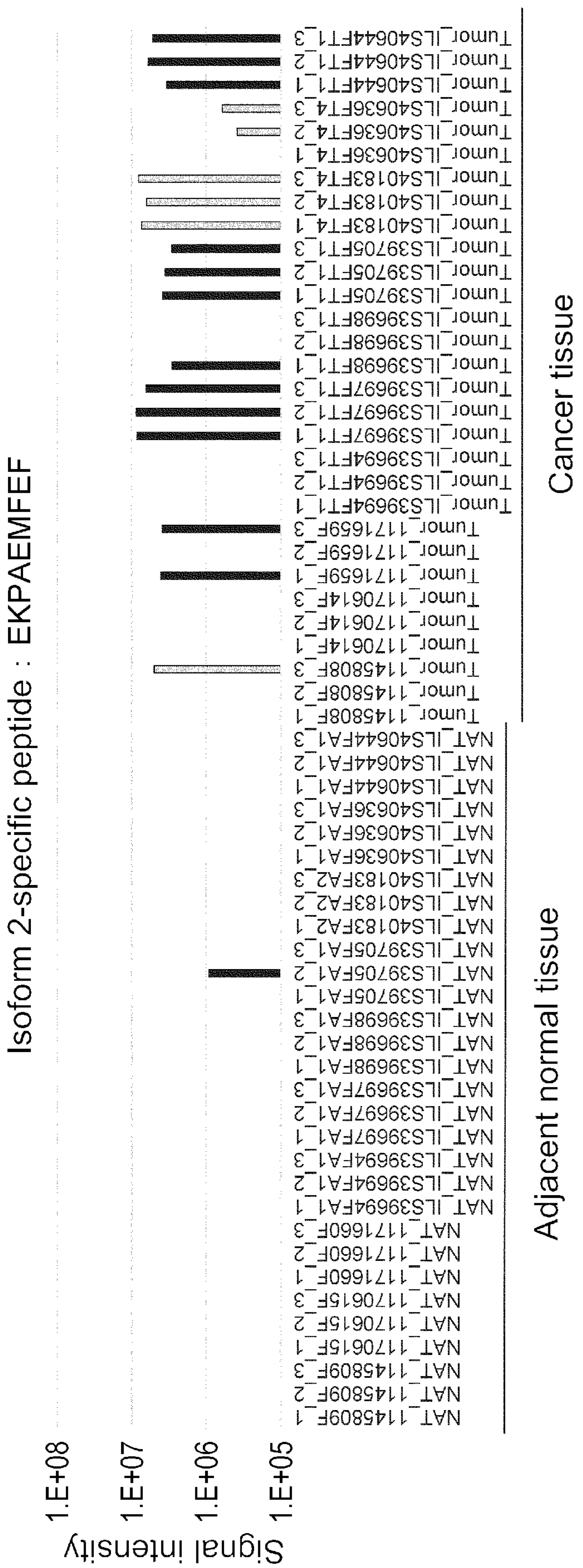
Isoform 2-specific peptide : EKPAEMFEF
Signal intensity
1.E+08
1.E+07
1.E+06
1.E+05
NAT_1145809F_1
NAT_1145809F_2
NAT_1145809F_3
NAT_1170615F_1
NAT_1170615F_2
NAT_1170615F_3
NAT_1171660F_1
NAT_1171660F_2
NAT_1171660F_3
NAT_ILS39694FA1_1
NAT_ILS39694FA1_2
NAT_ILS39694FA1_3
NAT_ILS39697FA1_1
NAT_ILS39697FA1_2
NAT_ILS39697FA1_3
NAT_ILS39698FA1_1
NAT_ILS39698FA1_2
NAT_ILS39698FA1_3
NAT_ILS39705FA1_1
NAT_ILS39705FA1_2
NAT_ILS39705FA1_3
NAT_ILS40183FA2_1
NAT_ILS40183FA2_2
NAT_ILS40183FA2_3
NAT_ILS40636FA1_1
NAT_ILS40636FA1_2
NAT_ILS40636FA1_3
NAT_ILS40644FA1_1
NAT_ILS40644FA1_2
NAT_ILS40644FA1_3
Tumor_1145808F_1
Tumor_1145808F_2
Tumor_1145808F_3
Tumor_1170614F_1
Tumor_1170614F_2
Tumor_1170614F_3
Tumor_1171659F_1
Tumor_1171659F_2
Tumor_1171659F_3
Tumor_ILS39694FT1_1
Tumor_ILS39694FT1_2
Tumor_ILS39694FT1_3
Tumor_ILS39697FT1_1
Tumor_ILS39697FT1_2
Tumor_ILS39697FT1_3
Tumor_ILS39698FT1_1
Tumor_ILS39698FT1_2
Tumor_ILS39698FT1_3
Tumor_ILS39705FT1_1
Tumor_ILS39705FT1_2
Tumor_ILS39705FT1_3
Tumor_ILS40183FT4_1
Tumor_ILS40183FT4_2
Tumor_ILS40183FT4_3
Tumor_ILS40636FT4_1
Tumor_ILS40636FT4_2
Tumor_ILS40636FT4_3
Tumor_ILS40644FT1_1
Tumor_ILS40644FT1_2
Tumor_ILS40644FT1_3
Adjacent normal tissue
Cancer tissue

FIG. 3-1

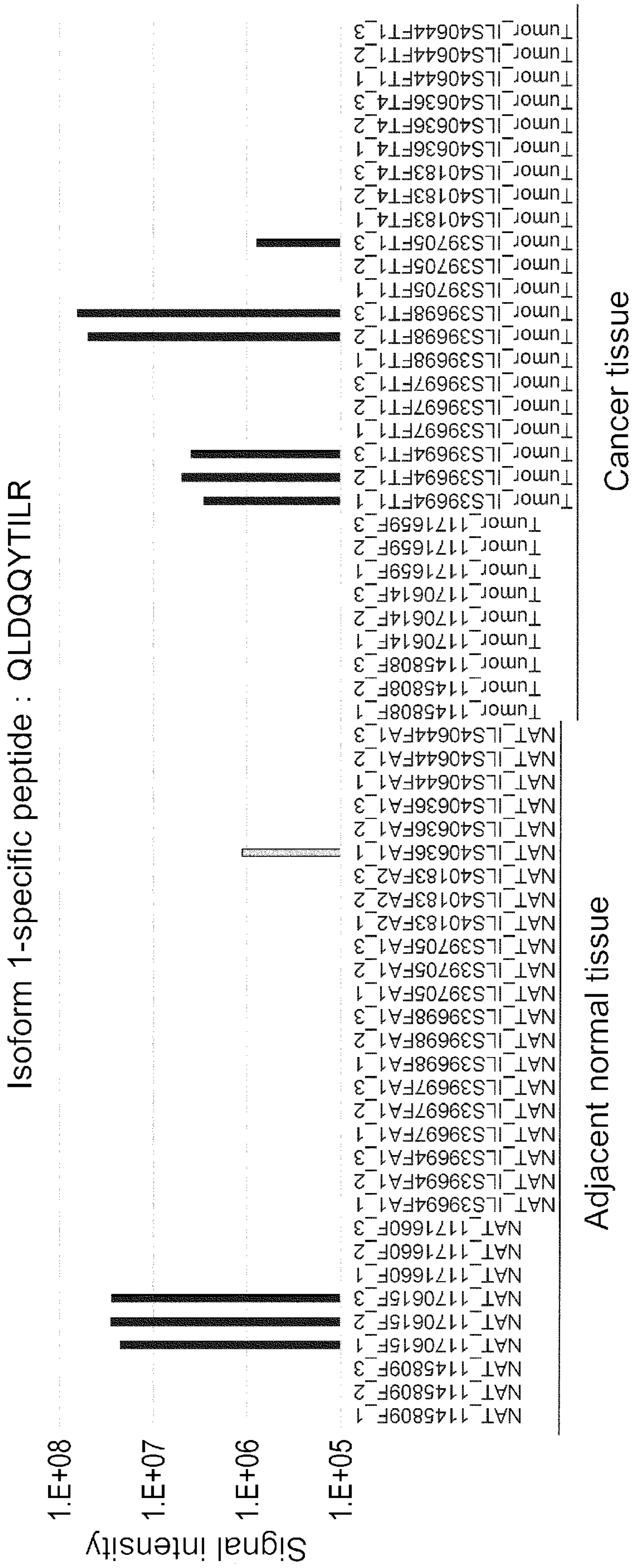
Isoform 1-specific peptide : QLDQQYTILR
Signal intensity
1.E+08
1.E+07
1.E+06
1.E+05
NAT_1145809F_1
NAT_1145809F_2
NAT_1145809F_3
NAT_1170615F_1
NAT_1170615F_2
NAT_1170615F_3
NAT_1171660F_1
NAT_1171660F_2
NAT_1171660F_3
NAT_ILS39694FA1_1
NAT_ILS39694FA1_2
NAT_ILS39694FA1_3
NAT_ILS39697FA1_1
NAT_ILS39697FA1_2
NAT_ILS39697FA1_3
NAT_ILS39698FA1_1
NAT_ILS39698FA1_2
NAT_ILS39698FA1_3
NAT_ILS39705FA1_1
NAT_ILS39705FA1_2
NAT_ILS39705FA1_3
NAT_ILS40183FA2_1
NAT_ILS40183FA2_2
NAT_ILS40183FA2_3
NAT_ILS40636FA1_1
NAT_ILS40636FA1_2
NAT_ILS40636FA1_3
NAT_ILS40644FA1_1
NAT_ILS40644FA1_2
NAT_ILS40644FA1_3
Tumor_1145808F_1
Tumor_1145808F_2
Tumor_1145808F_3
Tumor_1170614F_1
Tumor_1170614F_2
Tumor_1170614F_3
Tumor_1171659F_1
Tumor_1171659F_2
Tumor_1171659F_3
Tumor_ILS39694FT1_1
Tumor_ILS39694FT1_2
Tumor_ILS39694FT1_3
Tumor_ILS39697FT1_1
Tumor_ILS39697FT1_2
Tumor_ILS39697FT1_3
Tumor_ILS39698FT1_1
Tumor_ILS39698FT1_2
Tumor_ILS39698FT1_3
Tumor_ILS39705FT1_1
Tumor_ILS39705FT1_2
Tumor_ILS39705FT1_3
Tumor_ILS40183FT4_1
Tumor_ILS40183FT4_2
Tumor_ILS40183FT4_3
Tumor_ILS40636FT4_1
Tumor_ILS40636FT4_2
Tumor_ILS40636FT4_3
Tumor_ILS40644FT1_1
Tumor_ILS40644FT1_2
Tumor_ILS40644FT1_3
Adjacent normal tissue
Cancer tissue

FIG. 3-2

```
XPR1
MKFAEHLSAH ITPEWRKQYI QYEAFKDMLY SAQDQAPSVE VTDEDTVKRY  50
FAKFEEKFFQ TCEKELAKIN TFYSEKLAEA QRRFATLQNE LQSSLDAQKE 100
STGVTTLRQR RKPVFHLSHE ERVQHRNIKD LKLAFSEFYL SLILLQNYQN 150
LNFTGFRKIL KKHDKILETS RGADWRVAHV EVAPFYTCKK INQLISETEA 200
VVTNELEDGD RQKAMKRLRV PPLGAAQPAP AWTTFRVGLF CGIFIVLNIT 250
LVLAAVFKLE TDRSIWPLIR IYRGGFLLIE FLFLLGINTY GWRQAGVNHV 300
LIFELNPRSN LSHQHLFEIA GFLGILWCLS LLACFFAPIS VIPTYVYPLA 350
LYGFMVFFLI NPTKTFYYKS RFWLLKLLFR VFTAPFHKVG FADFWLADQL 400
NSLSVILMDL EYMICFYSLE LKWDESKGLL PNNSEESGIC HKYTYGVRAI 450
VQCIPAWLRF IQCLRRYRDT KRAFPHLVNA GKYSTTFFMV TFAALYSTHK 500
ERGHSDTMVF FYLWIVFYII SSCYTLIWDL KMDWGLFDKN AGENTFLREE 550
IVYPQKAYYY CAIIEDVILR FAWTIQISIT STTLLPHSGD IIATVFAPLE 600
VFRRFVWNFF RLENEHLNNC GEFRAVRDIS VAPLNADDQT LLEQMMDQDD 650
GVRNRQKNRS WKYNQSISLR RPRLASQSKA RDTKVLIEDT DDEANT
```

# FIG. 4-1

NOX1

MGNWVVNHWF SVLFLVVWLG LNVFLFVDAF LKYEKADKYY YTRK**ILGSTL** 50
**ACAR**ASALCL NFNSTLILLP VCRNLLSFLR GTCSFCSRTL RKQLDHNLTF 100
HKLVAYMICL HTAIHIIAHL FNFDCYSRSR **QATDGSLASI LSSLSHDEKK** 150
**GGSWLNPIQS R**NTTVEYVTF TSIAGLTGVI MTIALILMVT SATEFIRRSY 200
FEVFWYTHHL FIFYILGLGI HGIGGIVRGQ TEESMNESHP R**KCAESFEMW** 250
**DDRDSHCR**RP KFEGHPPESW KWILAPVILY ICERILRFYR SQQKVVITKV 300
VMHPSKVLEL QMNKRGFSME VGQYIFVNCP SISLLEWHPF TLTSAPEEDF 350
FSIHIRAAGD WTENLIRAFE QQYSPIPRIE VDGPFGTASE DVFQYEVAVL 400
VGAGIGVTPF ASILKSIWYK FQCADHNLKT KKIYFYWICR ETGAFSWFNN 450
LLTSLEQEME ELGKVGFLNY RLFLTGWDSN IVGHAALNFD KATDIVTGLK 500
QKTSFGRPMW DNEFSTIATS HPKSVVGVFL CGPRTLAKSL RKCCHRYSSL 550
DPRKVQFYFN KENF

FIG. 4-2

MARVELD3

```
MEDPSGAREP RARPRERDPG RRPHPDQGRT HDRPRDRPGD PRRKRSSDGN  50
RRRDGDRDPE RDQERDGNRD RNRDRERERE RERDPDRGPR RDTHRDAGPR 100
AGEHGVWEKP RQSRTRDGAR GLTWDAAAPP GPAPWEAPEP PQPQRKGDPG 150
RRRPESEPPS ERYLPSTPRP GREEVEYYQS EAEGLLECHK CKYLCTGRAC 200
CQMLEVLLNL LILACSSVSY SSTGGYTGIT SLGGIYYYQF GGAYSGFDGA 250
DGEKAQQLDV QFYQLKLPMV TVAMACSGAL TALCCLFVAM GVLRVPWHCP 300
LLLVTEGLLD MLIAGGYIPA LYFYFHYLSA AYGSPVCKER QALYQSKGYS 350
GFGCSFHGAD IGAGIFAALG IVVFALGAVL AIKGYRKVRK LKEKPAEMFE 400
F
```

FIG. 4-3

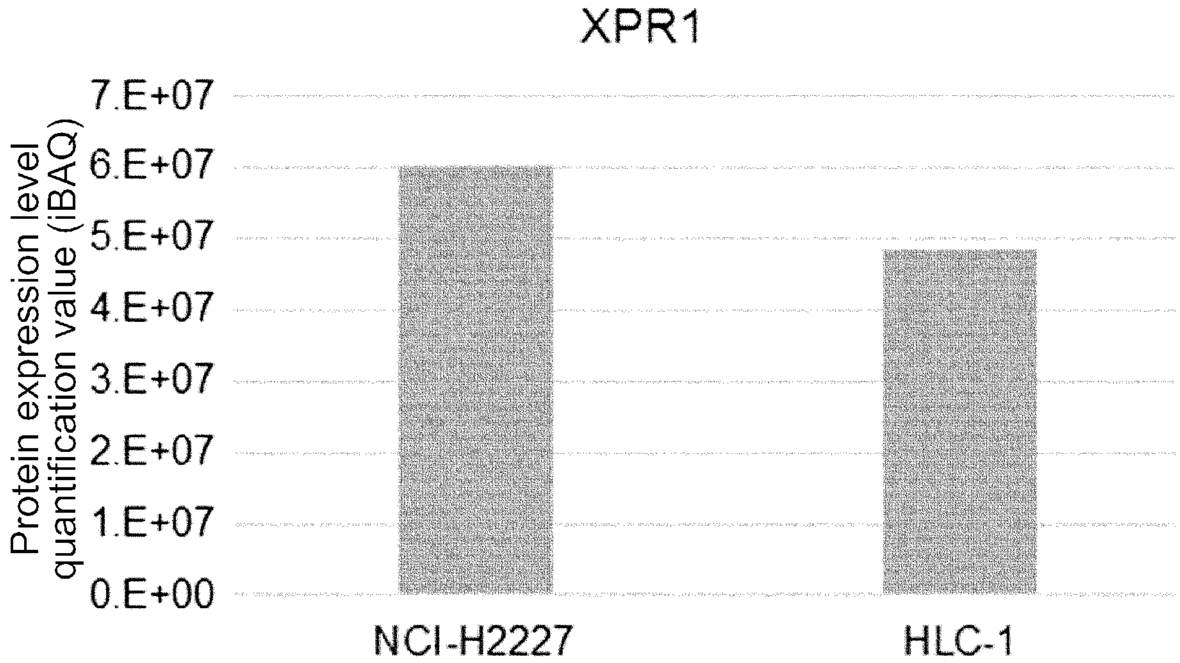
XPR1
Protein expression level quantification value (iBAQ)
7.E+07
6.E+07
5.E+07
4.E+07
3.E+07
2.E+07
1.E+07
0.E+00
NCI-H2227
HLC-1

FIG. 5-1

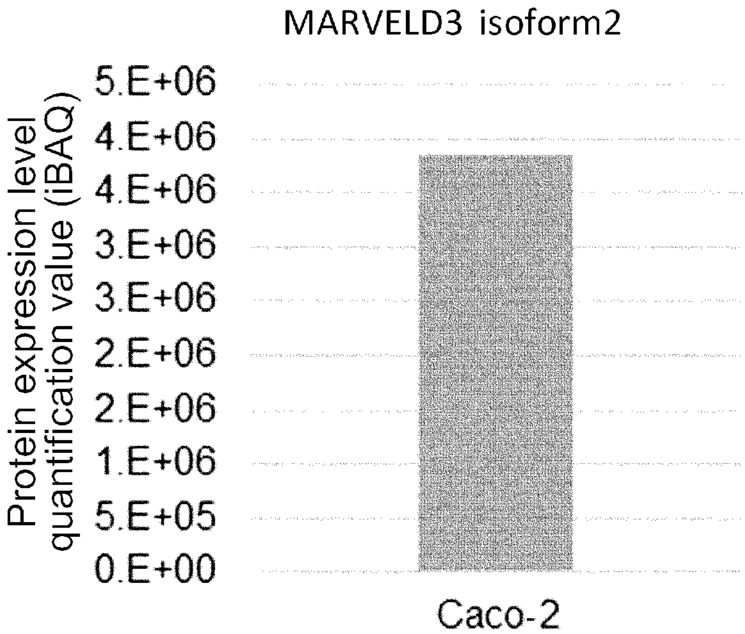
MARVELD3 isoform2
Protein expression level quantification value (iBAQ)
5.E+06
4.E+06
4.E+06
3.E+06
3.E+06
2.E+06
2.E+06
1.E+06
5.E+05
0.E+00
Caco-2

FIG. 5-2

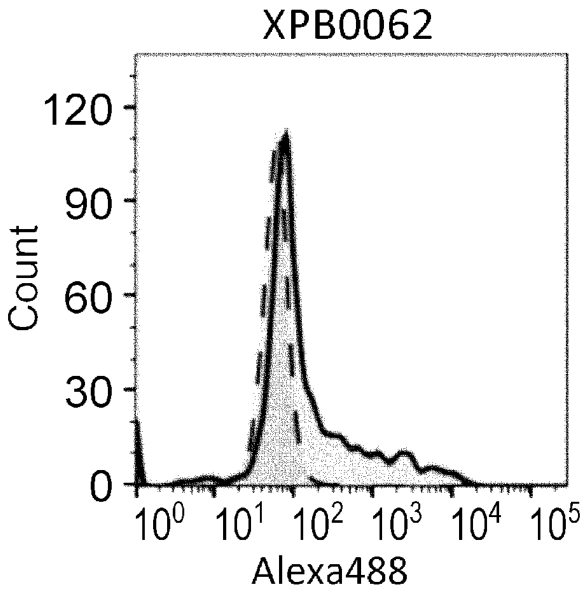


Dashed line: Expi293
Solid line: Expi293-human XPR1-Myc

FIG. 6

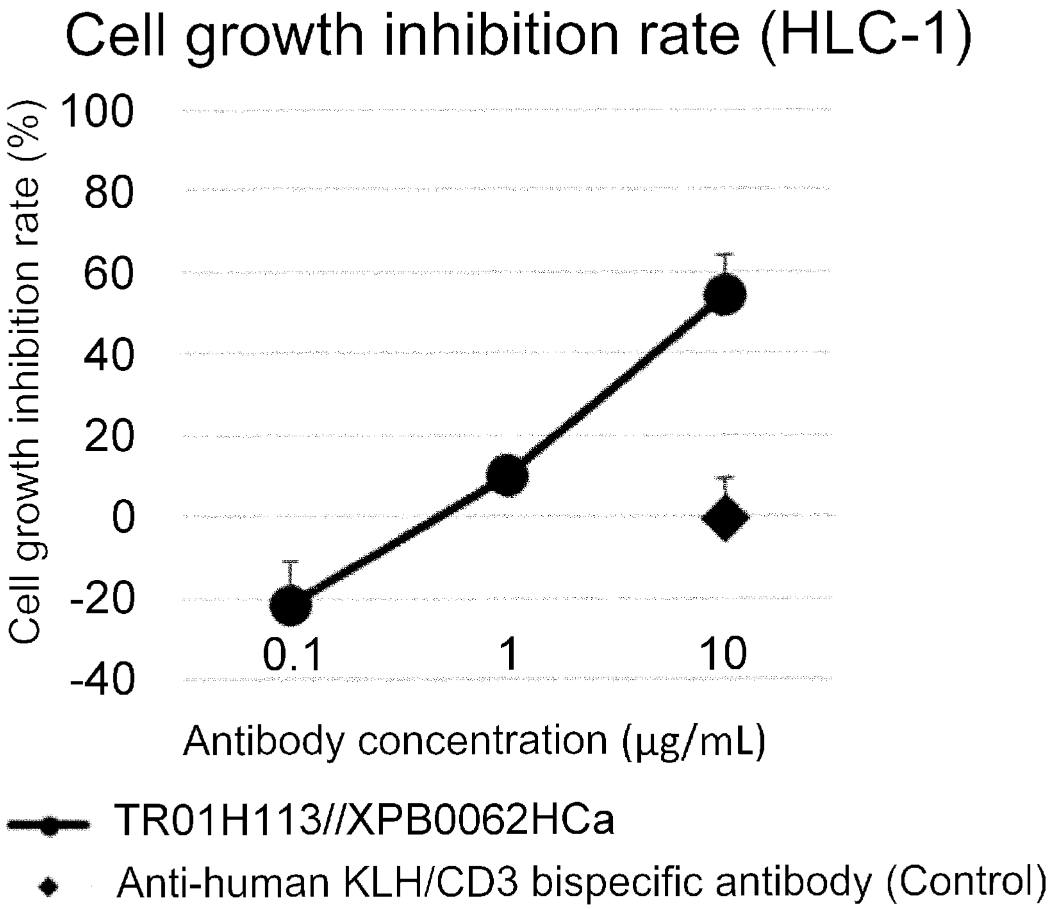
Cell growth inhibition rate (HLC-1)
Cell growth inhibition rate (%)
100
80
60
40
20
0
-20
-40
0.1
1
10
Antibody concentration (μg/mL)
TR01H113//XPB0062HCa
Anti-human KLH/CD3 bispecific antibody (Control)

FIG. 7

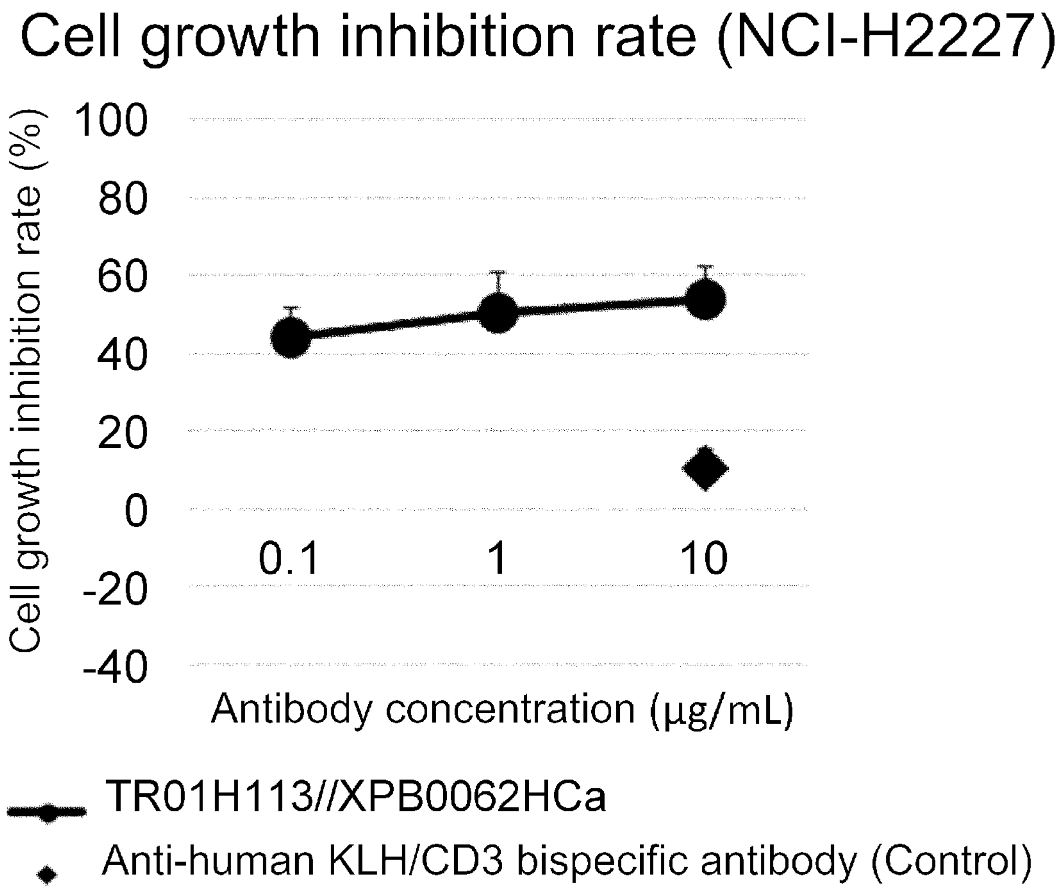
Cell growth inhibition rate (NCI-H2227)
Cell growth inhibition rate (%)
100
80
60
40
20
0
-20
-40
0.1
1
10
Antibody concentration (μg/mL)
TR01H113//XPB0062HCa
Anti-human KLH/CD3 bispecific antibody (Control)

FIG. 8

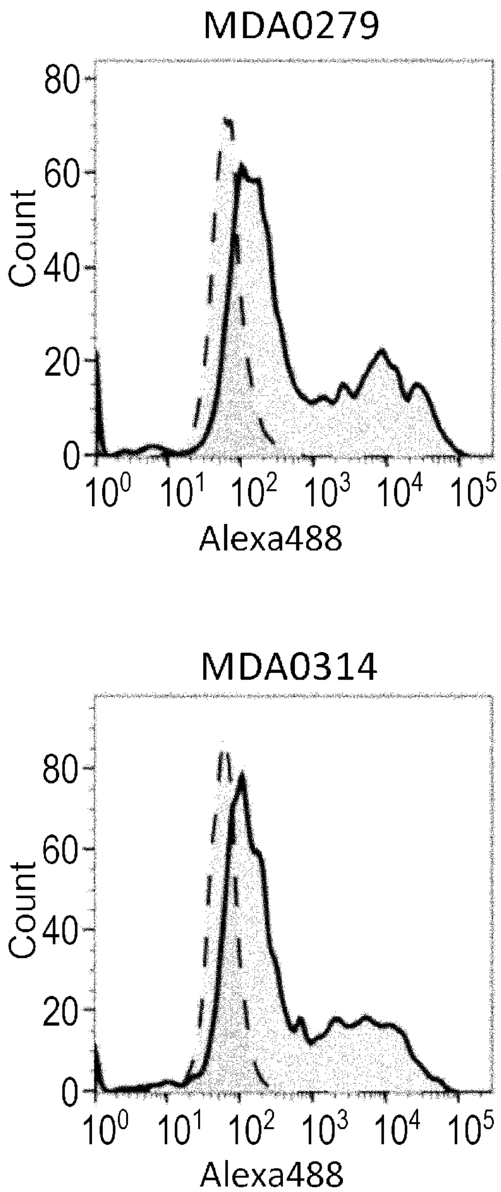


Dashed line: Expi293
Solid line: Expi293-Myc-human MARVELD3

Dashed line: Expi293
Solid line: Expi293-Myc-human MARVELD3

FIG. 9

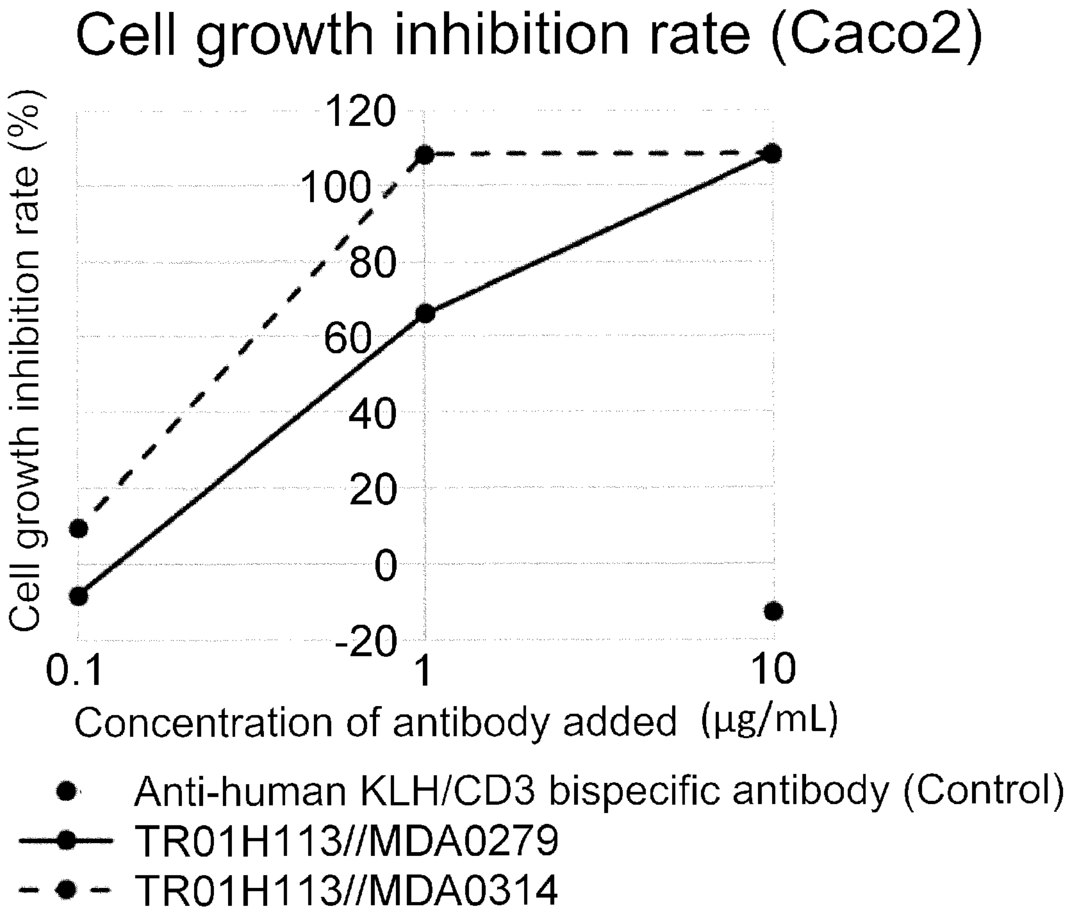
Cell growth inhibition rate (Caco2)
Cell growth inhibition rate (%)
120
100
80
60
40
20
0
-20
0.1
1
10
Concentration of antibody added (μg/mL)
Anti-human KLH/CD3 bispecific antibody (Control)
TR01H113//MDA0279
TR01H113//MDA0314

FIG. 10

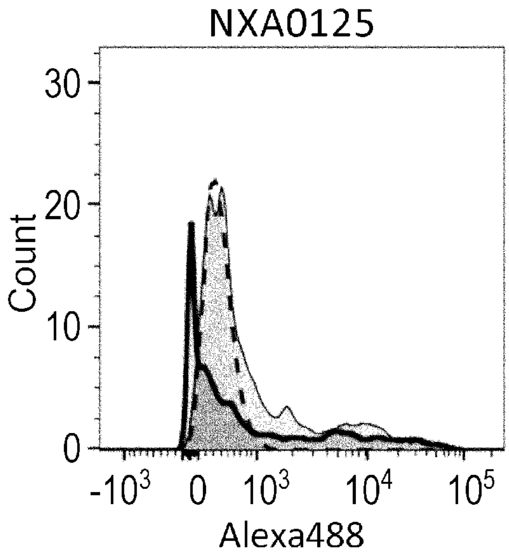


Dashed line: Expi293
Thick line: Expi293-NOX1_Nx1B_564-myc
Thin line: Expi293-human NOX1-Strep

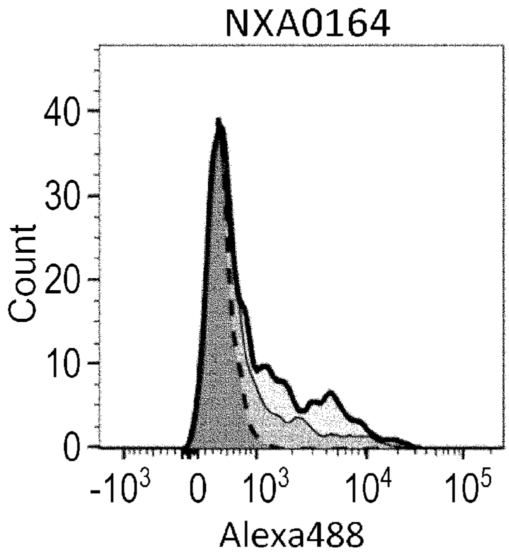


Dashed line: Expi293
Thick line: Expi293-NOX1_Nx1B_564-myc
Thin line: Expi293-human NOX1-Strep

FIG. 11

**INTERNATIONAL SEARCH REPORT**

International application No. PCT/JP2020/002476

A. CLASSIFICATION OF SUBJECT MATTER
A61K 39/395(2006.01)i; A61P 1/00(2006.01)i; A61P 11/00(2006.01)i; A61P 35/00(2006.01)i; C07K 16/28(2006.01)i; C07K 16/46(2006.01)i; C12N 15/13(2006.01)i
FI: C07K16/46; A61K39/395 T; A61P35/00; A61P11/00; A61P1/00; C12N15/13; C07K16/28 ZNA
According to International Patent Classification (IPC) or to both national classification and IPC

B. FIELDS SEARCHED
Minimum documentation searched (classification system followed by classification symbols)
A61K39/395; A61P1/00; A61P11/00; A61P35/00; C07K16/28; C07K16/46; C12N15/13

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus (STN)

C. DOCUMENTS CONSIDERED TO BE RELEVANT

| Category* | Citation of document, with indication, where appropriate, of the relevant passages | Relevant to claim No. |
|---|---|---|
| X | JP 2018-531599 A (METAFORA BIOSYSTEMS) 01.11.2018 (2018-11-01) in particular, claims 1-15 | 1, 2, 12, 13 |
| Y | in particular, claims 1-15 | 1-15 |
| Y | LAURENT. E. et al., "Nox1 is over-expressed in human colon cancers and correlates with activating mutations in K-Ras.", International Journal of Cancer, 2008, vol. 123, pp. 100-107 in particular, abstract | 1-15 |
| Y | KOJIMA, T. et al., "Downregulation of Tight Junction-Associated MARVEL Protein marvelD3 During Epithelial-Mesenchymal Transition in Human Pancreatic Cancer Cells.", Experimental Cell Research, 2011, vol. 317, pp. 2288-2298 in particular, abstract | 1-15 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 March 2020 (06.03.2020) | 24 March 2020 (24.03.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT

International application No. PCT/JP2020/002476

C (Continuation). DOCUMENTS CONSIDERED TO BE RELEVANT

| Category* | Citation of document, with indication, where appropriate, of the relevant passages | Relevant to claim No. |
|---|---|---|
| Y | FUKUSHIMA, T. et al., "Aberrant Methylation and Silencing of the SPINT2 Gene in High-Grade Gliomas.", Cancer Science, 2018, vol. 109, pp. 2970-2979 in particular, abstract | 1-15 |
| Y | HAIMAN, C. A. et al., "Genome-wide Testing of Putative Functional Exonic Variants in Relationship with Breast and Prostate Cancer Risk in a Multiethnic Population.", PLoS Genetics, 2013, vol. 9, no. 3, e1003419, pp. 1-15 in particular, abstract | 1-15 |
| Y | CONG, D. et al., "Ion transporters in brain tumors.", Current Medicinal Chemistry, 2015, vol. 22, no. 10, pp. 1171-1181 in particular, abstract | 1-15 |
| Y | UEKITA, T. and SAKAI, R., "Roles of CUB domain-containing protein 1 signaling in cancer invasion and metastasis.", Cancer Science, 2011, vol. 102, no. 11, pp. 1943-1948 in particular, abstract | 1-15 |
| Y | ISHIKAWA, N. et al., "Characterization of SEZ6L2 cell-surface protein as a novel prognostic marker for lung cancer.", Cancer Science, 2006, vol. 97, no. 8, pp. 737-745 in particular, abstract | 1-15 |
| Y | PENG, C. et al., "FLVCR1 Promotes the Proliferation and Tumorigenicity of Synovial Sarcoma Through Inhibiting Apoptosis and Autophagy.", International Journal of Oncology, 2018, vol. 52, pp. 1559-1568 in particular, abstract | 1-15 |
| Y | DING, K. et al., "GSE1 Predicts Poor Survival Outcome in Gastric Cancer Patients by SLC7A5 Enhancement of Tumor Growth and Metastasis.", Journal of Biological Chemistry, 2018, vol. 293, no. 11, pp. 3949-3964 in particular, abstract | 1-15 |
| Y | GOMES, I. M. et al., "Knockdown of STEAP1 Inhibits Cell Growth and Induces Apoptosis in LNCaP Prostate Cancer Cells Counteracting the Effect of Androgens.", Medical oncology, 2018, vol. 35, no. 40, pp. 1-10 in particular, abstract | 1-15 |
| Y | DONG, Y. et al., "Increased expression of MMP14 correlates with the poor prognosis of Chinese patients with gastric cancer.", Gene, 2015, vol. 563, pp. 29-34 in particular, abstract | 1-15 |

Form PCT/ISA/210 (continuation of second sheet) (January 2015)

INTERNATIONAL SEARCH REPORT

International application No.
PCT/JP2020/002476

C (Continuation). DOCUMENTS CONSIDERED TO BE RELEVANT

| Category* | Citation of document, with indication, where appropriate, of the relevant passages | Relevant to claim No. |
|---|---|---|
| Y | WU, H. et al., "Upregulated miR 20a 5p expression promotes proliferation and invasion of head and neck squamous cell carcinoma cells by targeting of TNFRSF21.", Oncology Reports, 2018, vol. 40, pp. 1138-1146 in particular, abstract | 1-15 |
| Y | KIM, S. et al., "TMPRSS4 induces invasion and epithelial-mesenchymal transition through upregulation of integrin α5 and its signaling pathways.", Carcinogenesis, 2010, vol. 31, no. 4, pp. 597-606 in particular, abstract | 1-15 |
| Y | WO 2017/159287 A1 (CHUGAI PHARMACEUTICAL CO., LTD.) 21.09.2017 (2017-09-21) in particular, claims 1-15, paragraphs [0016]-[0027] | 1-15 |

Form PCT/ISA/210 (continuation of second sheet) (January 2015)

**INTERNATIONAL SEARCH REPORT**

International application No.
PCT/JP2020/002476

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

INTERNATIONAL SEARCH REPORT

International application No.
PCT/JP2020/002476

<Continuation of Box No. III>

Document 15: WO 2017/159287 A1

Claim 1 sets forth an isolated antibody binding to any protein which is XPR1, NOX1, MARVELD3 isoform 1, MARVELD3 isoform 2, SPINT2, MANSC1, SLC12A2, CDCP1, SEZ6L2, FLVCR1, SLC7A5, STEAP1, MMP14, TNFRSF21, or TMPRSS4. These antibodies share the common technical feature of "an isolated antibody binding to a tumor antigen," but said technical feature does not make a contribution over the prior art in light of the disclosure of document 15, and thus is not a special technical feature. Also, there are no other identical or corresponding special technical features between these antibodies.

Therefore, the invention in claim 1 is classified into 15 inventions. This also applies to claims 2-15 referring to claim 1, and the invention in claims 1-15 includes 15 inventions.

Form PCT/ISA/210 (extra sheet) (January 2015)

**INTERNATIONAL SEARCH REPORT**
Information on patent family members

International application No.
PCT/JP2020/002476

| Patent Documents referred in the Report | Publication Date | Patent Family | Publication Date |
|---|---|---|---|
| JP 2018-531599 A | 01 Nov. 2018 | US 2018/0280549 A1<br>in particular, claims 16-32<br>WO 2017/060304 A1<br>EP 3359203 A1<br>CN 108472394 A | |
| WO 2017/159287 A1 | 21 Sep. 2017 | EP 3431102 A1<br>in particular, claims 1-15, paragraphs [0020]-[0034]<br>CN 109069640 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200492219 A, Hinton
- WO 200042072 A, Presta
- US 5500362 A
- US 5821337 A
- US 6737056 B, Presta
- US 6194551 B1
- WO 199951642 A
- WO 9316185 A
- US 5571894 A
- US 5587458 A
- US 5869046 A
- EP 404097 A
- WO 199301161 A
- US 6248516 B1
- US 4816567 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A1
- US 20080069820 A
- WO 2008077546 A
- US 20030157108 A
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1
- WO 2004056312 A1
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- WO 2006029879 A
- WO 2005100402 A
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- WO 9411523 A
- EP 239400 A
- WO 1996002576 A
- WO 1993012227 A
- WO 1992003918 A
- WO 1994002602 A
- WO 1994025585 A
- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- US 5208020 A
- US 5416064 A
- EP 0425235 B1
- US 5635483 A
- US 5780588 A

- US 7498298 B
- US 5712374 A
- US 5714586 A
- US 5739116 A
- US 5767285 A
- US 5770701 A
- US 5770710 A
- US 5773001 A
- US 5877296 A
- US 6630579 B
- WO 9411026 A
- US 4737456 A
- US 20050260186 A
- US 20060104968 A
- US 6267958 B
- US 6171586 B
- WO 2006044908 A


**Non-patent literature cited in the description**


- **JANICE M REICHERT ; CLARK J ROSENSWEIG ; LAURA B FADEN ; MATTHEW C DEWITZ.** Monoclonal antibody successes in the clinic. *Nat. Biotechnol.,* 2005, vol. 23, 1073-1078
- **PAVLOU AK ; BELSEY MJ.** The therapeutic antibodies market to 2008. *Eur. J. Pharm. Biopharm.,* 2005, vol. 59 (3), 389-396
- **WEINER LM ; SURANA R ; WANG S.** Monoclonal antibodies: versatile platforms for cancer immunotherapy. *Nat. Rev. Immunol.,* 2010, vol. 10 (5), 317-327
- **LEWIS GD ; FIGARI I ; FENDLY B ; WONG WL ; CARTER P ; GORMAN C ; SHEPARD HM.** Differential responses of human tumor cell lines to anti-p185HER2 monoclonal antibodies. *Cancer Immunol. Immunotherapy,* 1993, vol. 37, 255-263
- **DE BONO JS ; TOLCHER AW ; FORERO A ; VANHOVE GF ; TAKIMOTO C ; BAUER RJ ; HAMMOND LA ; PATNAIK A ; WHITE ML ; SHEN S.** ING-1, a monoclonal antibody targeting Ep-CAM in patients with advanced adenocarcinomas. *Clin. Cancer Res.,* 2004, vol. 10 (22), 7555-7565
- **SATOH M ; IIDA S ; SHITARA K.** Non-fucosylated therapeutic antibodies as next-generation therapeutic antibodies. *Expert Opin. Biol. Ther.,* 2006, vol. 6 (11), 1161-1173
- **DESJARLAIS JR ; LAZAR GA ; ZHUKOVSKY EA ; CHU SY.** Optimizing engagement of the immune system by anti-tumor antibodies: an engineer's perspective. *Drug Discov. Today,* 2007, vol. 12 (21-22), 898-910
- **ALLEY SC ; OKELEY NM ; SENTER PD.** Antibody-drug conjugates: targeted drug delivery for cancer. *Curr. Opin. Chem. Biol.,* 2010, vol. 14 (4), 529-537
- **BAEUERLE PA ; KUFER P ; BARGOU R.** BiTE: Teaching antibodies to engage T-cells for cancer therapy. *Curr. Opin. Mol. Ther.,* 2009, vol. 11 (1), 22-30
- **LUTTERBUESE R ; RAUM T ; KISCHEL R ; HOFFMANN P ; MANGOLD S ; RATTEL B ; FRIEDRICH M ; THOMAS O ; LORENCZEWSKI G ; RAU D.** T cell-engaging BiTE antibodies specific for EGFR potently eliminate KRAS- and BRAF-mutated colorectal cancer cells. *Proc. Natl. Acad. Sci. U.S.A.,* 2010, vol. 107 (28), 12605-12610
- **RIECHELMANN H ; SAUTER A ; GOLZE W ; HANFT G ; SCHROEN C ; HOERMANN K ; ERHARDT T ; GRONAU S.** Phase I trial with the CD44v6-targeting immunoconjugate bivatuzumab mertansine in head and neck squamous cell carcinoma. *Oral Oncol.,* 2008, vol. 44 (9), 823-829
- **KIM SJ ; PARK Y ; HONG HJ.** Antibody engineering for the development of therapeutic antibodies. *Mol. Cells.,* 2005, vol. 20 (1), 17-29
- **RALEIGH D.R.** *Mol. Biol. Cell,* 2010, vol. 21
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **HARLOW ; LANE.** Antibodies: A Laboratory Manual. Cold Spring Harbor Laboratory, 1988
- **DAËRON.** *Annu. Rev. Immunol.,* 1997, vol. 15, 203-234
- **RAVETCH ; KINET.** *Annu. Rev. Immunol,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249

- **GHETIE ; WARD.** *Immunol. Today,* 1997, vol. 18 (12), 592-598
- **GHETIE et al.** *Nature Biotechnology,* 1997, vol. 15 (7), 637-640
- **HINTON et al.** *J. Biol. Chem.,* 2004, vol. 279 (8), 6213-6216
- **SHIELDS et al.** *J. Biol. Chem.,* 2001, vol. 9 (2), 6591-6604
- **CLYNES et al.** *PNAS (USA),* 1998, vol. 95, 652-656
- **GAZZANO-SANTORO et al.** *J. Immunol. Methods,* 1996, vol. 202, 163
- **IDUSOGIE et al.** *J. Immunol.,* 2000, vol. 164, 4178-4184
- **FLATMAN et al.** *J. Chromatogr. B,* 2007, vol. 848, 79-87
- *Proc Natl Acad Sci USA.,* 1999, vol. 96, 927-932
- *Proc Natl Acad Sci USA.,* 1999, vol. 96, 1385-1390
- *Nat Genet.,* 1999, vol. 21, 216-219
- *J Biol Chem.,* 1995, vol. 270, 25435-25444
- *Trends Genet.,* 1995, vol. 11, 209-211
- *Trends Biochem Sci.,* 1996, vol. 21, 383-387
- *Mol Gen Genet.,* 1996, vol. 252, 709-716
- *Biochem Biophys Res Commun.,* 20 August 2010, vol. 399 (2), 129-32
- *J Virol.,* 08 October 2007, vol. 1 (19), 10550-7
- *Retrovirology,* 07 October 2009, vol. 6, 87
- *J Virol.,* 08 November 2010, vol. 4 (22), 11970-80
- *J Virol.,* 07 November 1999, vol. 3 (11), 9362-8
- *Retrovirology,* 15 December 2005, vol. 2, 76
- *Retrovirology,* 30 November 2010, vol. 7, 101
- *Virology,* 04 October 2016, vol. 97, 53-58
- *Physiol Rev.,* 2007, vol. 87, 245-313
- *Cell Mol. Life Sci.,* 2012, vol. 69, 2327-2343
- *Free Radical Biol. Med.,* 2007, vol. 43, 332-347
- *Cancer Sci.,* 2009, vol. 100 (8), 1382-1388
- *Cancer. Lett.,* 2008, vol. 266 (1), 37-52
- *Anticancer Agents Med Chem.,* 2013, vol. 13 (3), 502-14
- *Clinical Science,* 2015, vol. 128, 863-875
- *Int J Cancer.,* 2008, vol. 123 (1), 100-7
- *Genes Cells.,* January 2013, vol. 18 (1), 32-41
- *Gene,* 2001, vol. 16, 269 (1-2), 131-40
- *Biochem Biophys Res Commun.,* 2014, vol. 17;443 (3), 1060-5
- *Mol. Biol. Cell.,* 2010, vol. 21, 1200-1213
- *BMC Cell Biol.,* 22 December 2009, vol. 10, 95
- *J Cell Sci,* 2013, vol. 126, 554-564
- *Exp Cell Res.,* 01 October 2011, vol. 317 (16), 2288-98
- *Mol. Biol. Cell,* 2010, vol. 21, 1200-1213
- **CHEN et al.** *J. Mol. Biol.,* 1999, vol. 293, 865-881
- **PRESTA et al.** *Cancer Res.,* 1997, vol. 57, 4593-4599
- **HUDSON et al.** *Nat. Med.,* 2003, vol. 9, 129-134
- **PLUCKTHÜN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91

- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CHOWDHURY.** *Methods Mol. Biol.,* 2008, vol. 207, 179-196
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **DUNCAN ; WINTER.** *Nature,* 1988, vol. 322, 738-40
- **KAM et al.** *Proc. Natl. Acad. Sci. USA,* 2005, vol. 102, 11600-11605
- *J. Immunol.,* 1979, vol. 123 (4), 1548-1550
- *Current Topics in Microbiology and Immunology,* 1978, vol. 81, 1-7
- *C. Eur. J. Immunol.,* 1976, vol. 6 (7), 511-519
- *Cell,* 1976, vol. 8 (3), 405-415
- *Nature,* 1978, vol. 276 (5685), 269-270
- *J. Immunol. Methods,* 1980, vol. 35 (1-2), 1-21
- *J. Exp. Med.,* 1978, vol. 148 (1), 313-323
- *Nature,* 1979, vol. 277 (5692), 131-133
- **KOHLER ; MILSTEIN et al.** *Methods Enzymol.,* 1981, vol. 73, 3-46
- **VANDAMME et al.** *Eur. J. Biochem.,* 1990, vol. 192 (3), 767-775
- *Biochemistry,* 1979, vol. 18 (24), 5294-5299
- *Anal. Biochem.,* 1987, vol. 162 (1), 156-159
- *Proc. Natl. Acad. Sci. USA,* 1988, vol. 85 (23), 8998-9002
- *Nucleic Acids Res.,* 1989, vol. 17 (8), 2919-2932
- *Bio/Technology,* 1994, vol. 12 (7), 699-702
- **SATO, K. et al.** *Cancer Res.,* 1993, vol. 53, 851-856
- *Nat Biotechnol.,* December 2000, vol. 18 (12), 1287-92
- *Nucleic Acids Res.,* 2006, vol. 34 (19), e127
- *Proc Natl Acad Sci U S A.,* 02 March 2004, vol. 101 (9), 2806-10
- *Proc Natl Acad Sci U S A.,* 22 June 2004, vol. 101 (25), 9193-8
- *Protein Eng Des Sel.,* April 2008, vol. 21 (4), 247-55
- *Proc Natl Acad Sci U S A.,* 26 September 2000, vol. 97 (20), 10701-5
- *MAbs.,* September 2010, vol. 2 (5), 508-18
- *Methods Mol Biol.,* 2012, vol. 911, 183-98
- **HINMAN et al.** *Cancer Res.,* 1993, vol. 53, 3336-3342
- **LODE et al.** *Cancer Res.,* 1998, vol. 58, 2925-2928
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834

- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **VITETTA et al.** *Science,* 1987, vol. 238, 1098
- **CHARI et al.** *Cancer Res.,* 1992, vol. 52, 127-131
- Remington's Pharmaceutical Sciences. 1980
- *Cell,* 01 November 2018, vol. 175 (4), 1131-1140